(19) Europäisches Patentamt
European Patent Office
Office européen des brevets

(11) Veröffentlichungsnummer: **0 173 956**
**A1**

# (12) EUROPÄISCHE PATENTANMELDUNG

(21) Anmeldenummer: 85110821.7

(22) Anmeldetag: 19.08.85

(51) Int. Cl.⁴: C 07 D 251/46
C 07 D 251/16, C 07 D 251/18
A 01 N 47/44

Ein Antrag gemäss Regel 88 EPÜ auf Berichtigung der Seite 53 der Beschreibung liegt vor. Über diesen Antrag wird im Laufe des Verfahrens von der Prüfungsabteilung eine Entscheidung getroffen werden.

(30) Priorität: 30.08.84 DE 3431925
17.05.85 DE 3517821

(43) Veröffentlichungstag der Anmeldung:
12.03.86 Patentblatt 86/11

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Diehr, Hans-Joachim, Dr.
Höhe 35
D-5600 Wuppertal 1(DE)

(72) Erfinder: Fest, Christa, Dr.
Im Johannistal 20
D-5600 Wuppertal 1(DE)

(72) Erfinder: Kirsten, Rolf, Dr.
Carl-Langhans-Strasse 27
D-4019 Monheim(DE)

(72) Erfinder: Kluth, Joachim, Dr.
Kurt-Schumacher-Strasse 9
D-4018 Langenfeld(DE)

(72) Erfinder: Müller, Klaus-Helmut, Dr.
Bockhackstrasse 55
D-4000 Düsseldorf 13(DE)

(72) Erfinder: Pfister, Theodor, Dr.
Lichtenberger Strasse 30
D-4019 Monheim(DE)

(72) Erfinder: Priesnitz, Uwe, Dr.
Severinstrasse 58
D-5650 Solingen 1(DE)

(72) Erfinder: Riebel, Hans-Jochem, Dr.
In der Beek 92
D-5600 Wuppertal 1(DE)

(72) Erfinder: Roy, Wolfgang, Dr.
Walter-Kolb-Strasse 47
D-4018 Langenfeld(DE)

(72) Erfinder: Santel, Hans-Joachim, Dr.
Gerstenkamp 19
D-5000 Köln 80(DE)

(72) Erfinder: Schmidt, Robert R., Dr.
Im Waldwinkel 110
D-5060 Bergisch-Gladbach 2(DE)

## (54) Sulfonylguanidinotriazin-Derivate.

(57) Die Erfindung betrifft neue Sulfonylguanidinotriazin-Derivate der allgemeinen Formel I

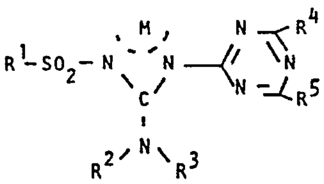

(I)

(worin die Reste M, $R^1$, $R^2$, $R^3$, $R^4$ und $R^5$ die in der Beschreibung angegebenen Bedeutungen haben)

sowie 1:1-Addukte von Verbindungen der Formel (I) mit starken Säuren, Verfahren zu ihrer Herstellung und ihre Verwendung als Herbizide.

0173956

BAYER AKTIENGESELLSCHAFT 5090 Leverkusen, Bayerwerk

Konzernverwaltung RP Bi/mü

Patentabteilung Ib

Sulfonylguanidinotriazin-Derivate

Die Erfindung betrifft neue Sulfonylguanidinotriazin-Derivate, Verfahren zu ihrer Herstellung und ihre Verwendung als Herbizide.

Verschiedene Guanidin-Derivate sind aus Patentschriften (vgl. DE-AS 1 089 210, DD-PS 71 016 und 84 530) als potentielle Herbizide bekannt geworden, haben jedoch bisher als Mittel zur Unkrautbekämpfung und/oder Regulierung des Pflanzenwachstums keine größere Bedeutung erlangt. Weitere Guanidin-Derivate sind bekannt (vgl. DE-OS 3 344 455).

Es wurden nun neue Sulfonylguanidinotriazin-Derivate der allgemeinen Formel (I)

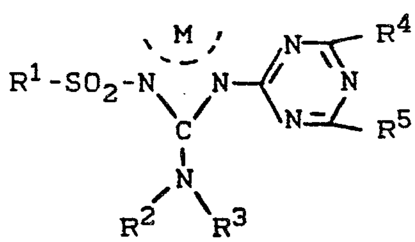 (I)

in welcher

M für Wasserstoff oder ein Metalläquivalent steht,

$R^1$ für einen gegebenenfalls substituierten Phenylrest

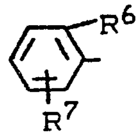 steht, worin

$R^6$ und $R^7$ gleich oder verschieden sind und für Wasserstoff, Halogen [wie insbesondere Fluor, Chlor und/oder Brom], Cyano, Nitro, $C_1$-$C_4$-Alkyl, [welches gegebenenfalls durch Fluor, Chlor, Brom, Cyano, $C_1$-$C_4$-Alkoxy-carbonyl, $C_1$-$C_4$-Alkylamino-carbonyl, Di-($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_4$-Alkoxy [welches gegebenenfalls durch Fluor, Chlor, Brom, Cyano, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_4$-Alkylthio, $C_1$-$C_4$-Alkylsulfinyl oder für $C_1$-$C_4$-Alkylsulfonyl [welche gegebenenfalls durch Fluor, Chlor, Brom, Cyano oder $C_1$-$C_4$-Alkoxy-carbonyl substituiert sind], für $C_1$-$C_4$-Alkylamino-sulfonyl, Di-($C_1$-$C_4$-alkyl)amino-sulfonyl, $C_1$-$C_4$-Alkoxyamino-sulfonyl,

Benzyloxyaminosulfonyl, $C_1$-$C_4$-Alkoxy-$C_1$-$C_4$-alkylamino-sulfonyl, für Phenyl oder Phenoxy, für $C_1$-$C_4$-Alkoxysulfonyl, für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano oder $C_1$-$C_4$-Alkoxy-carbonyl substituiert ist], für $C_3$-$C_6$-Alkenoxy [welches gegebenenfalls durch Fluor, Chlor, Brom, Cyano oder $C_1$-$C_4$-Alkoxy-carbonyl substituiert ist], für $C_3$-$C_6$-Alkenylthio [welches gegebenenfalls durch Fluor, Chlor, Brom, Cyano oder $C_1$-$C_4$-Alkoxy-carbonyl substituiert ist], für $C_1$-$C_6$-Alkoxy-carbonyl [welches gegebenenfalls durch Fluor, Chlor, Brom, Cyano oder $C_1$-$C_4$-Alkoxy substituiert ist], für $C_3$-$C_6$-Cycloalkoxy-carbonyl, für $C_1$-$C_6$-Alkylthiocarbonyl [welches gegebenenfalls durch Fluor, Chlor, Brom, Cyano oder $C_1$-$C_4$-Alkoxy-carbonyl substituiert ist], für Aminocarbonyl, $C_1$-$C_4$-Alkylamino-carbonyl, Di-($C_1$-$C_4$-alkyl)-amino-carbonyl, $C_1$-$C_4$-Alkoxyamino-carbonyl, $C_3$-$C_4$-Alkenyloxy-amino-carbonyl, Benzyloxyaminocarbonyl,

$C_1$-$C_4$-Alkoxy-$C_1$-$C_4$-alkyl-amino-carbonyl, Dimethylhydrazinocarbonyl, Dimethylhydrazinosulfonyl, für $C_3$-$C_6$-Alkinyloxy oder $C_3$-$C_6$-Alkinylthio stehen,

in welcher weiter

$R^1$ für einen gegebenenfalls substituierten Benzylrest

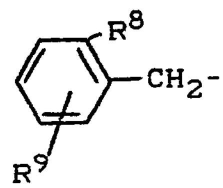 steht, worin

$R^8$ und $R^9$ gleich oder verschieden sind und für Wasserstoff, Halogen [wie insbesondere Fluor, Chlor oder Brom], Cyano, Nitro, $C_1$-$C_4$-Alkyl [welches gegebenenfalls durch Fluor, und/oder Chlor substituiert ist], $C_1$-$C_4$-Alkoxy [welches gegebenenfalls durch Fluor und/oder Chlor substituiert ist], $C_1$-$C_4$-Alkoxy-carbonyl, $C_1$-$C_4$-Alkylthio, $C_1$-$C_4$-Alkylsulfinyl, $C_1$-$C_4$-Alkylsulfonyl oder Di-($C_1$-$C_4$-alkyl)-aminosulfonyl stehen;

in welcher weiter

$R^2$ für Wasserstoff, $C_1$-$C_4$-Alkyl oder einen Sulfonylrest

$R^{10}$-$SO_2$- steht, worin

$R^{10}$ für $C_1$-$C_{12}$-Alkyl [welches gegebenenfalls durch Fluor, Chlor, Brom, Cyano, Nitro, $C_1$-$C_4$-Alkoxy-carbonyl, $C_1$-$C_4$-Alkylamino-carbonyl, Di-($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_{12}$-Alkoxy [welches gegebenenfalls durch Fluor, Chlor, Brom, Cyano, Nitro, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_2$-$C_{12}$-Alkenyl [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_2$-$C_{12}$-Alkenyloxy [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_1$-$C_8$-Alkylamino, Di-($C_1$-$C_4$-alkyl)-amino, $C_3$-$C_6$-Cycloalkyl-amino oder Di-($C_3$-$C_6$-cycloalkyl)-amino [welche gegebenenfalls durch Fluor, Chlor, Brom, Cyano, Nitro, Phenyl, Phenoxy, $C_1$-$C_4$-Alkoxy,$C_1$-$C_4$-Alkylthio, $C_1$-$C_4$-Alkylsulfinyl oder $C_1$-$C_4$-Alkylsulfonyl substituiert sind], für $C_2$-$C_8$-Alkenylamino [welches gegebenenfalls durch Fluor, Chlor, Brom, Cyano, Nitro oder Phenyl substituiert ist], für Phenylamino oder Benzylamino [welche gegebenenfalls durch Fluor, Chlor, Brom,

$C_1$-$C_4$-Alkyl, Trifluormethyl, $C_1$-$C_4$-Alkoxy, $C_1$-$C_2$-Fluoralkoxy, $C_1$-$C_4$-Alkylthio, $C_1$-$C_4$-Alkylsulfinyl, $C_1$-$C_4$-Alkylsulfonyl, Cyano, Nitro und/oder $C_1$-$C_4$-Alkoxy-carbonyl substituiert sind], für Benzyl, [welches gegebenenfalls durch Fluor, Chlor, Brom, Cyano, Nitro, $C_1$-$C_4$-Alkyl, Trifluormethyl, $C_1$-$C_4$-Alkoxy-carbonyl, $C_1$-$C_4$-Alkoxy oder Di-($C_1$-$C_4$-alkyl)-aminosulfonyl substituiert ist] oder für einen gegebenenfalls substituierten Phenylrest

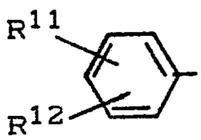

steht, worin

$R^{11}$ und $R^{12}$ gleich oder verschieden sind und die oben für $R^6$ und $R^7$ angegebenen Bedeutungen haben, jedoch nicht in jedem Einzelfall mit $R^6$ und $R^7$ identisch sind;

in welcher weiter

$R^3$ für den Rest $-O-R^{13}$ steht, worin

$R^{13}$ für $C_1$-$C_{12}$-Alkyl [welches gegebenenfalls durch Fluor, Chlor, Brom, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_3$-$C_6$-Cycloalkyl, $C_3$-$C_6$-Cycloalkyl-

$C_1$-$C_2$-alkyl, $C_3$-$C_6$-Alkenyl [welches gegebenenfalls durch Fluor, Chlor oder Brom substituiert ist], $C_3$-$C_6$-Alkinyl, $C_1$-$C_4$-Alkoxy-carbonyl-$C_1$-$C_2$-alkyl, Aminocarbonyl-methyl, $C_1$-$C_4$-Alkylamino-carbonyl-methyl, Di-($C_1$-$C_4$-alkyl)-amino-carbonyl-methyl oder für Phenyl, Benzyl oder Phenylethyl [welche gegebenenfalls durch Fluor, Chlor, Nitro, Cyano, $C_1$-$C_4$-Alkyl, $C_1$-$C_4$-Alkoxy oder $C_1$-$C_4$-Alkoxycarbonyl substituiert sind] steht;

in welcher weiter

$R^3$ für den Rest

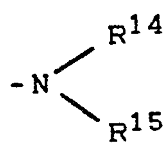

steht, worin

$R^{14}$ für Wasserstoff oder $C_1$-$C_4$-Alkyl steht und

$R^{15}$ für Wasserstoff, $C_1$-$C_4$-Alkyl [welches gegebenenfalls durch Fluor, Chlor, Brom, Cyano, $C_1$-$C_4$-Alkoxy oder $C_1$-$C_4$-Alkoxycarbonyl substituiert ist], für $C_3$-$C_6$-Cycloalkyl, für Phenyl, Benzyl oder Phenylethyl [welche gegebenenfalls durch Fluor, Chlor, Brom, Cyano, Nitro, $C_1$-$C_4$-Alkyl, $C_1$-$C_4$-Alkoxy oder $C_1$-$C_4$-Alkoxycarbonyl substituiert sind], für $C_1$-$C_4$-Alkyl-carbonyl, $C_1$-$C_4$-Alkoxy-carbonyl, $C_1$-$C_4$-Alkyl-sulfonyl oder Phenylsulfonyl

[welches gegebenenfalls durch Fluor, Chlor, Brom, Cyano, Nitro oder Methyl substituiert ist] steht;

in welcher weiter

- für den Fall, daß $R^2$ für einen Sulfonylrest $R^{10}$-$SO_2$- steht, worin $R^{10}$ die oben angegebene Bedeutung hat - $R^3$ auch für Wasserstoff steht;

in welcher weiter

$R^4$ für Fluor, Chlor, Brom, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für $C_1$-$C_4$-Alkylthio [welches gegebenenfalls durch Fluor und/oder Chlor substituiert ist] oder für $C_1$-$C_4$-Alkyl- oder Di-($C_1$-$C_4$-alkyl)-amino [welche gegebenenfalls durch Fluor substituiert sind] steht und

$R^5$ für Fluor, Chlor, Brom, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für $C_1$-$C_4$-Alkylthio [welches gegebenenfalls durch Fluor und/oder Chlor substituiert ist], für $C_1$-$C_4$-Alkyl- oder Di-($C_1$-$C_4$-alkyl)-amino [welche gegebenenfalls durch Fluor substituiert sind] steht,

sowie 1:1-Addukte von Verbindungen der Formel (I) mit starken Säuren gefunden,

wobei folgende Verbindungen ausgenommen sind:

N'-(4-Methoxy-6-methyl-s-triazin-2-yl)-N"-dimethylamino-N'''-(2-difluormethoxy-benzolsulfonyl)-guanidin,
N'-(4,6-Dimethoxy-s-triazin-2-yl)-N"-methoxy-N",N'''-bis-(2-trifluormethyl-benzolsulfonyl)-guanidin,
N'-(4-Dimethylamino-6-methyl-s-triazin-2-yl)-N"-methoxy-N'''-(2-methyl-benzolsulfonyl)-guanidin und
N'-(4-Dimethylamino-6-methyl-s-triazin-2-yl)-N"-dimethylamino-N'''-(2-methyl-benzolsulfonyl)-guanidin.

Die allgemeine Formel (I) steht - wenn M für Wasserstoff steht - für die einzelnen Tautomeren der Formeln (IA) und (IB)

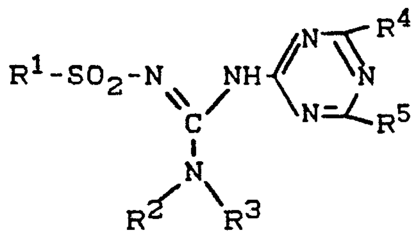

(IA)

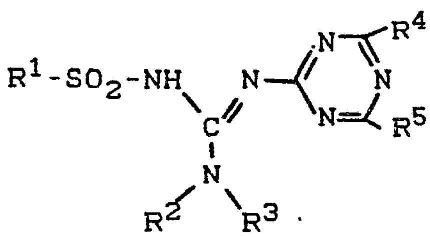

(IB)

in welchen

$R^1$, $R^2$, $R^3$, $R^4$ und $R^5$ die oben angegebenen Bedeutungen haben,

sowie für Gemische der Tautomeren (IA) und (IB).

Das Mischungsverhältnis (IA)/(IB) hängt von aggregationsbestimmenden Faktoren, wie z.B. Temperatur, Lösungsmittel und Konzentration ab.

Für den Fall, daß neben M auch $R^2$ für Wasserstoff steht, ist eine weitere tautomere Form (IC) möglich:

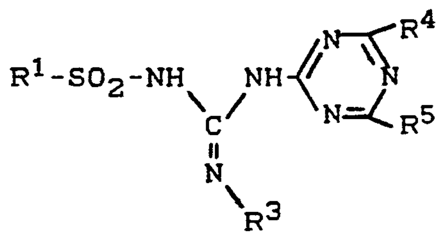

(IC)

in welcher

$R^1$, $R^3$, $R^4$ und $R^5$ die oben angegebenen Bedeutungen haben.

Man erhält die neuen Sulfonylguanidinotriazin-Derivate der Formel (I)

(a) für den Fall, daß M für Wasserstoff steht,

wenn man Guanidinotriazin-Derivate der Formel (II)

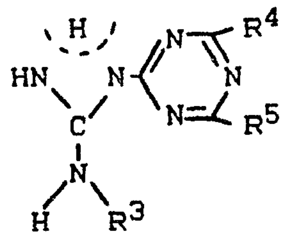

(II)

in welcher

$R^3$, $R^4$ und $R^5$ die oben angegebenen Bedeutungen haben,

mit Sulfonsäurechloriden der Formel (III)

$R^1-SO_2-Cl$ (III)

in welcher

$R^1$ die oben angegebene Bedeutung hat,

in Gegenwart von Säureakzeptoren und gegebenenfalls in Gegenwart von Verdünnungsmitteln umsetzt;

oder

(b) für den Fall, daß M für Wasserstoff steht und $R^2$ für Wasserstoff oder $C_1$-$C_4$-Alkyl steht,

wenn man die nach dem oben unter (a) angegebenen Verfahren erhältlichen Sulfonylguanidinotriazin-Derivate der Formel (ID)

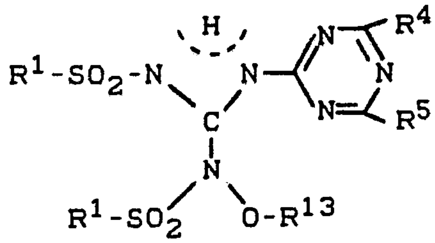

(ID)

in welcher

$R^1$, $R^4$, $R^5$ und $R^{13}$ die oben angegebenen Bedeutungen haben,

mit Aminoverbindungen der Formel (IV)

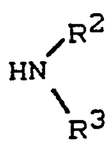

(IV)

in welcher

$R^2$ für Wasserstoff oder $C_1$-$C_4$-Alkyl steht und
$R^3$ die oben angegebene Bedeutung hat,

bzw. mit Hydrochloriden von Aminoverbindungen der Formel (IV), gegebenenfalls in Gegenwart von Säureakzeptoren und gegebenenfalls in Gegenwart von Verdünnungsmitteln umsetzt;

oder

(c) für den Fall, daß M für Wasserstoff steht und $R^2$ für einen gegebenenfalls substituierten Sulfonylrest

$R^{10}-SO_2-$ steht, worin

$R^{10}$ die oben angegebene Bedeutung hat,

wenn man Sulfonylguanidinotriazin-Derivate der Formel (IE)

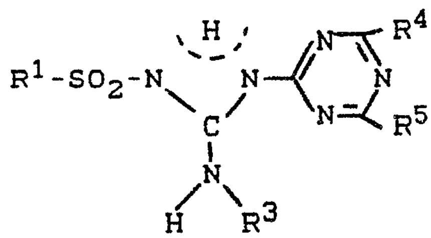 (IE)

in welcher

$R^1$, $R^3$, $R^4$ und $R^5$ die oben angegebenen Bedeutungen haben,

mit Sulfonsäurechloriden der Formel (V)

$$R^{10}-SO_2-Cl \qquad (V)$$

in welcher

$R^{10}$ die oben angegebene Bedeutung hat,

in Gegenwart von Säureakzeptoren und gegebenenfalls in Gegenwart von Verdünnungsmitteln umsetzt;

oder

(d) ebenfalls für den Fall, daß M für Wasserstoff steht und $R^2$ für einen gegebenenfalls substituierten Sulfonylrest

$R^{10}-SO_2-$ steht, worin

$R^{10}$ die oben angegebene Bedeutung hat,

wenn man Sulfonylguanidinotriazin-Derivate der Formel (VI)

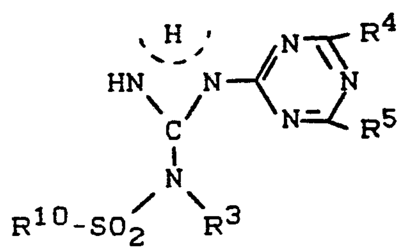

(VI)

in welcher

$R^3$, $R^4$, $R^5$ und $R^{10}$ die oben angegebenen Bedeutungen haben,

mit Sulfonsäurechloriden der Formel (III)

$$R^1-SO_2-Cl \qquad (III)$$

in welcher

$R^1$ die oben angegebene Bedeutung hat,

in Gegenwart von Säureakzeptoren und gegebenenfalls in Gegenwart von Verdünnungsmitteln umsetzt;

oder

(e) für den Fall, daß M für Wasserstoff steht und $R^2$ für Wasserstoff oder $C_1$-$C_4$-Alkyl steht,

wenn man Sulfonylisothioureidotriazin-Derivate der Formel (VII)

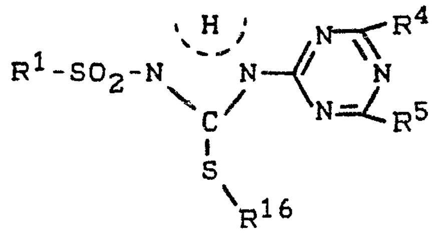

(VII)

in welcher

$R^1$, $R^4$ und $R^5$ die oben angegebenen Bedeutungen haben und

$R^{16}$ für $C_1$-$C_4$-Alkyl oder Benzyl steht,

mit Aminoverbindungen der Formel (IV)

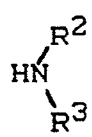

(IV)

in welcher

$R^2$ für Wasserstoff oder $C_1$-$C_4$-Alkyl steht und $R^3$ die oben angegebene Bedeutung hat,

gegebenenfalls in Gegenwart von Verdünnungsmitteln umsetzt;

oder

(f) für den Fall, daß M für ein Metalläquivalent steht, wenn man die nach den oben unter (a), (b), (c), (d) und (e) angegebenen Verfahren erhältlichen Verbindungen der Formel (I), in welcher M für Wasserstoff steht und $R^1$, $R^2$, $R^3$, $R^4$ und $R^5$ die oben angegebenen Bedeutungen haben,

mit Metallhydroxiden, -hydriden oder -alkanolaten oder mit metallorganischen Verbindungen, gegebenenfalls in Gegenwart von Verdünnungsmitteln umsetzt;

oder

(g) für den Fall, daß 1:1-Addukte von Verbindungen der Formel (I) mit starken Säuren herzustellen sind,

wenn man Verbindungen der Formel (I), in welcher M für Wasserstoff steht und $R^1$, $R^2$, $R^3$, $R^4$ und $R^5$ die oben angegebenen Bedeutungen haben,

mit starken Säuren, gegebenenfalls in Gegenwart von Verdünnungsmitteln umsetzt.

Die neuen Sulfonylguanidinotriazin-Derivate der Formel (I) und ihre Addukte mit starken Säuren zeichnen sich durch eine starke herbizide Wirksamkeit aus.

Überraschenderweise zeigen die neuen Verbindungen der Formel (I) erheblich bessere herbizide Wirkung als vorbekannte Guanidine gleicher Wirkungsrichtung.

Gegenstand der Erfindung sind vorzugsweise Verbindungen der Formel (I), in welcher

M für Wasserstoff oder ein Natrium-, Kalium- oder Calcium-äquivalent steht,

$R^1$ für einen gegebenenfalls substituierten Phenylrest

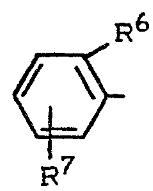

steht, worin

$R^6$ und $R^7$ gleich oder verschieden sind und für Wasserstoff, Fluor, Chlor, Brom, Cyano, Nitro, Methyl [welches gegebenenfalls durch Fluor, Chlor, Cyano, $C_1$-$C_3$-Alkoxy-carbonyl, $C_1$-$C_3$-Alkoxy, $C_1$-$C_3$-Alkyl-

thio, $C_1$-$C_3$-Alkylsulfinyl oder $C_1$-$C_3$-Alkyl sulfonyl substituiert ist], für $C_1$-$C_2$-Alk - oxy [welches gegebenenfalls durch Fluor, Chlor, Cyano, $C_1$-$C_3$-Alkoxy-carbonyl, $C_1$-$C_3$-Alkoxy, $C_1$-$C_3$-Alkylthio, $C_1$-$C_3$-Alkylsulfinyl oder $C_1$-$C_3$-Alkylsulfonyl substituiert ist], für $C_1$-$C_3$-Alkylthio, $C_1$-$C_3$-Alkylsulfinyl oder $C_1$-$C_3$-Alkylsulfonyl [welche gegebenenfalls durch Fluor, Chlor, Cyano oder $C_1$-$C_3$-Alkoxy-carbonyl substituiert sind], für Di-($C_1$-$C_3$-alkyl)-aminosulfonyl, $C_1$-$C_3$-Alkoxy-methylaminosulfonyl, Phenyl, Phenoxy, für $C_1$-$C_4$-Alkoxycarbonyl [welches gegebenenfalls durch Fluor, Chlor, Brom, Cyano oder $C_1$-$C_3$-Alkoxy substituiert ist], $C_3$-$C_6$-Cycloalkyloxycarbonyl, $C_1$-$C_4$- Alkylthio-carbonyl, Di-($C_1$-$C_3$-alkyl)-aminocarbonyl, $C_1$-$C_3$-Alkoxy-methylamino-carbonyl oder für $C_2$-$C_4$-Alkenyl [welches gegebenenfalls durch Fluor, Chlor, Cyano oder $C_1$-$C_3$-Alkoxy-carbonyl substituiert ist] stehen;

in welcher weiter

$R^1$ für einen gegebenenfalls substituierten Benzylrest

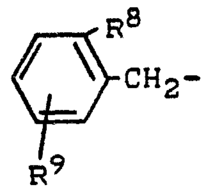 steht, worin

$R^8$ und $R^9$ gleich oder verschieden sind und für Wasserstoff, Fluor, Chlor, Cyano, Nitro, Methyl, Methoxy oder $C_1$-$C_3$-Alkoxy-carbonyl stehen;

in welcher weiter

$R^2$ für Wasserstoff, Methyl oder einen Sulfonylrest

$R^{10}$-$SO_2$- steht, worin

$R^{10}$ für $C_1$-$C_8$-Alkyl [welches gegebenfalls durch Fluor, Chlor oder Cyano substituiert ist], für $C_1$-$C_8$-Alkoxy [welches gegebenenfalls durch Fluor, Chlor oder Cyano substituiert ist], für $C_2$-$C_8$-Alkenyl [welches gegebenenfalls durch Fluor, Chlor, Cyano oder Phenyl substituiert ist], für $C_2$-$C_8$-Alkenyloxy [welches gegebenenfalls durch Fluor, Chlor, Cyano oder Phenyl substituiert ist],

für $C_1$-$C_6$-Alkylamino, $C_3$-$C_6$-Cycloalkylamino oder Di-($C_3$-$C_6$-alkyl)-amino [welche gegebenenfalls durch Fluor, Chlor, Cyano, Phenyl, Phenoxy oder $C_1$-$C_2$-Alkoxy substituiert sind], für $C_2$-$C_6$-Alkenylamino [welches gegebenenfalls durch Fluor, Chlor, Cyano oder Phenyl substituiert ist], für Phenylamino oder Benzylamino [welche gegebenenfalls durch Fluor, Chlor, Brom, $C_1$-$C_4$-Alkyl, Trifluormethyl, $C_1$-$C_3$-Alkoxy, $C_1$-$C_2$-Fluoralkoxy, $C_1$-$C_3$-Alkylthio, $C_1$-$C_3$-Alkylsulfinyl, $C_1$-$C_3$- Alkylsulfonyl, Cyano, Nitro und/oder $C_1$-$C_3$-Alkoxy-carbonyl substituiert sind], für Benzyl [welches gegebenenfalls durch Fluor, Chlor, Cyano, Nitro, Methyl, Methoxy und/oder $C_1$-$C_2$-Alkoxy-carbonyl substituiert ist] oder für einen gegebenenfalls substituierten Phenylrest

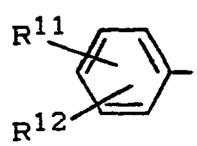 steht, worin

$R^{11}$ und $R^{12}$ gleich oder verschieden sind und die vorausgehend für $R^7$ und $R^8$ vorzugweise angegebenen Bedeutungen haben, jedoch nicht in jedem Einzelfall mit $R^7$ und $R^8$ identisch sind;

in welcher weiter

$R^3$ für den Rest $-O-R^{13}$ steht, worin

$R^{13}$ für $C_1$-$C_8$-Alkyl [welches gegebenenfalls durch Fluor oder Chlor substituiert ist], für $C_3$-$C_6$-Alkenyl [welches gegebenenfalls durch Chlor substituiert ist], für $C_1$-$C_3$-Alkoxy-carbonyl-$C_1$-$C_2$-alkyl, Phenyl, Phenylethyl oder Benzyl [welches gegebenenfalls durch Fluor, Chlor, Nitro, Methyl, Methoxy oder $C_1$-$C_2$-Alkoxy-carbonyl substituiert ist] steht;

in welcher weiter

$R^3$ für den Rest $-N(R^{14})(R^{15})$ steht, worin

$R^{14}$ für Wasserstoff oder Methyl steht und

$R^{15}$ für Wasserstoff, $C_1$-$C_3$-Alkyl [welches gegebenenfalls durch Fluor, Chlor, Cyano, $C_1$-$C_2$-Alkoxy oder $C_1$-$C_2$-Alkoxy-carbonyl substituiert ist], für $C_3$-$C_6$-Cycloalkyl, Phenyl, Benzyl oder Phenylethyl [welches gegebenenfalls durch Fluor, Chlor, Brom, Nitro, Cyano, $C_1$-$C_2$-Alkyl, $C_1$-$C_2$-Alkoxy oder $C_1$-$C_2$-Alkoxy-carbonyl substituiert ist], für Acetyl, Methoxycarbonyl, Benzolsulfonyl oder Toluolsulfonyl steht;

in welcher weiter

$R^4$ für Fluor, Chlor, Cyclopropyl, Methyl [welches gegebenenfalls durch Fluor und/oder Chlor substituiert ist], für $C_1$-$C_3$-Alkoxy [welches gegebenenfalls durch Fluor und/oder Chlor substituiert ist], für $C_1$-$C_3$-Alkylthio [welches gegebenenfalls durch Fluor und/oder Chlor substituiert ist] oder für $C_1$-$C_3$-Alkyl- oder Di-($C_1$-$C_3$-alkyl)-amino [welche gegebenenfalls durch Fluor substituiert sind] steht und

$R^5$ für Fluor, Chlor, Cyclopropyl, Methyl [welches gegebenenfalls durch Fluor und/oder Chlor substituiert ist], für $C_1$-$C_3$-Alkoxy [welches gegebenenfalls durch Fluor und/oder Chlor substituiert ist], für $C_1$-$C_3$-Alkylthio [welches gegebenenfalls durch Fluor und/oder Chlor substituiert ist] oder für $C_1$-$C_3$-Alkyl- oder Di-($C_1$-$C_3$-alkyl)-amino [welche gegebenenfalls durch Fluor substituiert sind] steht;

wobei die oben einzeln genannten Verbindungen ausgenommen sind.

Gegenstand der Erfindung sind vorzugsweise ferner 1:1-Addukte von Verbindungen der Formel (I) - wie vorausgehend definiert, wobei M für Wasserstoff steht - mit Halogenwasserstoffsäuren, wie Hydrogenchlorid, Hydrogenbromid und Hydrogeniodid, mit Schwefelsäure, Trifluor-

essigsäure, mit gegebenenfalls durch Fluor oder Chlor substituierten Alkansulfonsäuren mit bis zu 4 Kohlenstoffatomen oder auch mit Benzol- oder Naphthalin-sulfonsäuren, welche gegebenenfalls durch Fluor, Chlor, Brom oder Methyl substituiert sind.

Gegenstand der Erfindung sind insbesondere Verbindungen der Formel (I), in welcher

M für Wasserstoff oder ein Natrium-, Kalium- oder Calcium-äquivalent steht,

$R^1$ für einen substituierten Phenylrest

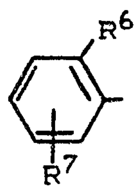

steht, worin

$R^6$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Diethylaminosulfonyl, Methoxymethylamino-sulfonyl, Dimethylaminosulfonyl, Phenyl, $C_1$-$C_4$-Alkoxy-carbonyl, Difluormethylthio oder Trifluormethylthio steht und

$R^7$ für Wasserstoff steht;

in welcher weiter

$R^2$ für Wasserstoff, Methyl oder einen Sulfonylrest

$R^{10}-SO_2-$ steht, worin

$R^{10}$ für $C_1$-$C_4$-Alkyl [welches gegebenenfalls durch Fluor oder Chlor substituiert ist], für $C_1$-$C_4$-Alkylamino oder Di-($C_1$-$C_2$-alkyl)-amino [welche gegebenenfalls durch Fluor substituiert sind] oder für einen gegebenfalls substituierten Phenylrest

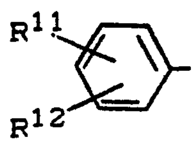

steht, worin

$R^{11}$ und $R^{12}$ gleich oder verschieden sind und für Wasserstoff, Fluor, Chlor, Brom, Cyano, Nitro, Methyl, Trifluormethyl, $C_1$- $C_2$-Alkoxy [welches gegebenenfalls durch Fluor, Chlor, Cyano oder $C_1$-$C_3$-Alkoxycarbonyl substituiert ist], $C_1$-$C_3$-Alkylthio, $C_1$-$C_3$-Alkylsulfinyl oder $C_1$-$C_3$-Alkylsulfonyl [welche gegebenenfalls durch Fluor und/oder Chlor substituiert sind], Dimethylaminosulfonyl, Diethylaminosulfonyl, N-Methoxy-N-methylaminosulfonyl, Phenyl oder $C_1$-$C_4$-Alkoxy-carbonyl [welches gegebe-

nenfalls durch Fluor, Chlor, Cyano oder $C_1$- $C_2$-Alkoxy substituiert ist] stehen;

in welcher weiter

$R^3$ für den Rest $-O-R^{13}$ steht, worin

$R^{13}$ für $C_1$-$C_4$-Alkyl, $C_3$-$C_4$-Alkenyl, $C_1$-$C_4$-Alkoxy-carbonyl-methyl, Phenyl, Phenylethyl oder Benzyl [welches gegebenenfalls durch Fluor, Chlor, Nitro, Cyano, Methyl, Methoxy oder Methoxycarbonyl substituiert ist] steht,

in welcher weiter

$R^3$ für den Rest 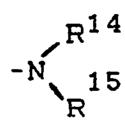 steht, worin

$R^{14}$ für Wasserstoff oder Methyl steht und

$R^{15}$ für Wasserstoff, $C_1$-$C_3$-Alkyl, $C_3$-$C_6$-Cycloalkyl, Phenyl, Acetyl, Methoxycarbonyl, Benzolsulfonyl oder Toluolsulfonyl steht;

in welcher weiter

$R^4$ für Methyl, Methoxy, Ethoxy, Methylthio, Ethylthio, Methylamino, Ethylamino, Dimethylamino oder Diethylamino steht und

$R^5$ für Methyl, Methoxy, Ethoxy, Methylthio, Ethylthio, Methylamino, Ethylamino, Dimethylamino oder Diethylamino steht,

- wobei die oben einzeln genannten Verbindungen ausgenommen sind -

sowie - für den Fall daß M für Wasserstoff steht - die 1:1-Addukte der vorausgehend definierten Verbindungen mit Salzsäure, Schwefelsäure, Trifluoressigsäure, Methansulfonsäure, Benzolsulfonsäure und Toluolsulfonsäure.

Die oben einzeln genannten Verbindungen sind auch beim bevorzugten bzw. besonders bevorzugten Bereich ausgenommen.

Verwendet man beispielsweise für die Verfahrensvariante (a) 2-Fluor-benzolsulfonsäurechlorid und N'-(4-Methoxy-6-methyl-s-triazin-2-yl)-N"-ethoxy-guanidin als Ausgangsstoffe, so kann der Reaktionsablauf durch das folgende Formelschema skizziert werden:

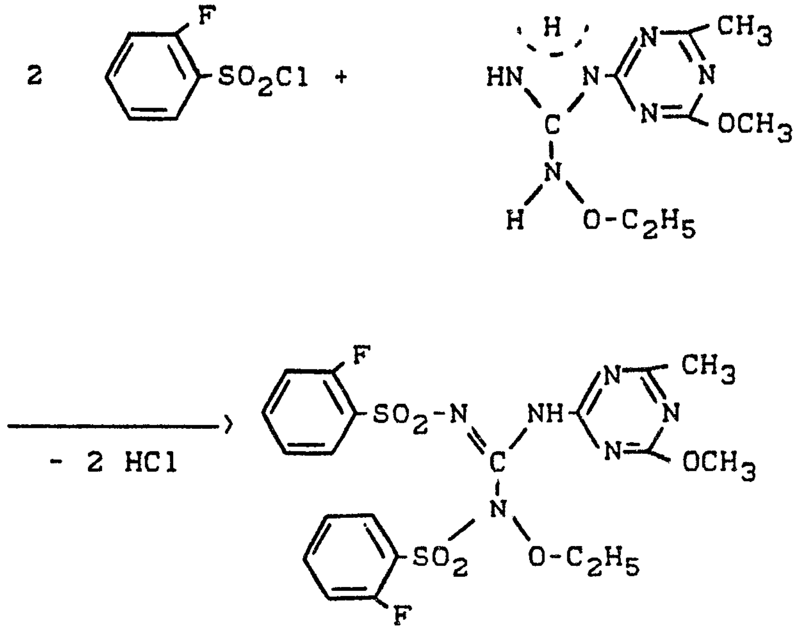


Verwendet man beispielsweise für die Verfahrensvariante (b) N'-(4-Methoxy-6-methylthio-s-triazin-2-yl)-N"-methoxy-N",N'''-bis-(2-trifluormethyl-benzolsulfonyl)-guanidin und Phenylhydrazin als Ausgangsstoffe, so kann der Reaktionsablauf durch das folgende Formelschema skizziert werden:

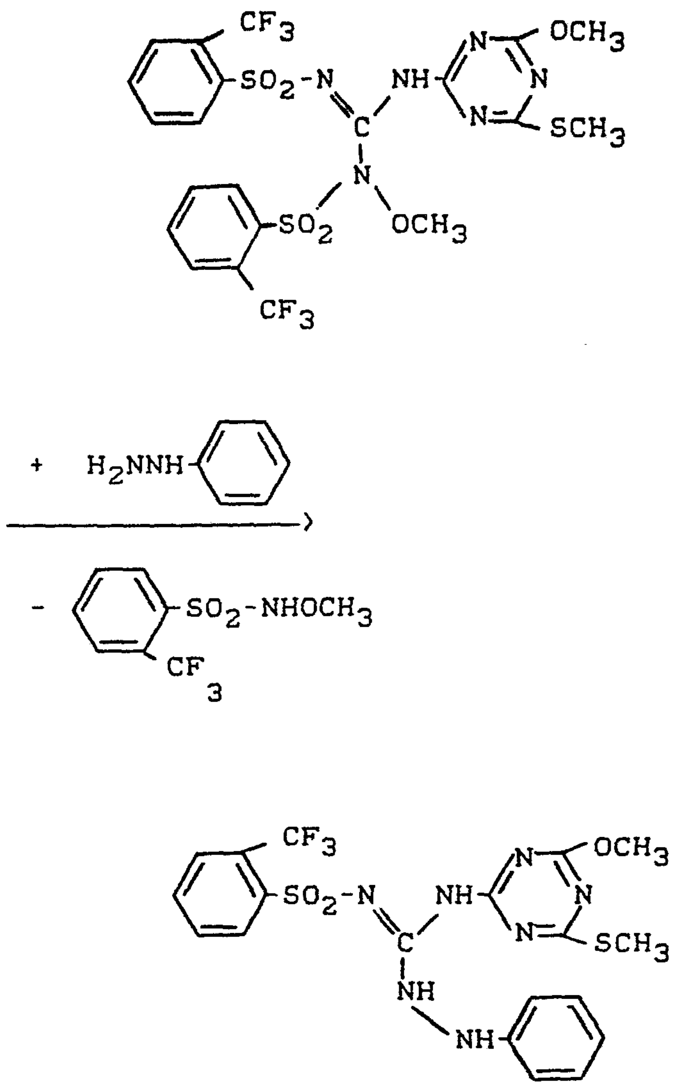

Verwendet man beispielsweise für die Verfahrensvariante (c) N'-(4,6-Dimethoxy-s-triazin-2-yl)-N"-benzyloxy-N'''-(2-chlor-phenylmethylsulfonyl)-guanidin und Trifluormethan-sulfonsäurechlorid als Ausgangsstoffe, so kann der Reaktionsablauf durch das folgende Formelschema skizziert werden:

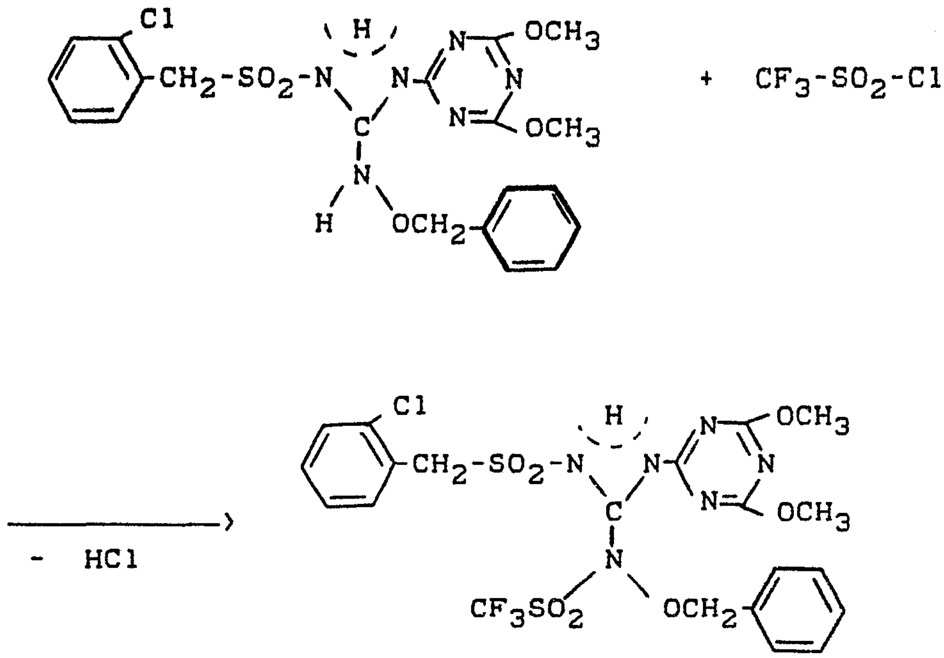


Verwendet man beispielsweise für die Verfahrensvariante (d) N'-(4-Ethoxy-6-methyl-s-triazin-2-yl)-N"-methoxy-N"-(2-ethoxycarbonyl-benzolsulfonyl)-guanidin und 2-Dimethylaminosulfonyl-benzolsulfonsäurechlorid als Ausgangsstoffe, so kann der Reaktionsablauf durch das folgende Formelschema skizziert werden:

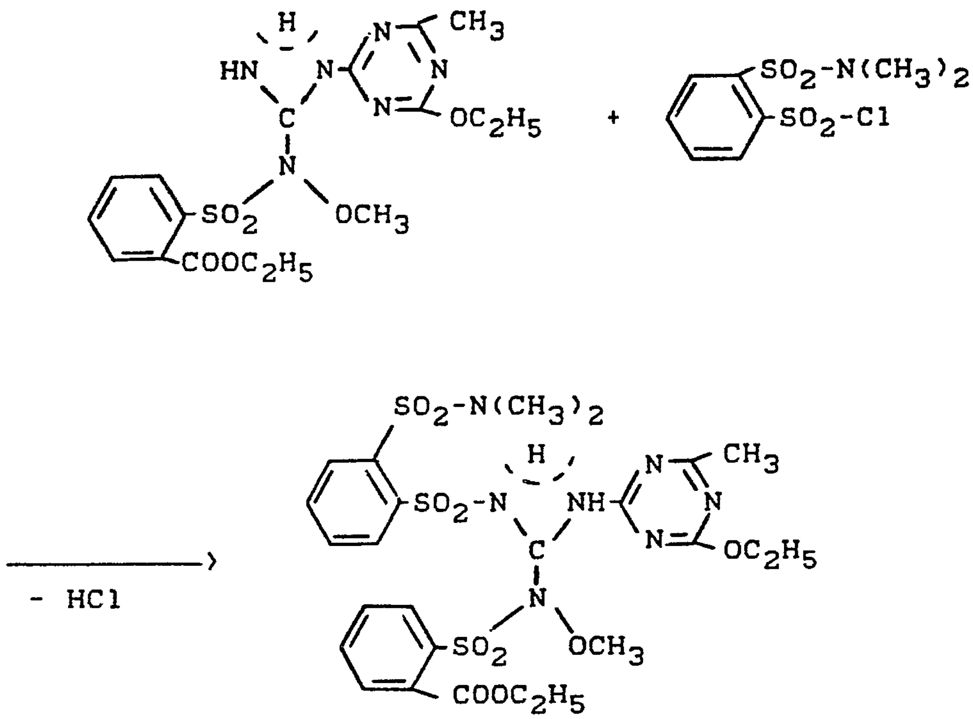


Verwendet man beispielsweise für die Verfahrensvariante (e) N'-(4-Methoxy-6-methyl-s-triazin-2-yl)-N"-(2-methylbenzolsulfonyl)-S-methyl-isothioharnstoff und N,N-Dimethylhydrazin als Ausgangsstoffe, so kann der Reaktionsablauf durch das folgende Formelschema skizziert werden:

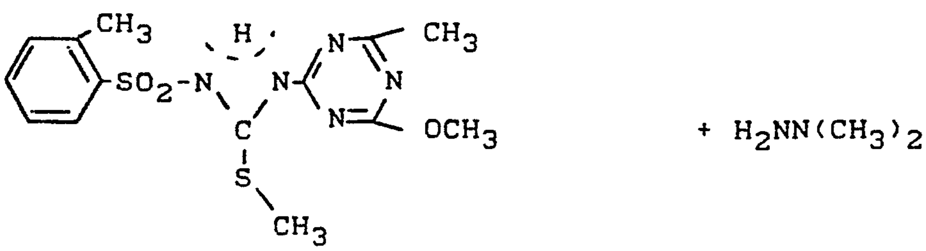

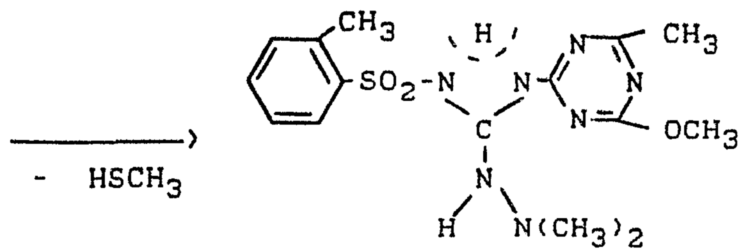


Verwendet man beispielsweise für die Verfahrensvariante (f) N'-(4,6-Dimethoxy-s-triazin-2-yl)-N"-isopropoxy-N'''-(2-methoxycarbonylphenylmethylsulfonyl)-guanidin und Kaliumethanolat als Ausgangsstoffe, so kann der Reaktionsablauf durch das folgende Formelschema skizziert werden:

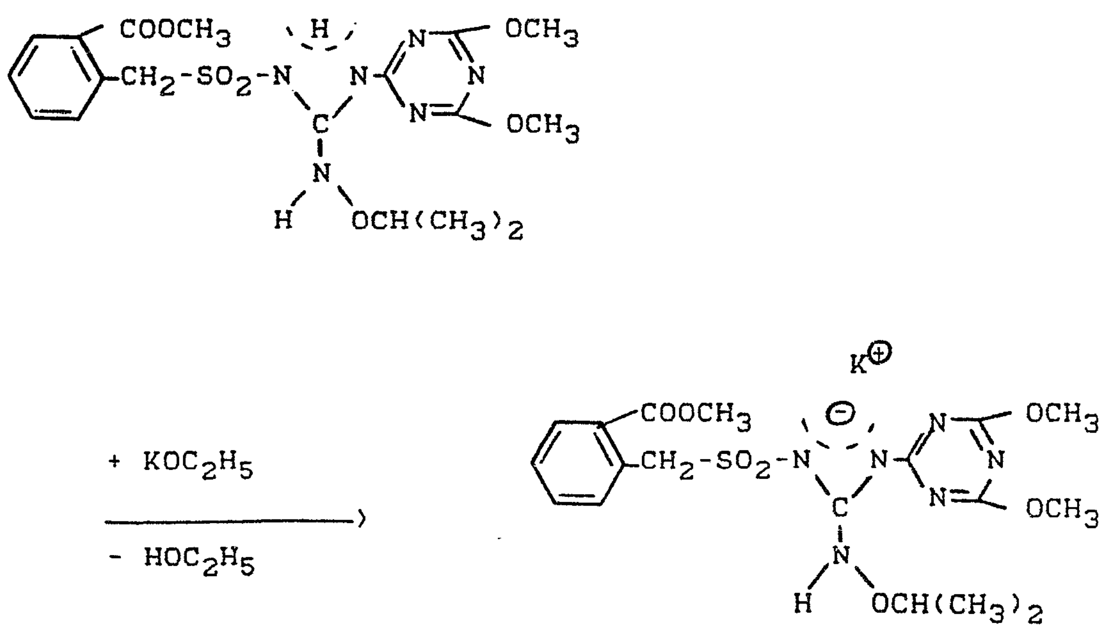

Verwendet man beispielsweise für die Verfahrensvariante (g) N'-(4-Methylamino-6-methylthio-s-triazin-2-yl)-N"-methoxy-N"-(2-chlor-benzolsulfonyl)-N'''-(2-difluormethoxy-benzolsulfonyl)-guanidin und Methansulfonsäure als Ausgangsstoffe, so kann der Reaktionsablauf durch das folgende Formelschema skizziert werden:

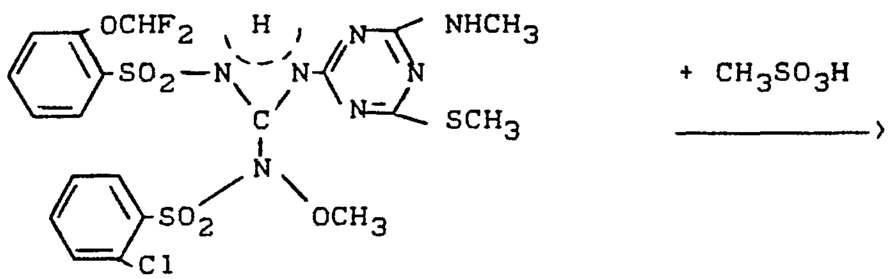


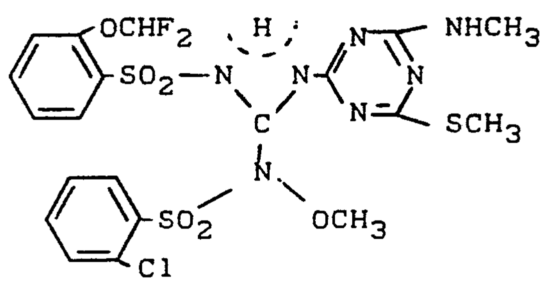

x $CH_3SO_3H$

Die als Ausgangsstoffe für die Verfahrensvariante (a) zu verwendenden Guanidinotriazin-Derivate sind durch die Formel (II) allgemein definiert. In Formel (II) haben $R^3$, $R^4$ und $R^5$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Ausgangsstoffe der Formel (II) seien beispielsweise genannt:

N'-(4,6-Dimethyl-s-triazin-2-yl)-, N'-(4-Methoxy-6-methyl-s-triazin-2-yl)-, N'-(4-Ethoxy-6-methyl-s-triazin-2-yl)-, N'-(4,6-Dimethoxy-s-triazin-2-yl)-, N'-(4,6-Diethoxy-s-triazin-2-yl)-, N'-(4-Ethoxy-6-methoxy-s-triazin-2-yl)-, N'-(4-Methyl-6-methylthio-s-triazin-2-yl)-, N'-(4-Ethylthio-6-methyl-s-triazin-2-yl)-, N'(4-Methoxy-6-methylthio-s-triazin-2-yl)-, N'-(4-Ethoxy-6-methylthio-s-triazin-2-yl)-, N'-(4-Ethylthio-6-methoxy-s-triazin-2-yl)-, N'-(4-Ethoxy-6-ethylthio-s-triazin-2-yl)-, N'-(4,6-Bis-methylthio-s-triazin-2-yl)-, N'-(4,6-Bis-ethylthio-s-triazin-2-yl)-, N'-(4-Methyl-6-methylamino-s-triazin-2-yl)-, N'-(4-Ethylamino-6-methyl-s-triazin-2-yl)-, N'-(4-Dimethylamino-6-methyl-s-triazin-2-yl)-, N'-(4-Diethylamino-6-methyl-s-triazin-2-yl)-, N'-(4-Methoxy-6-methylamino-s-triazin-2-yl)-, N'-(4-Ethylamino-6-methoxy-s-triazin-2-yl)-, N'-(4-Dimethylamino-6-methoxy-s-triazin-2-yl)-, N'-(4-Diethylamino-6-methoxy-s-triazin-2-yl)-, N'-(4-Ethoxy-6-methylamino-s-triazin-2-yl)-, N'-(4-Ethoxy-6-ethylamino-s-triazin-2-yl)-, N'-(4-Dimethylamino-6-ethoxy-s-triazin-2-yl)-, N'-(4-Diethylamino-6-ethoxy-s-triazin-2-yl)-, N'-(4-Methylamino-6-methylthio-s-triazin-2-yl)-, N'-(4-Ethylamino-6-methylthio-s-triazin-2-yl)-, N'-(4-Dimethylamino-6-methylthio-s-triazin-2-yl)-, N'-(4-Diethylamino-6-methylthio-s-triazin-2-yl)-, N'-(4-Ethylthio-6-methylamino-s-triazin-2-yl)-, N'-(4-Dimethylamino-6-ethylthio-s-triazin-2-yl)- und N'-(4-Diethylamino-6-ethylthio-s-triazin-2-yl)-N"-methoxy-guanidin, -N"-ethoxy-guanidin, -N"-propoxy-guanidin, -N"-isopropoxy-guanidin, -N"-butoxy-guanidin, -N"-isobutoxy-guanidin, -N"-sec.-butoxy-guanidin, -N"-pentoxy-guanidin, -N"-isopentoxy-guanidin, -N"-hexyloxy-guanidin, -N"-octyloxy-guanidin, -N"-allyloxy-guanidin, -N"-(2-chlor-ethoxy)-guanidin, -N"-(2-fluor-ethoxy)-guanidin, -N"-(2-chlor-propoxy)-guanidin, -N"-(2-fluor-propoxy)-guanidin, -N"-(3-chlor-propoxy)-guanidin, -N"-(4-chlor-butoxy)-guanidin, -N"-methoxycarbonylmethoxy-guanidin, -N"-ethoxycarbonylmethoxy-guanidin, -N"-(1-methoxy-carbonyl-ethoxy)-guanidin, -N"-(1-ethoxycarbonyl-ethoxy)-guanidin, -N"-dimethylamino-carbonylmethoxy-guanidin, -N"-(2-phenyl-ethoxy)-guanidin, -N"-phenoxy-guanidin, -N"-(4-methylbenzyloxy)-guanidin, -N"-(4-fluor-benzyloxy)-guanidin, -N"-(4-chlor-benzyloxy)-guanidin, -N"-(4-nitro-benzyloxy)-guanidin, -N"-(2,6-dichlor-benzyloxy)-guanidin, -N"-(4-methoxycarbonylbenzyloxy)-guanidin und -N"-(4-ethoxycarbonyl-benzyloxy)-guanidin.

Die Ausgangsstoffe der Formel (II) sind bekannt (vgl. DE-OS 3 334 455).

Man erhält die Guanidinotriazin-Derivate der Formel (II), wenn man Cyanaminotriazin-Derivate der Formel (VIII)

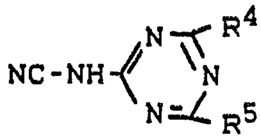

(VIII)

in welcher

$R^4$ und $R^5$ die oben angegebenen Bedeutungen haben,

mit Aminoverbindungen der Formel (IX)

$H_2N-R^3$ (IX)

in welcher

$R^3$ die oben angegebene Bedeutung hat,

bzw. mit deren Hydrochloriden, gegebenenfalls in Gegenwart von Verdünnungsmitteln, wie z.B. Ethanol, Isopropanol oder Butanol, bei Temperaturen zwischen 20°C und 150°C, vorzugsweise zwischen 50°C und 120°C, umsetzt und gegebenenfalls die Umsetzungsprodukte mit Säureakzeptoren, wie z.B. Ammoniak, Natronlauge oder Soda behandelt.

Die Cyanaminotriazin-Derivate der Formel (VIII) sind bekannt (vgl. DE-OS 3 334 455).

Man erhält die Verbindungen der Formel (VIII) im wesentlichen nach folgenden Synthesewegen:

(a[1]) Durch Umsetzungen von Alkalimetall- oder Erdalkalimetall-Salzen von Cyanamid - wie z.B. Natriumcyanamid oder Calciumcyanamid - mit Chlortriazinen der Formel (X)

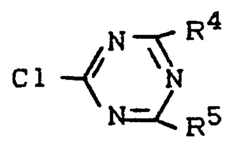 (X)

in welcher

$R^4$ und $R^5$ die oben angegebenen Bedeutungen haben,

gegebenenfalls in Gegenwart von inerten Verdünnungsmitteln, wie z.B. Ethanol, Aceton, Acetonitril oder Dimethylformamid, bei Temperaturen zwischen 0°C und 100°C. Nach Einengen und Auflösen des Rückstandes in Wasser können die Cyanaminotriazin-Derivate der Formel (VIII) durch Ansäuern, z.B. mit Salzsäure, ausgefällt und durch Absaugen isoliert werden.

Alternativ erhält man Cyanaminotriazin-Derivate der Formel (VIII), wenn man

(a[2]) Aminotriazine der Formel (XI)

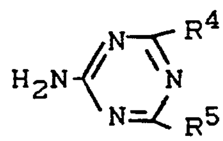 (XI)

in welcher

$R^4$ und $R^5$ die oben angegebene Bedeutung haben,

mit Carbonylisothiocyanaten der Formel (XII)

$$R^{17}-\overset{O}{\overset{\|}{C}}-N=C=S \qquad (XII)$$

in welcher

$R^{17}$ für Ethoxy oder Phenyl steht,

gegebenenfalls in Gegenwart von inerten Verdünnungsmitteln, wie z.B. Aceton, Acetonitril oder Toluol, bei Temperaturen zwischen 0°C und 100°C umsetzt, die hierbei gebildeten Carbonylthioharnstoffe der Formel (XIII)

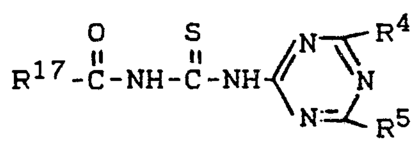

(XIII)

in welcher

$R^4$, $R^5$ und $R^{17}$ die oben angegebenen Bedeutungen haben,

gegebenenfalls nach Einengen durch Absaugen isoliert und mit wässrigen Alkalimetall- oder Erdalkalimetallhydroxidlösungen, wie z.B. Natronlauge, gegebenenfalls in Gegenwart eines organischen Lösungsmittels, wie z.B. Tetrahydrofuran oder Dioxan, bei Temperaturen zwischen 0°C und 120°C umsetzt, und die nach Ansäuern, z.B. mit Salzsäure, kristallin erhaltenen Thioharnstoffe der Formel (XIV)

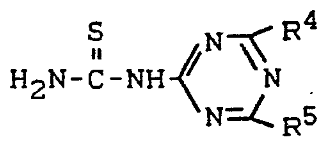 (XIV)

in welcher

$R^4$ und $R^5$ die oben angegebenen Bedeutungen haben,

durch Absaugen isoliert und mit Metallverbindungen, welche Schwefelwasserstoff binden können, wie z.B. mit Blei(II)-acetat, Kupfer(II)-acetat, Quecksilber-(II)-acetat oder Eisen(II)-acetat, in Gegenwart von wässrigen Alkalimetall- oder Erdalkalimetall-hydroxidlösungen, wie z.B. Natronlauge, bei Temperaturen zwischen 20°C und 100°C umsetzt, nach Ende der Umsetzung filtriert und das Filtrat mit einer Säure, wie z.B. Essigsäure, ansäuert.

Die hierbei kristallin anfallenden Produkte der Formel (VIII) können durch Absaugen isoliert werden.

Die als Ausgangsstoffe für die vorausgehend unter ($a^1$) und ($a^2$) beschriebenen Herstellungsverfahren für die Cyanaminotriazin-Derivate der Formel (VIII) zu verwendenden Chlortriazine der Formel (X), Aminotriazine der Formel (XI) und Carbonylisothiocyanate der Formel (XII) sind bekannt und/oder können nach an sich bekannten Verfahren hergestellt werden (vgl. US-PS 3 154 547 und US-PS 4 160 037).

In einem weiteren Alternativverfahren erhält man Cyanaminotriazin-Derivate der Formel (VIII), wenn man

($a^3$) Dichlortriazine der Formel (Xa)

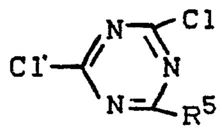

(Xa)

in welcher

$R^5$ die oben angegebene Bedeutung hat,

mit Alkalimetallsalzen oder Erdalkalimetallsalzen von Cyanamid, wie z.B. mit Dinatriumcyanamid, in Gegenwart eines Verdünnungsmittels, wie z.B. Wasser, bei Temperaturen zwischen -10°C und +50°C umsetzt und das hierbei anfallende Metallsalz des Monochlor-cyanaminotriazins der Formel (VIIIa)

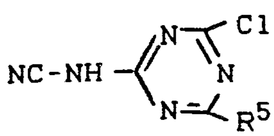

(VIIIa)

in welcher

$R^5$ die oben angegebene Bedeutung hat,

mit einer Säure, wie z.B. Salzsäure, in das freie Monochlor-cyanaminotriazin der Formel (VIIIa) umwandelt.

Aus den Monochlor-cyanaminotriazinen der Formel (VIIIa) können durch Umsetzung mit Alkoholen bzw. Alkanthiolen oder mit Alkalimetallsalzen dieser Verbindungen bzw. mit Monoalkyl- oder Dialkylaminen die entsprechenden Cyanaminotriazine der Formel (VIII), in welcher $R^4$ für Alkoxy, Alkylthio, Alkylamino oder Dialkylamino steht, hergestellt werden.

In einem weiteren Alternativverfahren erhält man Cyanaminotriazin-Derivate der Formel (VIII), wenn man

($a^4$) Aminotriazine der Formel (XI)

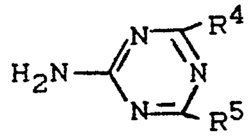

(XI)

in welcher

$R^4$ und $R^5$ die oben angegebene Bedeutung haben,

mit Chlorcyan (Cl-CN) in Gegenwart einer Base, wie z.B. Butyllithium, und in Gegenwart eines Verdünnungsmittels, wie Tetrahydrofuran, bei Temperaturen

zwischen 0°C und 50°C umsetzt, nach Ende der Umsetzung einengt, mit Wasser verdünnt und ansäuert.

Die weiter als Ausgangsstoffe zu verwendenden Aminoverbindungen der Formel (IX) sind ebenfalls bekannt und können nach an sich bekannten Verfahren hergestellt werden (vgl. Chem. Pharm. Bull. 15 (1967), 345; Bull. Soc. Chim. France 1958, 664; Synthesis 1976, 682; J. Chem. Soc. 1930, 228 und Helv. Chim. Acta 45 (1962), 1387).

Die weiter als Ausgangsstoffe für die Verfahrensvariante (a) zu verwendenden Sulfonsäurechloride sind durch die Formel (III) allgemein definiert. In Formel (III) hat $R^1$ vorzugsweise bzw. insbesondere die gleiche Bedeutung, wie sie oben im Rahmen der Subsituentendefinition der Formel (I) vorzugsweise bzw. als besonders bevorzugt genannt ist.

Als Ausgangsstoffe der Formel (III) seien beispielsweise genannt:
2-Chlor-, 3-Chlor-, 4-Chlor-, 2,5-Dichlor-, 2-Fluor-, 4-Fluor-, 2-Brom-, 4-Brom-, 2-Nitro-, 4-Nitro-, 2-Cyano-, 2-Methyl-, 4-Methyl-, 2-Chlormethyl-, 2-Trifluormethyl-, 2-Methoxy-, 2-Difluormethoxy-, 2-Trifluormethoxy-, 2-Methylthio-, 2-Methylthiomethyl-, 2-Methylsulfinylmethyl-, 2-Methylsulfonylmethyl-, 2-Dimethylaminosulfonyl-, 2-Diethylaminosulfonyl-, 2-Phenyl-, 2-Methoxycarbonyl-, 2-Ethoxycarbonyl-, 2-Propoxycarbonyl-, 2-Isopropoxycarbonyl-, 2-Butoxycarbonyl-, 2-Dimethylaminocarbonyl-, 2-Diethylaminocarbonyl-, 2-Phenoxy-, 2-Methyl-5-chlor-, 2-Chlor-5-trifluormethyl-, 2-Methylsulfonyl-, 2-Ethylsulfonyl-, 2-Chlor-4-trifluormethoxy-, 3-Chlor-4-trifluormethoxy-, 2-Trifluormethoxy-5-chlor- und 3,5-Dichlor-benzolsulfonsäurechlorid; (2-Chlor-phenyl)-, (2-Cyano-phenyl)- und (2-Methoxy-carbonyl-phenyl)-methansulfonsäurechlorid; ferner Methan-, Chlormethan-, Trifluormethan-, Ethan-, 2-Chlorethan-, Ethen-, Propan-, Butan-, Perfluorbutan- und Perfluoroctansulfonsäurechlorid; Methyl-, Ethyl-, Propyl-, Isopropyl-, Cyclohexyl-, Dimethyl- und Diethylsulfamidsäurechlorid.

Die Sulfonsäurechloride der Formel (III) sind bekannt und/oder können nach an sich bekannten Verfahren hergestellt werden (vgl. J. Org. Chem. 33 (1968), 2104; J. Org. Chem. 25 (1960), 1824; DE-AS 2 308 262; EP-OS 23 140, 23 141, 23 422, 35 893, 48 143, 51 466, 64 322, 70 041, 44 808, 44 809; US-PS 2 929 820, 4 282 242, 4 348 220 und 4 372 778 sowie Angew. Chem. 93 (1981), 151).

Man erhält diese Verbindungen im wesentlichen nach folgenden zwei Synthesewegen:

(1) durch Umsetzung der entsprechenden Sulfonsäuren $R^1-SO_3-H$ bzw. von deren Alkalimetall- oder Erdalkalimetall-Salzen mit Halogenierungsmitteln, wie z.B.Phosphor(V)chlorid (Phosphorpentachlorid), Phosphorylchlorid (Phosphoroxychlorid), Thionylchlorid, Phosgen oder Benzotrichlorid, gegebenenfalls in Gegenwart von Katalysatoren, wie z.B. Pyridin oder Dimethylformamid, und gegebenenfalls unter Verwendung von inerten Verdünnungsmitteln, wie z.B. Methylenchlorid, Chloroform, Acetonitril, Chlorbenzol und/oder Sulfolan bei Temperaturen zwischen -20°C und +150°C, vorzugsweise zwischen 0°C und +100°C; nach Verdünnen mit Wasser können die Sulfonsäurechloride - soweit sie kristallin anfallen - durch Absaugen isoliert werden oder durch Extrahieren mit einem mit Wasser nicht mischbaren Lösungsmittel, wie z.B. Methylenchlorid, Diethylether oder Hexan, Waschen und Trocknen der Extrakte, Einengen und Umkristallisieren bzw. Destillieren gereinigt werden; oder auch

(2) auf an sich bekannte Weise (vgl. J. Org. Chem. 25 (1960), 1824; DE-OS 2 308 262 und EP-OS 59 241) durch Umsetzung entsprechender Aminoverbindungen $R^1-NH_2$ mit Natriumnitrit und Salzsäure, gegebenenfalls in Gegenwart von Essigsäure, bei Temperaturen zwischen -10°C und +20°C, vorzugsweise zwischen -5°C und +10°C, und anschließend (in situ) mit Schwefeldioxid oder einem Salz der schwefeligen Säure, wie z.B. Natriumhydrogensulfit in Gegenwart einer Kupferverbindung, wie z.B. Kupferchlorid oder Kupfersulfat, als Katalysator bei Temperaturen zwischen 0°C und 80°C, vorzugsweise zwischen 10°C und 60°C.

Die Aufarbeitung kann auf übliche Weise erfolgen: Bei Verdünnen mit Wasser fallen die Sulfonsäurechloride im allgemeinen kristallin an und können durch Absaugen isoliert werden. Sie können aber auch aus der wässrigen Dispersion mit einem mit Wasser praktisch nicht mischbaren Lösungsmittel, wie z.B. Methylenchlorid oder Diethylether, extrahiert, getrocknet und durch Vakuumdestillation gereinigt werden.

Die als Ausgangsstoffe für die Verfahrensvariante (b) zu verwendenden Sulfonylguanidinotriazin-Derivate sind durch die Formel (ID) allgemein definiert. In Formel (ID) haben $R^1$, $R^4$, $R^5$ und $R^{13}$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Verbindungen der Formel (ID) seien genannt:
N'-(4,6-Dimethoxy-s-triazin-2-yl)-N"-methoxy-N", N'''-bis-(2-trifluormethyl-benzolsulfonyl)-guanidin, N'-(4-Diethylamino-6-methoxy-s-triazin-2-yl)-N"-methoxy-N",N'''-bis-(2-chlor-benzolsulfonyl)-guanidin und N'-(4-Methoxy-6-methyl-s-triazin-2-yl)-N"-methoxy-N",N'''-bis-(2-chlor-benzolsulfonyl)-guanidin.

Die Verbindungen der Formel (ID) können nach dem oben unter (a) beschriebenen Verfahren hergestellt werden.

Die bei der Verfahrensvariante (b) als Ausgangsstoffe zu verwendenden Aminoverbindungen sind durch die Formel (IV) allgemein definiert. In Formel (IV) haben $R^2$ und $R^3$ vorzugsweise bzw. insbesondere die gleichen Bedeutungen, wie sie oben im Rahmen der Substituentendefinition der Formel (I) als bevorzugt bzw.als insbesondere bevorzugt angegeben sind.
Als Ausgangsstoffe der Formel (IV) seien beispielsweise genannt:

N,N-Dimethylhydrazin, Phenylhydrazin, O-Methyl-hydroxylamin, O-Ethyl-hydroxylamin, O,N-Dimethylhydroxylamin, O-Propyl-, O-Isopropyl-, O-Butyl-, O-Isobutyl-, O-sec.-Butyl-, O-Pentyl-, O-Isopentyl-, O-sec.-Pentyl-, O-Hexyl-, O-Isohexyl-, O-Heptyl-, O-Isoheptyl-, O-Octyl-, O-Isooctyl-, O-Allyl-, O-Crotyl-, O-(2-Chlor-ethyl)-, O-(2-Fluor-ethyl)-, O-(2-Chlor-propyl)-, O-(3-Chlor-propyl)-, O-(4-Chlor-butyl)-, O-Methoxycarbonylmethyl-, O-Ethoxycarbonylmethyl-, O-(1-Methoxycarbonyl)-ethyl-, O-(1-Ethoxycarbonyl)-ethyl-, O-Aminocarbonylmethyl-, O-(2-Phenyl-ethyl)-, O-Phenyl-, O-(4-Methyl-benzyl)-, O-(4-Fluor-benzyl)-, O-(4-Chlor-benzyl)-, O-(4-Nitro-benzyl)-, O-(2,6-Dichlor-benzyl)-, O-(4-Methoxycarbonyl-benzyl)- und O-(4-Ethoxycarbonyl-benzyl)-hydroxylamin.

Aminoverbindungen der Formel (IV) sind bekannt und können nach an sich bekannten Verfahren hergestellt werden (vgl. Chem. Pharm. Bull. 15 (1967), 345; Bull. Soc. Chim. France 1958, 664; Synthesis 1976, 682; J. Chem. Soc. 1930, 228 und Helv. Chim. Acta 45 (1962), 1387).

Die bei der Verfahrensvariante (c) als Ausgangsstoffe zu verwendenden Sulfonylguanidinotriazin-Derivate sind durch die Formel (IE) allgemein definiert. In Formel (IE) haben $R^1$, $R^3$, $R^4$ und $R^5$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Verbindungen der Formel (IE) seien genannt:

N'-(4,6-Dimethyl-s-triazin-2-yl)-, N'-(4-Methoxy-6-methyl-s-triazin-2-yl)-, N'-(4-Ethoxy-6-methyl-s-triazin-2-yl)-, N'-(4,6-Dimethoxy-s-triazin-2-yl)-, N'-(4,6-Diethoxy-s-triazin-2-yl)-, N'-(4-Ethoxy-6-methoxy-s-triazin-2-yl)-, N'-(4-Methyl-6-methylthio-s-triazin-2-yl)-, N'-(4-Ethylthio-6-methyl-s-triazin-2-yl)-, N'(4-Methoxy-6-methylthio-s-triazin-2-yl)-, N'-(4-Ethoxy-6-methylthio-s-triazin-2-yl)-, N'-(4-Ethylthio-6-methoxy-s-triazin-2-yl)-, N'-(4-Ethoxy-6-ethylthio-s-triazin-2-yl)-, N'-(4,6-Bis-methylthio-s-triazin-2-yl)-, N'-(4,6-Bis-ethylthio-s-triazin-2-yl)-, N'-(4-Methyl-6-methylamino-s-triazin-2-yl)-, N'-(4-Ethylamino-6-methyl-s-triazin-2-yl)-, N'-(4-Dimethylamino-6-methyl-s-triazin-2-yl)-, N'-(4-Diethylamino-6-methyl-s-triazin-2-yl)-, N'-(4-Methoxy-6-methylamino-s-triazin-2-yl)-, N'-(4-Ethylamino-6-methoxy-s-triazin-2-yl)-, N'-(4-Dimethylamino-6-methoxy-s-triazin-2-yl)-, N'-(4-Diethylamino-6-methoxy-s-triazin-2-yl)-, N'-(4-Ethoxy-6-methylamino-s-triazin-2-yl)-, N'-(4-Ethoxy-6-ethylamino-s-triazin-2-yl)-, N'-(4-Dimethylamino-6-ethoxy-s-triazin-2-yl)-, N'-(4-Diethylamino-6-ethoxy-s-triazin-2-yl)-, N'-(4- Methylamino 6-methylthio-s-triazin-2-yl)-, N'-(4-Ethylamino-6-methyl thio-s-triazin-2-yl)-, N'-(4-Dimethylamino-6-methylthio-s-triazin-2-yl)-, N'-(4-Diethylamino-6-methylthio-s-triazin-2-yl)-, N'-(4-Ethylthio-6-methylamino-s-triazin-2-yl)-, N'-(4-Dimethylamino-6-ethylthio-s-triazin-2-yl)- und N'-(4-Diethylamino-6-ethylthio-s-triazin-2-yl)-N"-methoxy-, -N"-ethoxy-, -N"-propoxy-, -N"-isopropoxy-, -N"-butoxy-, -N"-isobutoxy-, -N"-sec-.butoxy-, -N"-pentoxy-, -N"-hexyloxy-, -N"-octyloxy-, -N"-allyloxy-, -N"-crotyloxy-, -N"-(3-chlor-propoxy)-, -N"-methoxycarbonylmethoxy-, -N"-ethoxycarbonylmethoxy, -N"-(1-methoxycarbonylethoxy)-, -N"-(1-ethoxycarbonyl-ethoxy)-,

-N"-(2-phenylethoxy)-, -N"-phenoxy-, -N"-benzyloxy-, -N"-(4-methyl-benzyloxy)-, N"-(4-fluor-benzyloxy)-, -N"-(4-chlor-benzyloxy)-, -N"-(4-nitro-benzyloxy)-, -N"-(2,6-dichlor-benzyloxy)-, -N"-(4-methoxycarbonyl-benzyloxy)- und -N"-(4-ethoxycarbonyl-benzyloxy)-, -N'''-benzolsulfonyl-, -N'''-(2-chlor-benzolsulfonyl)-, -N'''-(3-chlor-benzolsulfonyl)-, -N'''-(4-chlor-benzolsulfonyl)-, -N'''-(2-fluor-benzolsulfonyl)-, -N'''-(4-fluor-benzolsulfonyl)-, -N'''-(2-brom-benzolsulfonyl)-, -N'''-(4-brombenzolsulfonyl)-, -N'''-(2-cyano-benzolsulfonyl)-, -N'''-(2-nitro-benzolsulfonyl)-, -N'''-(4-nitro-benzolsulfonyl)-, -N'''-(2-methyl-benzolsulfonyl)- -N'''-(4-methyl-benzolsulfonyl)-, -N'''-(2-chlormethyl-benzolsulfonyl)-, -N'''-(2-trifluormethyl-benzolsulfonyl)-, -N'''-(2-methoxy-benzolsulfonyl)-, -N'''-(4-methoxy-benzolsulfonyl)-, -N'''-(2-methylthio-benzolsulfonyl)-, -N'''-(2-difluormethoxybenzolsulfonyl)-, -N'''-(2-trifluormethoxy-benzolsulfonyl)-, -N'''-(2-methylthiomethyl-benzolsulfonyl)-, -N'''-(2-dimethylaminosulfonyl-benzolsulfonyl)-, -N'''-(2-phenyl-benzolsulfonyl)-, -N'''-(2-methoxysulfonyl-benzolsulfonyl)-, -N'''-(2-methoxycarbonyl-benzolsulfonyl)-, -N'''-(2-ethoxycarbonyl-benzolsulfonyl)-, -N'''-(2-propoxycarbonyl-benzolsulfonyl)-, -N'''-(2-methylaminocarbonyl-benzolsulfonyl)-,-N'''-(2-ethylaminocarbonyl-benzolsulfonyl)-,-N'''-(2-propylaminocarbonyl-benzolsulfonyl)-, -N'''-(2-methoxyaminocarbonyl-benzolsulfonyl)-, -N'''-(2-ethoxyaminocarbonyl-benzolsulfonyl)-, -N'''-(2-propoxyaminocarbonyl-benzolsulfonyl)-, -N'''-(2-dimethylaminocarbonyl-benzolsulfonyl)- und -N'''-(2-diethylaminocarbonyl-benzolsulfonyl)guanidin.

Die Verbindungen der Formel (IE) können nach den oben unter (a) und (b) beschriebenen Verfahren hergestellt werden.

Die bei der Verfahrensvariante (c) weiter als Ausgangsstoffe zu verwendenden Sulfonsäurechloride sind durch die Formel (V) allgemein definiert. In Formel (V) hat $R^{10}$ vorzugsweise bzw. insbesondere die gleiche Bedeutung, wie sie oben im Rahmen der Substituentendefinition der Formel (I) als bevorzugt bzw. als insbesondere bevorzugt angegeben ist.

Als Beispiele für die Ausgangsstoffe der Formel (V) können die Sulfonsäurechloride gelten, die oben als Beispiele für Verbindungen der Formel (III) genannt wurden.

Die Sulfonsäurechloride der Formel (V) sind bekannt und/oder können nach an sich bekannten Verfahren hergestellt werden (vgl. Literatur und Synthesewege für die Verbindungen der Formel (III) oben).

Die als Ausgangsstoffe für die Verfahrensvariante (d) zu verwendenden Sulfonylguanidinotriazin-Derivate sind durch die Formel (VI) allgemein definiert. In Formel (VI) haben $R^3$, $R^4$, $R^5$ und $R^{10}$ vorzugsweise bzw. insbesondere die gleichen Bedeutungen, wie sie oben im Rahmen der Subsituentendefinition der Formel (I) vorzugsweise bzw. als insbesondere bevorzugt angegeben sind.

Als Beispiele für die Verbindungen der Formel (VI) seien genannt:

N'-(4,6-Dimethyl-s-triazin-2-yl)-, N'-(4-Methoxy-6-methyl-s-triazin-2-yl)-, N'-(4-Ethoxy-6-methyl-s-triazin-2-yl)-, N'-(4,6-Dimethoxy-s-triazin-2-yl)-, N'-(4,6-Diethoxy-s-triazin-2-yl)-, N'-(4-Ethoxy-6-methoxy-s-triazin-2-yl)-, N'-(4-Methyl-6-methylthio-s-triazin-2-yl)-, N'-(4-Ethylthio-6-methyl-s-triazin-2-yl)-, N'-(4-Methoxy-6-methylthio-s-triazin-2-yl)-, N'-(4-Ethoxy-6- methylthio-s-triazin-2-yl)-, N'-(4-Ethylthio-6-methoxy-s-triazin-2-yl)-, N'-(4-Ethoxy-6-ethylthio-s-triazin-2-yl)-, N'-(4,6-Bis-methylthio-s-triazin-2-yl)-, N'-(4,6-Bis-ethylthio-s-triazin-2-yl)-, N'-(4-Methyl-6-methylamino-s-triazin-2-yl)-, N'-(4-Ethylamino-6-methyl-s-triazin--2-yl)-, N'-(4-Dimethylamino-6-methyl-s-triazin-2-yl)-, N'-(4-Diethylamino-6-methyl-s-triazin-2-yl)-, N'-(4-Methoxy-6-methylamino-s-triazin-2-yl)-, N'-(4-Ethylamino-6-methoxy-s-triazin-2-yl)-, N'-(4-Dimethylamino-6-methoxy-s-triazin-2-yl)-, N'-(4-Diethylamino-6-methoxy-s-triazin-2-yl)-, N'-(4-Ethoxy-6-methylamino-s-triazin-2-yl)-, N'-(4-Ethoxy-6-ethylamino-s-triazin- 2-yl)-, N'-(4-Dimethylamino-6-ethoxy-s-triazin-2-yl)-, N'-(4-Diethylamino-6-ethoxy-s-triazin-2-yl)-, N'-(4-Methylamino-6-methylthio-s-triazin-2-yl)-, N'-(4-Ethylamino-6-methylthio-s-triazin-2-yl)-, N'-(4-Dimethylamino-6-methylthio-s-triazin-2-yl)-, N'-(4-Diethylamino-6-methylthio-s-triazin-2-yl)-, N'-(4-Ethylthio-6-methylamino-s-triazin-2-yl)-, N'-(4-Dimethylamino-6-ethylthio-s-triazin-2-yl)- und N'-(4-Diethylamino-6-ethylthio-s-triazin-2-yl)-N"-methoxy-, -N"-ethoxy-, -N"-propoxy-, -N"-isopropoxy-, -N"-butoxy-, -N"-isobutoxy-, -N"-sec.-butoxy-, -N"-pentyloxy-, -N"-hexyloxy-, -N"-octyloxy-, -N"-allyloxy-, -N"-(3-chlor-propoxy)-, -N"-methoxycarbonylmethoxy-, -N"-ethoxycarbonylmethoxy, -N"-(2-phenylethoxy)-, -N"-benzyloxy-, -N"-(4-methyl-benzyloxy)-,

-N"-(4-fluor-benzyloxy)-, -N"-(4-chlor-benzyloxy)-, -N"-(4-nitro-benzyloxy)- und -N"-(4-methoxycarbonyl-benzyloxy)-, N"-benzolsulfonyl-, -N"-(2-chlor-benzolsulfonyl)-, -N"-(4-chlor-benzolsulfonyl)-, -N"-(2-fluor-benzolsulfonyl)-, -N"-(4-fluor-benzolsulfonyl)-, -N"-(2-brom-benzolsulfonyl)-, -N"-(3-chlor-benzolsulfonyl)-, -N"-(2-cyano-benzolsulfonyl)-, -N"-(2-nitro-benzolsulfonyl)-, -N"-(4-nitro-benzolsulfonyl)-, -N"-(2-methyl-benzolsulfonyl)-, -N"-(4-methyl-benzolsulfonyl)-, -N"-(2-chlormethyl-benzolsulfonyl)-, -N"-(2-trifluormethyl-benzolsulfonyl)-, -N"-(2-methoxy-benzolsulfonyl)-, -N"-(4-methoxy-benzolsulfonyl)-, -N"-(2-methylthio-benzolsulfonyl)-, -N"-(2-difluormethoxy-benzolsulfonyl)-, -N"-(2-trifluormethoxy-benzolsulfonyl)-, -N"-(2-methylthiomethyl-benzolsulfonyl)-, -N"-(2-dimethylaminosulfonyl-benzolsulfonyl)-, -N"-(2-phenyl-benzolsulfonyl)-, -N"-(2-methoxysulfonyl-benzolsulfonyl)-, -N"-(2-methoxycarbonyl-benzolsulfonyl)-, -N"-(2-ethoxycarbonyl-benzolsulfonyl)-, -N"-(2-propoxycarbonyl-benzolsulfonyl)-, -N"-[2-(N-methyl-N-methoxyaminocarbonyl-benzolsulfonyl]-, -N"-(2-dimethylaminocarbonyl-benzolsulfonyl)- und -N"-(2-diethylaminocarbonyl-benzolsulfonyl)-guanidin.

Die Sulfonylguanidinotriazin-Derivate der Formel (VI) sind neu und können analog Verfahren (a) durch Umsetzung von Guanidinotriazin-Derivaten der Formel (II) mit etwa äquimolaren Mengen von Sulfonsäurechloriden der Formel (V) hergestellt werden.

Die bei der Verfahrensvariante (d) weiter als Ausgangsstoffe zu verwendenden Sulfonsäurechloride sind durch

die Formel (III) allgemein definiert. In Formel (III) hat $R^1$ vorzugsweise bzw. insbesondere die gleiche Bedeutung, wie sie oben im Rahmen der Subsituentendefinition der Formel (I) vorzugsweise bzw. als insbesondere bevorzugt genannt ist.

Beispiele für die Sulfonsäurechloride der Formel (III) und ihre Herstellungsverfahren hierfür wurden bereits weiter oben im Zusammenhang mit der Beschreibung der Ausgangsstoffe für Verfahren (a) angegeben.

Die bei der Verfahrensvariante (e) als Ausgangsstoffe zu verwendenden Sulfonylisothioureidotriazin-Derivate sind durch die Formel (VII) allgemein definiert. In Formel (VII) haben $R^1$, $R^4$ und $R^5$ vorzugsweise bzw. insbesondere die gleichen Bedeutungen, wie sie oben im Rahmen der Substituentendefinition der Formel (I) als bevorzugt bzw. als insbesondere bevorzugt genannt sind und $R^{16}$ steht vorzugsweise wie auch insbesondere für Methyl.

Als Beispiele für die Verbindungen der Formel (VII) seien genannt:
N'-(4,6-Dimethyl-s-triazin-2-yl)-, N'-(4-Methoxy-6-methyl-s-triazin-2-yl)-, N'-(4-Ethoxy-6-methyl-s-triazin-2-yl)-, N'-(4,6-Dimethoxy-s-triazin-2-yl)-, N'-(4,6-Diethoxy-s-triazin-2-yl)-, N'-(4-Ethoxy-6-methoxy-s-triazin-2-yl)-, N'-(4-Methyl-6-methylthio-s-triazin-2-yl)-, N'-(4-Ethylthio-6-methyl-s-triazin-2-yl)-, N'-(4-Methoxy-6-methylthio-s-triazin-2-yl)-, N'-(4-Ethoxy-6-methylthio-s-triazin-2-yl)-, N'-(4-Ethylthio-6-methoxy-s-triazin-2-yl)-, N'-(4-Ethoxy-6-ethylthio-s-triazin-2-yl)-, N'-(4,6-Bis-methylthio-s-triazin-2-yl)-, N'-(4,6-Bis-ethylthio-s-triazin-2-yl)-, N'-(4-Methyl-6-methylamino-s-triazin-2-yl)-, N'-(4-Ethylamino-6-methyl-s-triazin-2-yl)-, N'-(4-Dimethylamino-6-methyl-s-triazin- 2-yl)-, N'-(4-Diethylamino-6-methyl-s-triazin-2-yl)-, N'-(4-Methoxy-6-methylamino-s-triazin-2yl)-, N'-(4-Ethylamino-6-methoxy-s-triazin-2-yl)-, N'-(4-Dimethylamino-6-methoxy-s-triazin-2-yl)-, N'-(4-Diethylamino-6-methoxy-s-triazin-2-yl)-, N'-(4-Ethoxy-6-methylamino-s-triazin-2-yl)-, N'-(4-Ethoxy-6-ethylamino-s-triazin-2-yl)-, N'-(4-Dimethylamino-6-ethoxy-s-triazin-2-yl)-, N'-(4-Diethylamino-6-ethoxy-s-triazin-2-yl)-, N'-(4-Methylamino-6-methylthio-s-triazin-2-yl)-, N'-(4-Ethylamino-6-methylthio-s-triazin-2-yl)-, N'-(4-Dimethylamino-6-methylthio-s-triazin-2-yl)-, N'-(4-Diethylamino-6-methylthio-s-triazin-2-yl)-, N'-(4-Ethylthio-6-methylamino-s-triazin-2-yl)-, N'-(4-Dimethylamino-6-ethylthio-s-triazin-2-yl)- und N'-(4-Diethylamino-6-ethylthio-s-triazin-2-yl)-N"-(2-fluor-benzolsulfonyl)-, -N"-(2- chlor-benzolsulfonyl)-, -N"-(2-brom-benzolsulfonyl)-, -N"-(2-methyl-benzolsulfonyl)-, -N"-(2-methoxy-benzolsulfonyl)-, -N"-(2-methoxycarbonyl-benzolsulfonyl)-, -N"-(2-ethoxycarbonyl-benzolsulfonyl)-, -N"-(2-propoxycarbonyl-benzolsulfonyl)-, -N"-(2-isopropoxycarbonyl-benzolsulfonyl)-, -N"-(2-phenyl-benzolsulfonyl)-, -N"-(2-trifluormethyl-benzolsulfonyl)-, -N"-(difluormethoxy-benzolsulfonyl)- und -N"-(2-trifluormethoxy-benzolsulfonyl)-S-methyl-isothioharnstoff.

Die Sulfonylisothioureidotriazin-Derivate der Formel (VII) sind bekannt und/oder können nach an sich bekannten Verfahren hergestellt werden (vgl. EP-OS 5 986).

Verbindungen der Formel (VII), in welcher $R^1$ für einen ausschließlich in ortho-Position durch $C_1$-$C_4$-Fluoralkoxy, $C_1$-$C_4$-Alkylthio, $C_1$-$C_4$-Alkylsulfonyl oder $C_1$-$C_4$-Alkylsulfonyl substituierten Phenylrest steht und $R^4$, $R^5$ und $R^{16}$ die oben angegebene Bedeutung haben, sind noch nicht bekannt (diese neuen Verbindungen werden in Anspruch 6 durch Formel (VII A) bezeichnet).

Man erhält die Verbindungen der Formel (VII), wenn man N-Sulfonyl-imino-dithiokohlensäureester der Formel (XV)

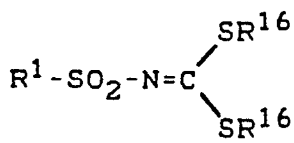

(XV)

in welcher

$R^1$ und $R^{16}$ die oben angegebenen Bedeutungen haben,

mit Aminotriazinen der Formel (XI)

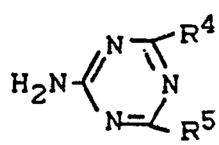

(XI)

in welcher

$R^4$ und $R^5$ die oben angegebenen Bedeutungen haben,

gegebenenfalls in Gegenwart von Basen, wie z.B. Natriumhydrid oder Kalium-tert.butylat, und gegebenenfalls in Gegenwart von Verdünnungsmitteln, wie z.B. Tetrahydrofuran, Dioxan oder Dimethylformamid bei Temperaturen zwischen 0°C und 100°C umsetzt, nach Reaktionsende mit Wasser verdünnt, mit einer starken Säure, wie z.B. Salzsäure, ansäuert und die kristallin anfallenden Produkte der Formel (VII) durch Absaugen isoliert.

0173956

Die hierbei als Ausgangsstoffe benötigten N-Sulfonyl-imino-dithiokohlensäureester sind durch die Formel (XV) allgemein definiert. In Formel (XV) haben $R^1$ und $R^{16}$ vorzugsweise bzw. insbesondere die gleichen Bedeutungen wie sie oben im Rahmen der Substituentendefinition der Formel (I) bzw. Formel (VII) vorzugsweise bzw. als insbesondere bevorzugt genannt sind.

Als Beispiele für die Verbindungen der Formel (XV) seien genannt:
N-(2-Fluor-benzolsulfonyl)-, N-(2-Chlor-benzolsulfonyl)-, N-(2-Brom-benzolsulfonyl)-, N-(2-Methyl-benzolsulfonyl)-, N-(2-Methoxycarbonyl-benzolsulfonyl)-, N-(2-Ethoxycarbonyl-benzolsulfonyl)-, N-(2-Propoxycarbonyl-benzolsulfonyl)-, N-(2-Isopropoxycarbonyl-benzolsulfonyl)-, N-(2-Phenyl-benzolsulfonyl)-, N-(2-Trifluormethyl-benzolsulfonyl)-, N-(2-Difluormethoxy-benzolsulfonyl)- und N-(2-Trifluormethoxy-benzolsulfonyl)-S,S-dimethyl-imino-dithiokohlensäureester.

Die N-Sulfonyl-imino-dithiokohlensäureester der Formel (XV) sind bekannt und/oder können nach an sich bekannten Verfahren hergestellt werden (vgl. Chem. Ber. 99 (1966), 2885 und EP-OS 5 986).

Man erhält die Verbindungen der Formel (XV), wenn man Sulfonsäureamide der Formel (XVI)

$$R^1\text{-}SO_2\text{-}NH_2 \qquad (XVI)$$

in welcher

$R^1$ die oben angegebene Bedeutung hat,

mit Schwefelkohlenstoff in Gegenwart einer starken Base, wie z.B. Natriumhydroxid, und gegebenenfalls in Gegenwart von Verdünnungsmitteln, wie z.B. Wasser und Dimethylformamid, bei Temperaturen zwischen 0°C und 100°C umsetzt und anschließend (in situ) mit einem Alkylierungsmittel der Formel (XVII)

$$X\text{-}R^{16} \qquad (XVII)$$

in welcher

$R^{16}$ die oben angegebene Bedeutung hat und
X für Chlor, Brom oder Iod steht,

bei Temperaturen zwischen 0°C und 100°C umsetzt.

Die nach Verdünnen mit Wasser kristallin anfallenden Produkte der Formel (XV) können durch Absaugen isoliert werden.

Die als Ausgangsstoffe benötigten Sulfonsäureamide sind durch die Formel (XVI) allgemein definiert. In Formel (XVI) hat $R^1$ vorzugsweise bzw. insbesondere die gleiche Bedeutung, wie sie oben im Rahmen der Substituentendefinition der Formel (I) vorzugsweise bzw. als insbesondere bevorzugt angegeben ist.

Als Beispiele für die Verbindungen der Formel (XVI) seien genannt:
2-Fluor-, 2-Chlor-, 2-Brom-, 2-Methyl-, 2-Methoxycarbonyl-, 2-Ethoxycarbonyl-, 2-Propoxycarbonyl-, 2-Isopropoxycarbonyl-, 2-Phenyl-, 2-Trifluormethyl-, 2-Difluormethoxy- und 2-Trifluormethoxy-benzolsulfonsäureamid.

Die Sulfonsäureamide der Formel (XVI) sind bekannt und/ oder können nach an sich bekannten Verfahren hergestellt werden (vgl. EP-OS 23 422, 30 140, 35 893, 44 807, 44 808, 44 809, 51 466, 64 804, 70 041 und 70 802; US-PS 4 372 778 und Zh. Org. Khim. [J. Org. Chem. UdSSR] 8 (1972), 1023-1026 [engl. 1032-1034]).

Man erhält diese Verbindungen auf an sich bekannte Weise durch Umsetzung von Sulfonsäurechloriden der Formel (III) - oben - mit Ammoniak, gegebenenfalls in Gegenwart von Verdünnungsmitteln, wie z.B. Diethylether, Tetrahydrofuran oder auch Wasser, bei Temperaturen zwischen 0°C und 100°C. Die hierbei kristallin anfallenden Produkte der Formel (XVI) können durch Absaugen isoliert werden.

Beispiele für geeignete Ausgangsverbindungen der Formel (III) und Herstellungsmethoden hierfür sind oben bei der Beschreibung der Ausgangsstoffe für Verfahren (a) aufgeführt.

Als Beispiele für die Ausgangsstoffe der Formel (XVII) seien genannt:
Methylchlorid, Methylbromid, Methyliodid, Ethylchlorid, Ethylbromid und Ethyliodid sowie Benzylchlorid und Benzylbromid.

Die Verbindungen der Formel (XVII) sind bekannt.

Die weiter als Ausgangsstoffe zu verwendenden Aminotriazine sind durch die Formel (XI) allgemein definiert. In Formel (XI) haben $R^4$ und $R^5$ vorzugsweise bzw. insbesondere die gleichen Bedeutungen, wie sie

oben im Rahmen der Substituentendefinition der Formel (I) vorzugsweise bzw. als insbesondere bevorzugt genannt sind.

Als Beispiele für die Verbindungen der Formel (XI) seien genannt:
4,6-Dimethyl-, 4-Methoxy-6-methyl-, 4-Ethoxy-6-methyl-, 4,6-Dimethoxy-, 4,6-Diethoxy-, 4-Ethoxy-6-methoxy-, 4-Methyl-6-methylthio-, 4-Ethylthio-6-methyl-, 4-Methoxy-6-methylthio-, 4-Ethoxy-6-methylthio-, 4-Ethylthio-6-methoxy-, 4-Ethoxy-6-ethylthio-, 4,6-Bis-methylthio-, 4,6-Bis-ethylthio-, 4-Methyl-6-methylamino-, 4-Ethylamino-6-methyl-, 4-Dimethylamino-6-methyl-, 4-Diethylamino-6-methyl-, 4-Methoxy-6-methylamino-, 4-Ethylamino-6-methoxy-, 4-Dimethylamino-6-methoxy-, 4-Diethylamino-6-methoxy-, 4-Ethoxy-6-methylamino-, 4-Ethoxy-6-ethylamino-, 4-Dimethylamino-6-ethoxy-, 4-Diethylamino-6-ethoxy-, 4-Methylamino-6-methylthio-, 4-Ethylamino-6-methylthio-, 4-Dimethylamino-6-methylthio-, 4-Diethylamino-6-methylthio-, 4-Ethylthio-6-methylamino-, 4-Dimethyl-amino-6-ethylthio und 4-Diethylamino-6-ethylthio-2-amino-s-triazin.

Die Aminotriazine der Formel (XI) sind bekannt und/oder können nach an sich bekannten Verfahren hergestellt werden.

Die bei der Verfahrensvariante (e) weiter als Ausgangsstoffe zu verwendenden Aminoverbindungen sind durch die

Formel (IV) allgemein definiert. In Formel (IV) steht $R^2$ vorzugsweise für Wasserstoff oder Methyl, insbesondere für Wasserstoff, und $R^3$ hat vorzugsweise bzw. insbesondere die gleiche Bedeutung, wie sie oben im Rahmen der Substituentendefinition für Formel (I) vorzugsweise bzw. als inbesondere bevorzugt angegeben ist.

Beispiele für geeignete Ausgangsstoffe der Formel (IV) und Literaturangaben hierzu sind oben bei der Beschreibung der Ausgangsstoffe für Verfahren (b) aufgeführt.

Die bei der Verfahrensvariante (f) als Ausgangsstoffe zu verwendenden Verbindungen sind durch die Formel (I) - mit der Maßgabe, daß M für Wasserstoff steht - allgemein definiert. In Formel (I) - soweit sie dieals Ausgangsstoffe für Verfahren (f) zu verwendenden Verbindungen betrifft - steht M für Wasserstoff und $R^1$, $R^2$, $R^3$, $R^4$ und $R^5$ haben vorzugsweise bzw. insbesondere die gleichen Bedeutungen, wie sie oben im Rahmen der Substituentendefinition der Formel (I) als bevorzugt bzw.als insbesondere bevorzugt angegeben sind.

Die als Ausgangsstoffe für Verfahren (f) zu verwendenden Verbindungen der Formel (I) können nach den unter (a), (b), (c), (d) und (e) beschriebenen Verfahren hergestellt werden.

Als Beispiele für die bei Verfahren (f) zu verwendenden Metallhydroxide, -hydride, -alkanolate bzw. metallorganischen Verbindungen seien genannt: Lithium-, Natrium-, Kalium-, Magnesium- und Calcium-hydroxid, Lithium-, Natrium- und Calcium-hydrid, Natrium-methanolat und -ethanolat, Kalium-methanolat, -ethanolat und Kalium-tert.-butanolat sowie Butyllithium und Isopropylmagnesiumchlorid.

Die bei der Verfahrensvariante (g) als Ausgangsstoffe zu verwendenden Verbindungen sind durch die Formel (I) - mit der Maßgabe, daß M für Wasserstoff steht - allgemein definiert. In Formel (I) - soweit sie die als Ausgangsstoffe für Verfahren (g) zu verwendenden Verbindungen betrifft - steht M für Wasserstoff und $R^1$, $R^2$, $R^3$, $R^4$ und $R^5$ haben vorzugsweise bzw. insbesondere die gleichen Bedeutungen, wie sie oben im Rahmen der Substituentendefinition der Formel (I) vorzugsweise bzw. als insbesondere bevorzugt angegeben sind.

Die als Ausgangsstoffe für Verfahren (g) zu verwendenden Verbindungen der Formel (I) können nach den unter (a), (b), (c), (d) und (e) beschriebenen Verfahren hergestellt werden.

Bei Verfahren (g) werden starke Säuren als Ausgangsstoffe eingesetzt. Vorzugsweise sind dies Halogenwasserstoffsäuren, wie Hydrogenchlorid, Hydrogenbromid oder Hydrogeniodid, weiter Schwefelsäure oder gegebenenfalls durch Fluor oder Chlor substituierte Alkansulfonsäuren mit bis zu 4 Kohlenstoffatomen, wie z.B. Methansulfonsäure, Ethansulfonsäure, Chlormethansulfonsäure, 2-Chlorethansulfonsäure und Trifluormethansulfonsäure, Trifluoressigsäure, ferner Benzolsulfonsäure, p-Toluolsulfonsäure, Naphthalin-1-sulfonsäure, Naphthalin-2-sulfonsäure, Naphthalin-1,4-, -1,5-, -1,6-, -2,6- und -2,7-disulfonsäure.

Das erfindungsgemäße Verfahren (a) zur Herstellung der neuen Verbindungen der Formel (I) wird vorzugsweise unter Verwendung von Verdünnungsmitteln durchgeführt. Als solche kommen praktisch alle inerten organischen, vorzugsweise jedoch aprotisch polare Solventien in Betracht. Hierzu gehören gegebenenfalls substituierte Kohlenwasserstoffe, wie z.B. Methylenchlorid, Chloroform, 1,2-Dichlorethan, Toluol, Xylol und Chlorbenzol, Nitrile, wie Acetonitril und Propionitril, Ether, wie z.B. 1,2-Dimethoxyethan, Tetrahydrofuran und Dioxan, sowie Dimethylformamid, Dimethylacetamid, Dimethylsulfoxid, Sulfolan, Pyridin und 2-Methyl-5-ethyl-pyridin.

Als Säureakzeptoren können bei Verfahren (a) praktisch alle üblicherweise verwendeten Säurebindemittel eingesetzt werden. Hierzu gehören insbesondere Alkalimetall- und Erdalkalimetallhydroxide, Alkalimetall- und Erdalkalimetallhydride, metallorganische Verbindungen, wie Butyllithium, ferner aliphatische, aromatische oder heterocyclische Amine, wie Trimethylamin, Triethylamin, N,N-Dimethyl-anilin, N,N-Dimethyl-benzylamin, Diazabicyclooctan (DABCO), Diazabicyclononen (DBU), Diazabicycloundecen (DBU), Pyridin, 2-Methyl-5-ethyl-pyridin und 4-Dimethylamino-pyridin.

Die Reaktionstemperaturen können bei Verfahren (a) innerhalb eines größeren Bereiches variiert werden. Im allgemeinen arbeitet man zwischen -80°C und +100°C, vorzugsweise zwischen -30°C und +50°C. Das erfindungsgemäße Verfahren (a) wird im allgemeinen bei Normaldruck durchgeführt.

Zur Durchführung von Verfahren (a) setzt man je Mol Guanidinotriazin-Derivat der Formel (II) im allgemeinen zwischen 1 und 5 Mol, vorzugsweise zwischen 2 und 3 Mol Sulfonsäurechlorid der Formel (III) ein.

Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung und Isolierung der neuen Verbindungen erfolgt nach üblichen Methoden. Gegebenenfalls nach Abdestillieren flüchtiger Komponenten wird mit Wasser und einem mit Wasser nicht mischbaren Lösungsmittel, wie z.B. Methylenchlorid, Chloroform oder Toluol, geschüttelt, die organische Phase mit Wasser gewaschen, getrocknet, filtriert und eingeengt. Die im Rückstand verbleibenden Produkte der Formel (I) werden durch Digerieren mit organischen Lösungsmitteln, wie z.B. Diethylether, Essigester, Ethanol oder Isopropanol zur Kristallisation gebracht und gegebenenfalls durch Umkristallisieren gereinigt.

Das erfindungsgemäße Verfahren (b) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie Methanol, Ethanol, n- und i-Propanol, Ether, wie Tetrahydrofuran, Dioxan und 1,2-Dimethoxyethan, Ester, wie Essigsäuremethylester und -ethylester, Nitrile, wie z.B. Acetonitril oder Propionitril, sowie Dimethylformamid und Wasser.

Als Säureakzeptoren können bei Verfahren (b) Säurebindemittel eingesetzt werden, welche in ihren nucleophilen Eigenschaften nicht nennenswert mit den Aminoverbindungen der Formel (IV) konkurrieren.
Als solche seien Alkalimetall- und Erdalkalimetallcarbonate, wie z.B. Kaliumcarbonat und Calciumcarbonat, tertiäre Amine, wie z.B. Triethylamin, N,N-Dimethylanilin und N,N-Dimethylbenzylamin, sowie Stickstoffheterocyclen, wie z.B. Pyridin, Diazabicyclooctan (DABCO) und Diazabicycloundecen (DBU) genannt.

Die Reaktionstemperatur kann bei Verfahren (b) innerhalb eines größeren Bereiches variiert werden. Im allgemeinen arbeitet man zwischen 0°C und 150°C, vorzugsweise zwischen 10°C und 100°C. Das erfindungsgemäße Verfahren (b) wird im allgemeinen bei Normaldruck durchgeführt.

Zur Durchführung des erfindungsgemäßen Verfahrens (b) setzt man je Mol Verbindung der Formel (ID) im allgemeinen zwischen 1 und 10 Mol, vorzugsweise zwischen 2 und 5 Mol Aminoverbindung der Formel (IV) oder deren Hydrochlorid ein.

Im allgemeinen wird die Verbindung der Formel (ID) mit dem Verdünnungsmittel bei 20°C oder unter leichtem Kühlen vorgelegt und die Aminoverbindung der Formel (IV) bzw. das Hydrochlorid hiervon und gegebenenfalls ein geeigneter Säureakzeptor dazugegeben. Das Reaktionsgemisch wird dann im allgemeinen bei Raumtemperatur oder erhöhter Temperatur bis zum Reaktionsende gerührt.

Die Aufarbeitung kann nach üblichen Methoden durchgeführt werden. Soweit die Produkte der Formel (I) aus dem Reaktionsgemisch kristallin anfallen, können sie durch Absaugen isoliert werden. Andernfalls wird - gegebenenfalls nach Einengen - mit Wasser verdünnt und mit einem mit Wasser praktisch nicht mischbaren Lösungsmittel, wie z.B. Methylenchlorid, extrahiert. Durch Waschen der Extraktionslösung mit Wasser, Trocknen, Filtrieren, Einengen des Filtrats und Umkristallisieren des Rückstandes können die Produkte der Formel (I) in reiner Form erhalten werden.

Das erfindungsgemäße Verfahren (c) wird vorzugsweise unter Verwendung von Verdünnungsmitteln durchgeführt. Als solche kommen praktisch alle inerten organischen, vorzugsweise jedoch aprotisch polare Solventien in Betracht, wie sie oben im Zusammenhang mit der Beschreibung des erfindungsgemäßen Verfahrens (a) genannt wurden.

Als Säureakzeptoren können bei Verfahren (c) praktisch alle üblicherweise verwendeten Säurebindemittel eingesetzt werden. Hierzu gehören insbesondere die oben im Zusammenhang mit der Beschreibung des erfindungsgemäßen Verfahrens (a) genannten Säurebindemittel.

Die Reaktionstemperaturen können bei Verfahren (c) innerhalb eines größeren Bereiches variiert werden. Im allgemeinen arbeitet man zwischen -80°C und +100°C, vorzugsweise zwischen -30°C und +50°C. Das Verfahren (c) wird im allgemeinen bei Normaldruck durchgeführt.

Zur Durchführung von Verfahren (c) setzt man je Mol Sulfonylguanidinotriazin-Derivat der Formel (IE) im allgemeinen zwischen 1 und 2 Mol, vorzugsweise zwischen 1,1 und 1,5 Mol Sulfonsäurechlorid der Formel (V) ein.

Die Durchführung und Aufarbeitung kann bei Verfahren (c) analog zu Verfahren (a) erfolgen.

Das erfindungsgemäße Verfahren (d) wird vorzugsweise unter Verwendung von Verdünnungsmitteln durchgeführt. Als solche kommen praktisch alle inerten organischen, vorzugsweise jedoch aprotisch polare Solventien in Betracht, wie sie oben im Zusammenhang mit der Beschreibung des erfindungsgemäßen Verfahrens (a) genannt wurden.

Als Säureakzeptoren können bei Verfahren (d) praktisch alle üblicherweise verwendeten Säurebindemittel eingesetzt werden. Hierzu gehören insbesondere die oben im Zusammenhang mit der Beschreibung des erfindungsgemäßen Verfahrens (a) genannten Säurebindemittel.

Die Reaktionstemperaturen können bei Verfahren (d) innerhalb eines größeren Bereiches variiert werden. Im allgemeinen arbeitet man zwischen -80°C und +100°C, vorzugsweise zwischen -30°C und +50°C. Das Verfahren (d) wird im allgemeinen bei Normaldruck durchgeführt.

Zur Durchführung von Verfahren (d) setzt man je Mol Sulfonylguanidinotriazin-Derivat der Formel (VI) im allgemeinen zwischen 1 und 2 Mol, vorzugsweise zwischen 1,1 und 1,5 Mol Sulfonsäurechlorid der Formel (III) ein.

Die Durchführung und Aufarbeitung kann bei Verfahren (d) analog zu Verfahren (a) erfolgen.

Das erfindungsgemäße Verfahren (e) wird vorzugsweise unter Verwendung von Verdünnungsmitteln durchgeführt. Als solche kommen praktisch alle inerten organischen, vorzugsweise jedoch aprotisch polare Solventien in Betracht. Hierzu gehören insbesondere Tetrahydrofuran, Dioxan, 1,2-Dimethoxy-ethan, Acetonitril, Aceton, Methylethylketon, Methylisobutylketon, Dimethylformamid, Dimethylacetamid und Dimethylsulfoxid.

Die Reaktionstemperatur kann bei Verfahren (e) innerhalb eines größeren Bereiches variiert werden. Im allgemeinen arbeitet man zwischen 0°C und 150°C, vorzugsweise zwischen 10°C und 100°C. Das Verfahren (e) wird im allgemeinen bei Normaldruck oder geringfügig vermindertem Druck durchgeführt.

Zur Durchführung des erfindungsgemäßen Verfahrens (e) setzt man je Mol Sulfonylisothioureidotriazin-Derivat der Formel (VII) im allgemeinen zwischen 1 und 10 Mol, vorzugsweise zwischen 1 und 5 Mol Aminoverbindung der Formel (IV) ein.

Im allgemeinen werden die Sulfonylisothioureidotriazin-Derivate der Formel (VII) und das Verdünnungsmittel vorgelegt und die Aminoverbindung der Formel (IV) wird zudosiert. Das Reaktionsgemisch wird dann bis zum Reaktionsende gerührt. Die Produkte der Formel (I) fallen gewöhnlich nach Abkühlen und gegebenenfalls nach Ansäuern, z.B. mit Salzsäure, kristallin an und können durch Absaugen isoliert werden.

Das erfindungsgemäße Verfahren (f) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie z.B. Ethanol, n- und iso-Propanol, Ether, wie z.B. Tetrahydrofuran, Dioxan und 1,2-Dimethoxyethan, Ester, wie z.B. Essigsäureethylester und -methylester sowie Nitrile, wie z.B. Acetonitril.

Die Reaktionstemperatur kann bei Verfahren (f) innerhalb eines größeren Bereiches variiert werden. Im allgemeinen arbeitet man zwischen -20°C und +50°C, vorzugsweise zwischen 0°C und +30°C. Das Verfahren (f) wird im allgemeinen bei Normaldruck durchgeführt.

Zur Durchführung des erfindungsgemäßen Verfahrens (f) setzt man je Mol Verbindung der Formel (I) im allgemeinen zwischen 0,9 und 1,2 Mol, vorzugsweise zwischen 0,95 und 1,1 Mol Metallverbindung ein.

Im allgemeinen werden die Verbindungen der Formel (I) und das Verdünnungsmittel vorgelegt und - gegebenenfalls unter leichter Außenkühlung - die Metallverbindung - gegebenenfalls im Verdünnungsmittel gelöst - zudosiert. Das Reaktionsgemisch wird bis zum Reaktionsende gerührt. Die salzartigen Produkte der Formel (I) fallen im allgemeinen kristallin an und können durch Absaugen isoliert werden.

Das erfindungsgemäße Verfahren (g) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie z.B. Methanol, Ethanol, n- und iso-Propanol, Ether, wie z.B. Tetrahydrofuran, Dioxan und 1,2-Dimethoxyethan, Ester, wie z.B. Essigsäuremethylester und -ethylester sowie Ketone, wie Aceton, Methylethylketon und Methylisobutylketon.

Soweit die als Ausgangsstoffe verwendeten Säuren in wässriger Lösung eingesetzt werden, kann vorteilhaft auch Essigsäureanhydrid als Verdünnungsmittel verwendet werden.

Die Reaktionstemperatur kann bei Verfahren (g) innerhalb eines größeren Bereiches variiert werden. Im allgemeinen arbeitet man zwischen -20°C und +50°C, vorzugsweise zwischen 0°C und +30°C. Das Verfahren (g) wird im allgemeinen bei Normaldruck durchgeführt.

Zur Durchführung des erfindungsgemäßen Verfahrens (g) setzt man je Mol Verbindung der Formel (I) im allgemeinen zwischen 1 und 10 Mol, vorzugsweise zwischen 1 und 5 Mol einer starken Säure ein.

Im allgemeinen werden die Verbindungen der Formel (I) und das Verdünnungsmittel vorgelegt und - gegebenenfalls unter leichter Außenkühlung - die starke Säure zudosiert. Das Reaktionsgemisch wird bis zum Reaktionsende gerührt. Die 1:1-Addukte fallen im allgemeinen kristallin an und können durch Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können zur selektiven Unkrautbekämpfung in mono- und dikolyten Kulturen wie z.B. Baumwolle, Reis, Getreide und Mais eingesetzt werden. [Die erfindungsgemäßen Wirkstoffe zeigen auch eine fungizide Wirkung gegen Pyricularia oryzae am Reis.]

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polaren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.
Es können in den Formulierungen Haftmittel wie Carboxymethylcellulose, natürliche und synthetische, pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethylharnstoff, 3-(3-Chlor-4-methylphenyl)-1,1-dimethylharnstoff, 3-(4-Isopropylphenyl)-1,1-dimethylharnstoff, 4-Amino-6-(1,1-dimethylethyl)-3-methylthio-1,2,4-triazin-5(4H)-on, 4-Amino-6-(1,1-dimethyl-ethyl)-3-ethylthio-1,2,4-triazin-5-(4H)-on, 1-Amino-6-ethylthio-3-(2,2-dimethylpropyl)-1,3,5-triazin-2,4-(1H,3H)-dion, 4-Amino-3-methyl-6-phenyl-1,2,4-triazin-5(4H)-on, 2-Chlor-4-ethylamino-6-isopropylamino-1,3,5-triazin, das R-Enantiomere des 2-[4-(3,5-Dichlor-pyridyl-2-oxy)-phenoxy]-propionsäure-(trimethylsilyl)-methylesters, das R-Enantiomere des 2-[4-(3,5-Dichlorpyridyl-2-oxy)-phenoxy]-propionsäure-(2-benzyloxy)-ethylesters, 2,4-Dichlorphenoxyessigsäure, 2-(2,4-Dichlorphenoxy)-propionsäure, 4-Chlor-2-methyl-phenoxyessigsäure, 2-(2-Methyl-4-chlor-phenoxy)-propionsäure, 3,5-Diiod-4-hydroxy-benzonitril, 3,5-Dibrom-4-hydroxybenzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 15 kg Wirkstoff pro Hektar Bodenfläche, vorzugsweise zwischen 0,005 und 10 kg pro ha.

Die Herstellung und die Verwendung der erfindungsgemäßen Wirkstoffe geht aus den nachfolgenden Beispielen hervor.

## Herstellungsbeispiele

### Beispiel 1

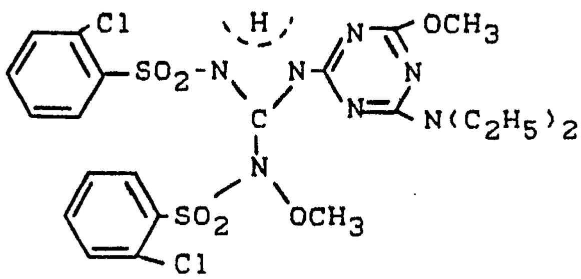

(Verfahren (a))

29,5 g (0,14 Mol) 2-Chlor-benzolsulfonsäurechlorid werden unter Rühren zu einer auf -7°C gekühlten Mischung aus 10,0 g (0,037 Mol) N'-(4-Diethylamino-6-methoxy-s-triazin-2-yl)-N"-methoxy-guanidin und 70 ml Pyridin gegeben und das Reaktionsgemisch wird 3 Tage bei 20°C gerührt. Dann wird mit Wasser und Diethylether verdünnt. Das hierbei kristallin anfallende Produkt wird durch Absaugen isoliert.

Man erhält 15,0 g (66% der Theorie) N'-(4-Diethylamino-6-methoxy-s-triazin-2-yl)-N"-methoxy-N",N'''-bis-(2-chlor-benzolsulfonyl)-guanidin vom Schmelzpunkt 133°C.

**Beispiel 2**

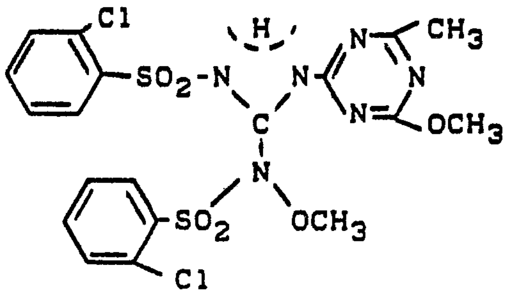

(Verfahren (c))

54 g (0,256 Mol) 2-Chlor-benzolsulfonsäurechlorid werden unter Rühren zu einer auf -20°C gekühlten Mischung von 97 g (0,25 Mol) N'-(4-Methoxy-6-methyl-s-triazin-2-yl)-N"-methoxy-N'''-(2-chlor-benzolsulfonyl)-guanidin und 300 ml Pyridin tropfenweise gegeben. Das Reaktionsgemisch wird 15 Stunden bei 20°C gerührt und eingeengt. Der Rückstand wird in 300 ml Methylenchlorid aufgenommen, diese Lösung zweimal mit je 150 ml verdünnter Salzsäure gewaschen, filtriert und eingeengt. Das beim Verreiben des Rückstandes mit Ethanol kristallin anfallende Produkt wird durch Absaugen isoliert.

Man erhält 80 g (57 % der Theorie) N'-(4-Methoxy-6-methyl-s-triazin-2-yl)-N"-methoxy-N"-N'''-bis-(2-chlor-benzolsulfonyl)-guanidin vom Schmelzpunkt 164°C.

Beispiel 3

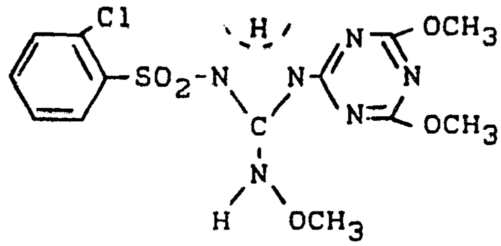

(Verfahren (e))

Eine Mischung aus 10,0 g (0,026 Mol) N'-(4,6-Dimethoxy-s-triazin-2-yl)-N"-(2-chlor-benzolsulfonyl)-S-methyl-isothioharnstoff, 3,0 g (0,065 Mol) O-Methyl-hydroxylamin und 80 ml Dioxan wird 60 Stunden bei 20°C gerührt. Dann wird eingeengt, der Rückstand mit Ethanol verrieben und das kristallin angefallene Produkt durch Absaugen isoliert.

Man erhält 5,8 g (57 % der Theorie ) N'-(4,6-Dimethoxy-s-triazin-2-yl)-N"-methoxy-N'''-(2-chlor-benzolsulfonyl)-guanidin vom Schmelzpunkt 150°C.

Beispiel 4

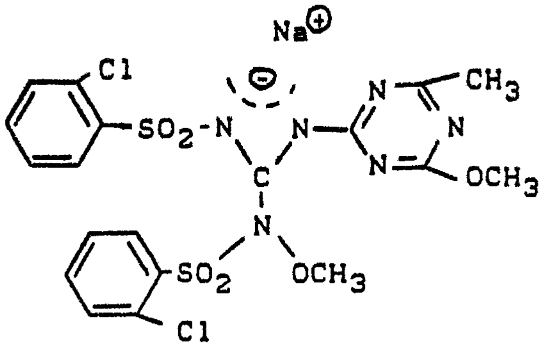

(Verfahren (f))

Eine Mischung aus 0,60 g (0,001 Mol) N'-(4-Methoxy-6-methyl-s-triazin-2-yl)-N"-methoxy-N",N'''-bis-(2-chlor-benzolsulfonyl)-guanidin, 0,054 g (0,001 Mol) Natrium-methylat und 5 ml Methanol wird eine Stunde bei 20°C gerührt. Dann wird das kristallin angefallene Produkt durch Absaugen isoliert.

Man erhält 0,50 g (84 % der Theorie) N'-(4-Methoxy-6-methyl-s-triazin-2-yl)-N"-methoxy-N",N'''-bis-(2-chlor-benzolsulfonyl)-guanidin-Natriumsalz vom Schmelzpunkt >220°C.

Beispiel 5

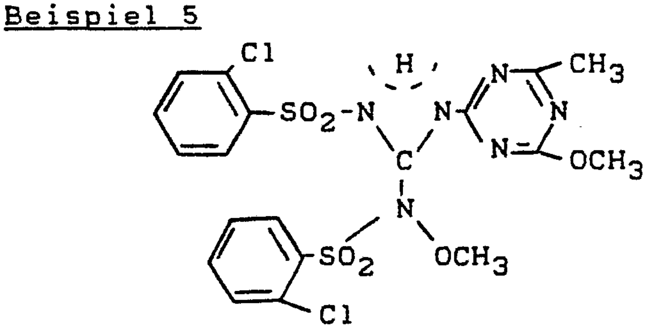

x $H_2SO_4$

(Verfahren (g))

Eine Mischung aus 5,7 g (0,01 Mol) N'-(4-Methoxy-6-methyl-s-triazin-2-yl)-N"-methoxy-N" ,N'''-bis-(2-chlorbenzolsulfonyl)-guanidin, 1,0 g (0,01 Mol) konzentrierter Schwefelsäure und 30 ml Aceton wird 15 Stunden bei 20°C gerührt. Dann wird das kristallin angefallene Produkt durch Absaugen isoliert.

Man erhält 5,3 g (81 % der Theorie) des 1:1-Adduktes aus N'-(4-Methoxy-6-methyl-s-triazin-2-yl)-N"-methoxy-N", N'''-bis-(2-chlor-benzolsulfonyl)-guanidin und Schwefelsäure vom Schmelzpunkt 148°C.

Nach den in den vorausgehenden Beispielen exemplarisch beschriebenen Verfahren können die in der nachstehenden Tabelle 1 angeführten Verbindungen der Formel (I) hergestellt werden:

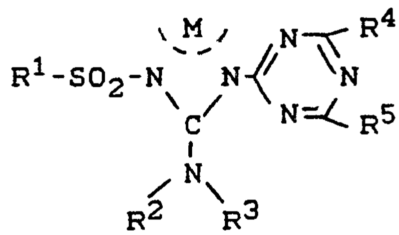

(I)

Tabelle 1

| Beispiel Nr. | $R^1$ | $R^2$ | $R^3$ | $R^4$ | $R^5$ | M | Schmelzpunkt (°C) |
|---|---|---|---|---|---|---|---|
| 6 | 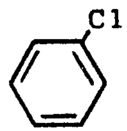 | H | $N(CH_3)_2$ | $CH_3$ | $N(CH_3)_2$ | H | 177 |
| 7 | 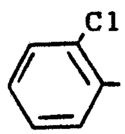 | 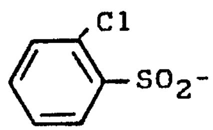 | $OCH_3$ | $CH_3$ | $N(CH_3)_2$ | H | 203 |
| 8 | 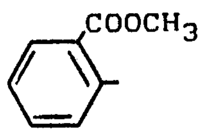 | 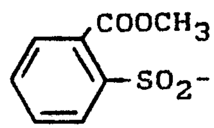 | $OCH_3$ | $OCH_3$ | $OCH_3$ | H | 105 |
| 9 | 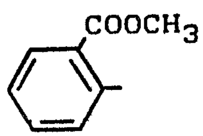 | 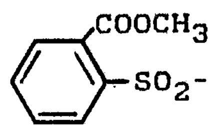 | $OCH_3$ | $OCH_3$ | $N(C_2H_5)_2$ | H | (Öl) |
| 10 | 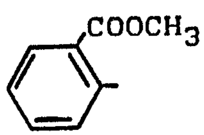 | 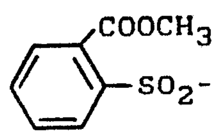 | $OCH_3$ | $SCH_3$ | $NHC_2H_5$ | H | (Öl) |
| 11 | 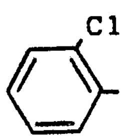 | 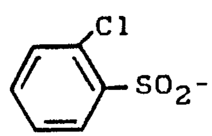 | $OCH_3$ | $SCH_3$ | $NHC_2H_5$ | H | (Öl) |

Tabelle 1-Fortsetzung

| Beispiel Nr. | $R^1$ | $R^2$ | $R^3$ | $R^4$ | $R^5$ | M | Schmelz-punkt(°C) |
|---|---|---|---|---|---|---|---|
| 12 | 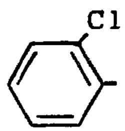 | 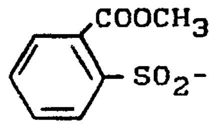 | $OCH_3$ | $CH_3$ | $OCH_3$ | H | (amorph) |
| 13 | 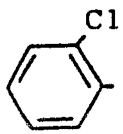 | 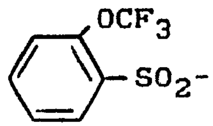 | $OCH_3$ | $CH_3$ | $OCH_3$ | H | 172 |
| 14 | 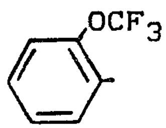 | 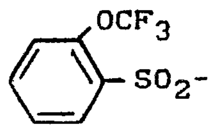 | $OCH_3$ | $CH_3$ | $OCH_3$ | H | |
| 15 | 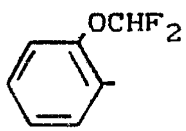 | 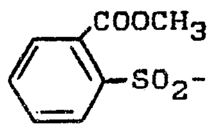 | $OCH_3$ | $OCH_3$ | $OCH_3$ | H | |
| 16 | 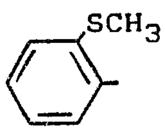 | 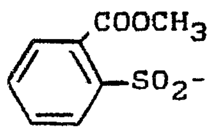 | $OCH_3$ | $OCH_3$ | $CH_3$ | H | |
| 17 | 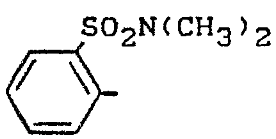 | 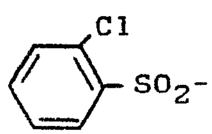 | $OCH_3$ | $OCH_3$ | $CH_3$ | H | |

Tabelle 1-Fortsetzung

| Beispiel Nr. | $R^1$ | $R^2$ | $R^3$ | $R^4$ | $R^5$ | M | Schmelzpunkt (°C) |
|---|---|---|---|---|---|---|---|
| 18 | 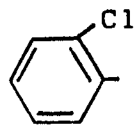 | 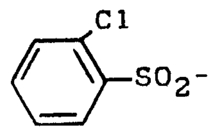 | $OCH_3$ | $CH_3$ | $CH_3$ | H | |
| 19 | 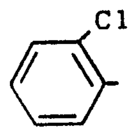 | H | $OCH_3$ | $CH_3$ | $N(CH_3)_2$ | H | |
| 20 | 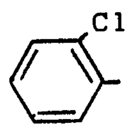 | H | $OCH_3$ | $CH_3$ | $OCH_3$ | H | 122 |
| 21 | 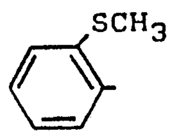 | H | $OCH_3$ | $CH_3$ | $OCH_3$ | H | (amorph) |
| 22 | 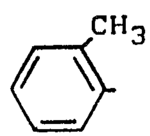 | H | $OCH_3$ | $OCH_3$ | $OCH_3$ | H | 105 |
| 23 | 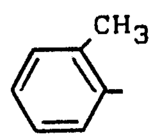 | H | $OCH_3$ | $CH_3$ | $OC_2H_5$ | H | 144 |

Tabelle 1-Fortsetzung

| Beispiel Nr. | $R^1$ | $R^2$ | $R^3$ | $R^4$ | $R^5$ | M | Schmelzpunkt (°C) |
|---|---|---|---|---|---|---|---|
| 24 | 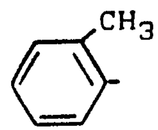 | H | $OCH_3$ | $CH_3$ | $CH_3$ | H | 132 |
| 25 | 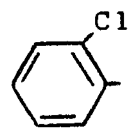 | H | $OCH_3$ | $CH_3$ | $CH_3$ | H | 165 |
| 26 | 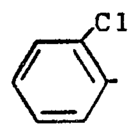 | H | $OCH_3$ | $OCH_3$ | $OCH_3$ | H | |
| 27 | 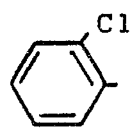 | H | $-OCH_2$ 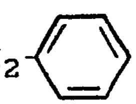 | $CH_3$ | $OCH_3$ | H | 115 |
| 28 | 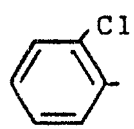 | 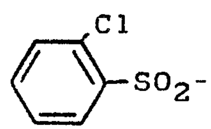 | $OCH(CH_3)_2$ | $OCH_3$ | $OCH_3$ | H | |
| 29 | 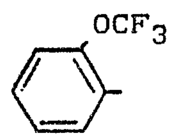 | H | $OCH_3$ | $CH_3$ | $OCH_3$ | H | |

Tabelle 1-Fortsetzung

| Beispiel Nr. | $R^1$ | $R^2$ | $R^3$ | $R^4$ | $R^5$ | M | Schmelz-punkt(°C) |
|---|---|---|---|---|---|---|---|
| 30 | 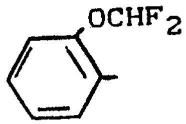 | H | $OCH_3$ | $OCH_3$ | $OCH_3$ | H | |
| 31 | 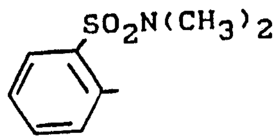 | H | $OCH_3$ | $CH_3$ | $OCH_3$ | H | |
| 32 | 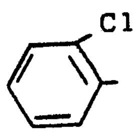 | H | $N(CH_3)_2$ | $CH_3$ | $OCH_3$ | H | 154 |
| 33 | 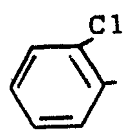 | H | $N(CH_3)_2$ | $OCH_3$ | $OCH_3$ | H | 127 |
| 34 | 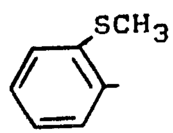 | H | $N(CH_3)_2$ | $CH_3$ | $OCH_3$ | H | 151 |
| 35 | 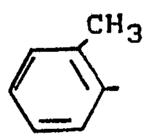 | H | $N(CH_3)_2$ | $OCH_3$ | $OCH_3$ | H | 150 |

Tabelle 1-Fortsetzung

| Beispiel Nr. | $R^1$ | $R^2$ | $R^3$ | $R^4$ | $R^5$ | M | Schmelzpunkt (°C) |
|---|---|---|---|---|---|---|---|
| 36 | 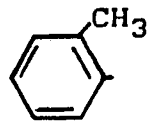 | H | $N(CH_3)_2$ | $CH_3$ | $OC_2H_5$ | H | 208 |
| 37 | 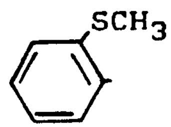 | 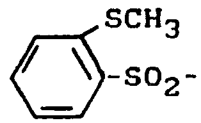 | $OCH_3$ | $CH_3$ | $OCH_3$ | H | |
| 38 | 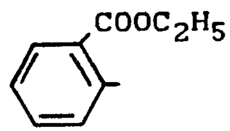 | H | $-CH_2CH=CH_2$ | $OCH_3$ | $OCH_3$ | H | |
| 39 | 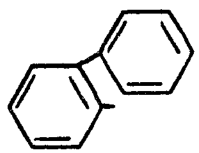 | H | 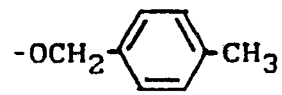 | $OCH_3$ | $OCH_3$ | H | |
| 40 | 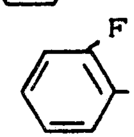 | H | $-OCH_2COOC_2H_5$ | $OCH_3$ | $OCH_3$ | H | |
| 41 | 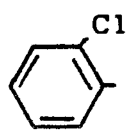 | 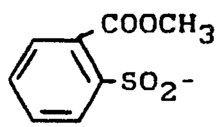 | $OCH_3$ | $C_2H_5$ | $OCH_3$ | H | (amorph) |

Tabelle 1-Fortsetzung

| Beispiel Nr. | $R^1$ | $R^2$ | $R^3$ | $R^4$ | $R^5$ | M | Schmelz-punkt(°C) |
|---|---|---|---|---|---|---|---|
| 42 | 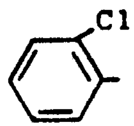 | 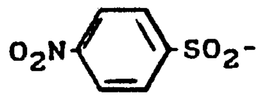 | $OCH_3$ | $CH_3$ | $OCH_3$ | H | 207 |
| 43 | 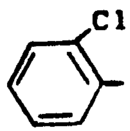 | 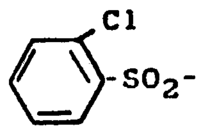 | $OCH_3$ | $C_2H_5$ | $OCH_3$ | H | (amorph) |
| 44 | 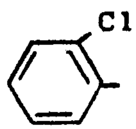 | H | $OCH_3$ | $C_2H_5$ | $OCH_3$ | H | 115 |
| 45 | 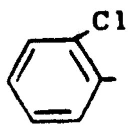 | H | $-N(CH_3)_2$ | $C_2H_5$ | $OCH_3$ | H | 173 |
| 46 | 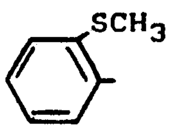 | 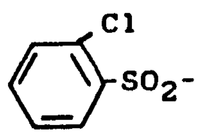 | $OCH_3$ | $CH_3$ | $OCH_3$ | H | (amorph) |
| 47 | 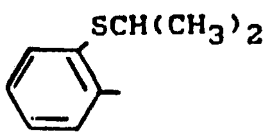 | H | $OCH_3$ | $CH_3$ | $OCH_3$ | H | 164 |

Tabelle 1-Fortsetzung

| Beispiel Nr. | $R^1$ | $R^2$ | $R^3$ | $R^4$ | $R^5$ | M | Schmelzpunkt (°C) |
|---|---|---|---|---|---|---|---|
| 48 | 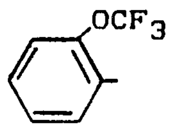 | H | $-OCH_2-$ 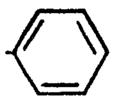 | $CH_3$ | $OC_2H_5$ | H | 136 |
| 49 | 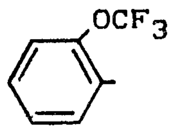 | H | $-N(CH_3)_2$ | $CH_3$ | $OC_2H_5$ | H | |
| 50 | 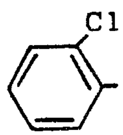 | 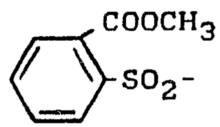 | $OCH_3$ | $CH_3$ | $CH_3$ | H | |
| 51 | 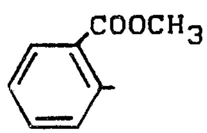 | 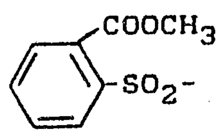 | $-OCH_2-$ 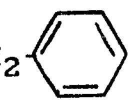 | $CH_3$ | $OC_2H_5$ | H | (amorph) |
| 52 | 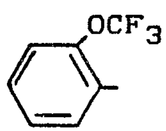 | 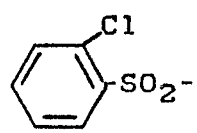 | $-OCH_2-$ 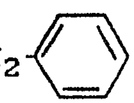 | $CH_3$ | $OC_2H_5$ | H | 153 |
| 53 | 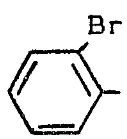 | 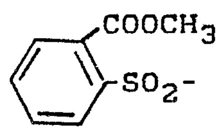 | $OCH_3$ | $CH_3$ | $OCH_3$ | H | |

Tabelle 1-Fortsetzung

| Beispiel Nr. | $R^1$ | $R^2$ | $R^3$ | $R^4$ | $R^5$ | M | Schmelzpunkt (°C) |
|---|---|---|---|---|---|---|---|
| 54 | 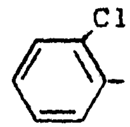 | 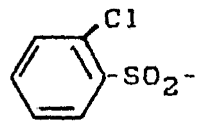 | OH | $CH_3$ | $OCH_3$ | H | 208 |
| 55 | 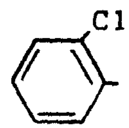 | 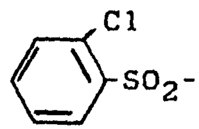 | 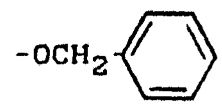 | $CH_3$ | $OC_2H_5$ | H | |
| 56 | 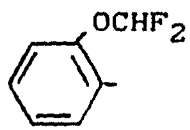 | 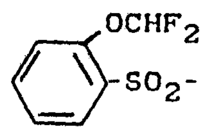 | $OCH_3$ | $CH_3$ | $OCH_3$ | H | |
| 57 | 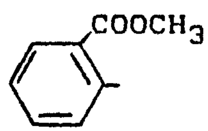 | 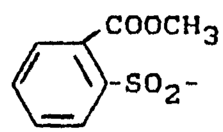 | $OCH_3$ | $CH_3$ | $OCH_3$ | H | |
| 58 | 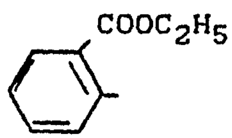 | 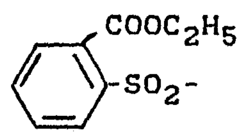 | $OCH_3$ | $CH_3$ | $OCH_3$ | H | |
| 59 | 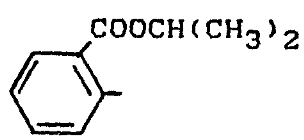 | 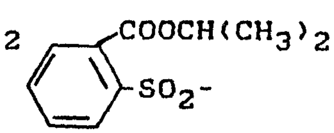 | $OCH_3$ | $CH_3$ | $OCH_3$ | H | |

Tabelle 1-Fortsetzung

| Beispiel Nr. | $R^1$ | $R^2$ | $R^3$ | $R^4$ | $R^5$ | M | Schmelz-punkt(°C) |
|---|---|---|---|---|---|---|---|
| 60 | 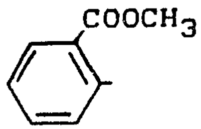 | 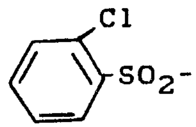 | $OCH_3$ | $CH_3$ | $OCH_3$ | H | |
| 61 | 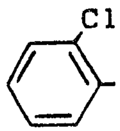 | 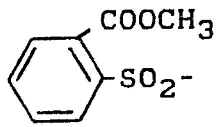 | $OCH_3$ | $CH_3$ | $OCH_3$ | H | |
| 62 | 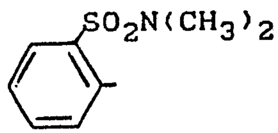 | 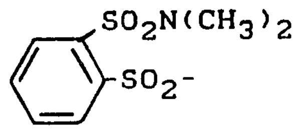 | $OCH_3$ | $CH_3$ | $OCH_3$ | H | |
| 63 | 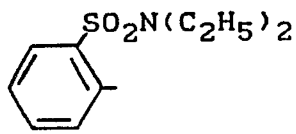 | 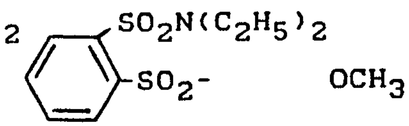 | $OCH_3$ | $CH_3$ | $OCH_3$ | H | |
| 64 | 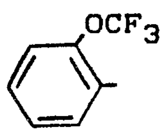 | 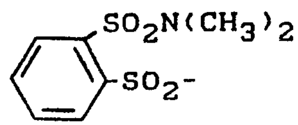 | $OCH_3$ | $CH_3$ | $OCH_3$ | H | |
| 65 | 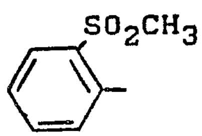 | 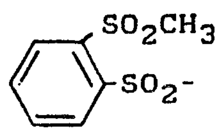 | $OCH_3$ | $CH_3$ | $OCH_3$ | H | |

Tabelle 1-Fortsetzung

| Beispiel Nr. | $R^1$ | $R^2$ | $R^3$ | $R^4$ | $R^5$ | M | Schmelzpunkt (°C) |
|---|---|---|---|---|---|---|---|
| 66 | 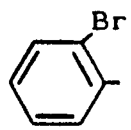 | 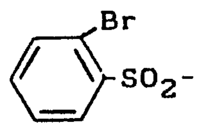 | $OCH_3$ | $CH_3$ | $OCH_3$ | H | |
| 67 | 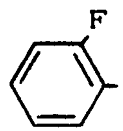 | 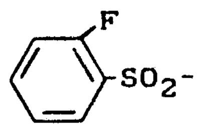 | $OCH_3$ | $CH_3$ | $OCH_3$ | H | |
| 68 | 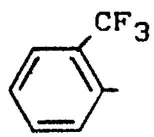 | 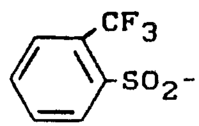 | $OC_2H_5$ | $CH_3$ | $OCH_3$ | H | |
| 69 | 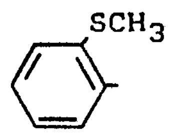 | 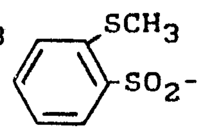 | 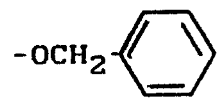 | $CH_3$ | $OCH_3$ | H | |
| 70 | 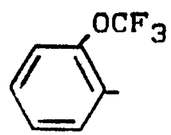 | 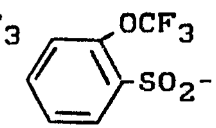 | 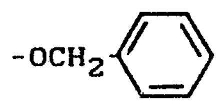 | $CH_3$ | $OCH_3$ | H | |
| 71 | 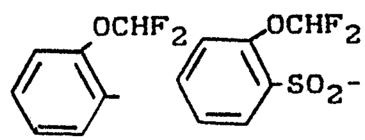 | | 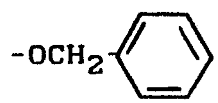 | $CH_3$ | $OCH_3$ | H | |

Tabelle 1-Fortsetzung

| Beispiel Nr. | $R^1$ | $R^2$ | $R^3$ | $R^4$ | $R^5$ | M | Schmelz-punkt(°C) |
|---|---|---|---|---|---|---|---|
| 72 | 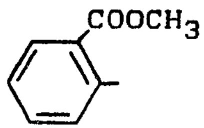 | 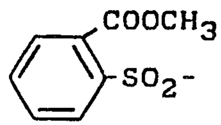 | $-OCH_2-$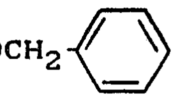 | $CH_3$ | $OCH_3$ | H | |
| 73 | 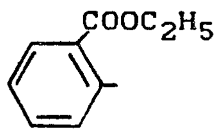 | 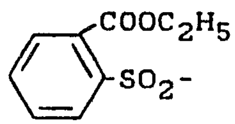 | $-OCH_2-$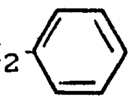 | $CH_3$ | $OCH_3$ | H | |
| 74 | 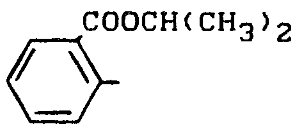 | 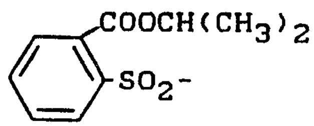 | $-OCH_2-$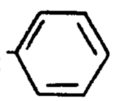 | $CH_3$ | $OCH_3$ | H | |
| 75 | 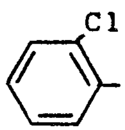 | 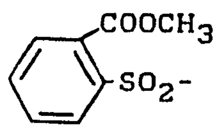 | $-OCH_2-$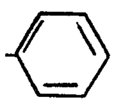 | $CH_3$ | $OCH_3$ | H | |
| 76 | 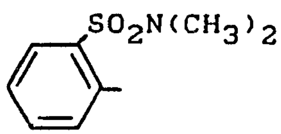 | 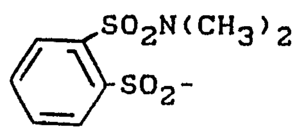 | $-OCH_2-$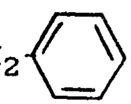 | $CH_3$ | $OCH_3$ | H | |
| 77 | 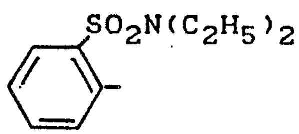 | 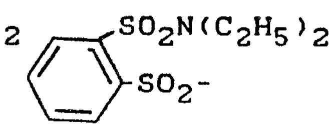 | $-OCH_2-$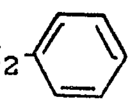 | $CH_3$ | $OCH_3$ | H | |

Tabelle 1-Fortsetzung

| Beispiel Nr. | $R^1$ | $R^2$ | $R^3$ | $R^4$ | $R^5$ | M | Schmelzpunkt (°C) |
|---|---|---|---|---|---|---|---|
| 78 | 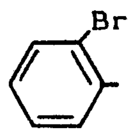 | 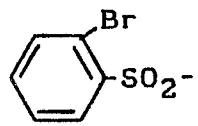 | 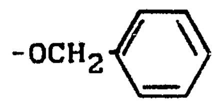 | $CH_3$ | $OCH_3$ | H | |
| 79 | 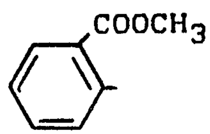 | 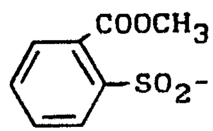 | $-OCH_2CH(CH_3)_2$ | $CH_3$ | $OCH_3$ | H | |
| 80 | 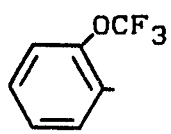 | 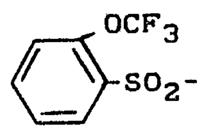 | $-OCH_2CH(CH_3)_2$ | $CH_3$ | $OCH_3$ | H | |
| 81 | 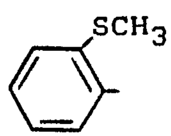 | 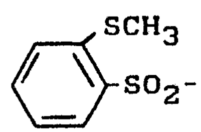 | $-OCH_2CH(CH_3)_2$ | $CH_3$ | $OCH_3$ | H | |
| 82 | 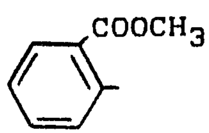 | 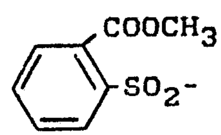 | $-OCH_2CH=CH_2$ | $CH_3$ | $OCH_3$ | H | |
| 83 | 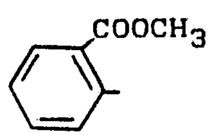 | H | 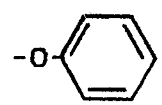 | $CH_3$ | $OCH_3$ | H | |

Tabelle 1-Fortsetzung

| Beispiel Nr. | $R^1$ | $R^2$ | $R^3$ | $R^4$ | $R^5$ | M | Schmelzpunkt (°C) |
|---|---|---|---|---|---|---|---|
| 84 | 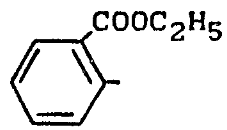 | 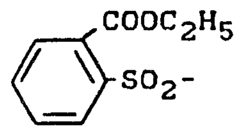 | $OCH_3$ | $CH_3$ | $OC_2H_5$ | H | |
| 85 | 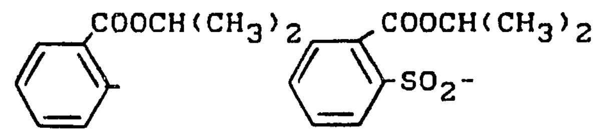 | | $OCH_3$ | $CH_3$ | $OC_2H_5$ | H | |
| 86 | 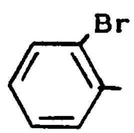 | 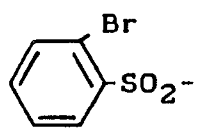 | $OCH_3$ | $CH_3$ | $OC_2H_5$ | H | |
| 87 | 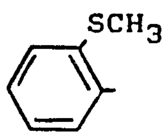 | 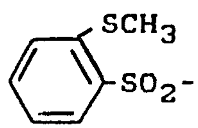 | $OCH_3$ | $CH_3$ | $OC_2H_5$ | H | |
| 88 | 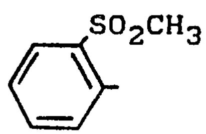 | 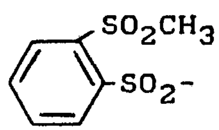 | $OCH_3$ | $CH_3$ | $OC_2H_5$ | H | |
| 89 | 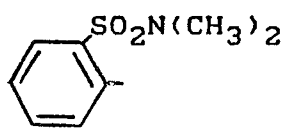 | 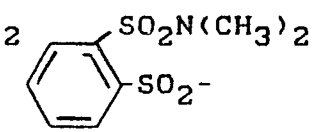 | $OCH_3$ | $CH_3$ | $OC_2H_5$ | H | |

Tabelle 1-Fortsetzung

| Beispiel Nr. | $R^1$ | $R^2$ | $R^3$ | $R^4$ | $R^5$ | M | Schmelzpunkt (°C) |
|---|---|---|---|---|---|---|---|
| 90 | 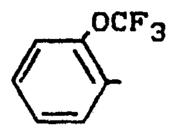 | 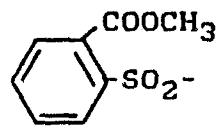 | $OCH_3$ | $CH_3$ | $OC_2H_5$ | H | |
| 91 | 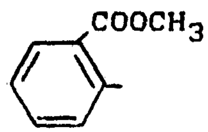 | 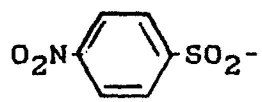 | $OCH_3$ | $CH_3$ | $OC_2H_5$ | H | |
| 92 | 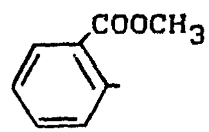 | H | $-N(CH_3)_2$ | $CH_3$ | $OCH_3$ | H | |
| 93 | 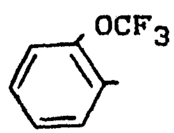 | 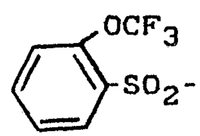 | $-OCH_2CH=CH_2$ | $CH_3$ | $OCH_3$ | H | |
| 94 | 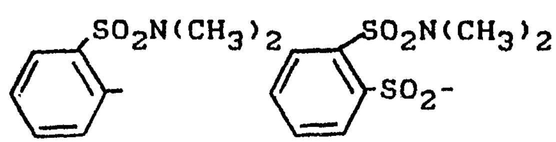 | | $-OCH_2CH=CH_2$ | $CH_3$ | $OCH_3$ | H | |
| 95 | 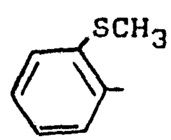 | 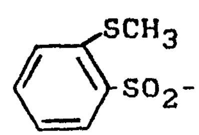 | $-OCH_2CH=CH_2$ | $CH_3$ | $OCH_3$ | H | |

Tabelle 1-Fortsetzung

| Beispiel Nr. | $R^1$ | $R^2$ | $R^3$ | $R^4$ | $R^5$ | M | Schmelzpunkt (°C) |
|---|---|---|---|---|---|---|---|
| 96 | 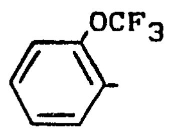 | 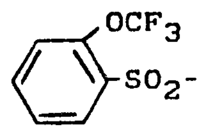 | $OCH_3$ | $OCH_3$ | $OCH_3$ | H | |
| 97 | 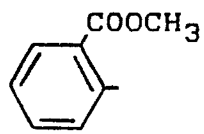 | 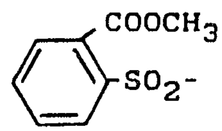 | $OCH_3$ | $OCH_3$ | $OCH_3$ | H | |
| 98 | 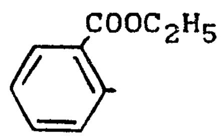 | 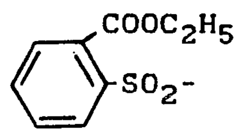 | $OCH_3$ | $OCH_3$ | $OCH_3$ | H | |
| 99 | 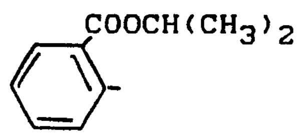 | 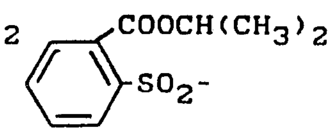 | $OCH_3$ | $OCH_3$ | $OCH_3$ | H | |
| 100 | 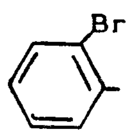 | 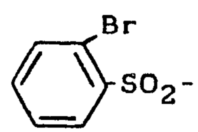 | $OCH_3$ | $OCH_3$ | $OCH_3$ | H | |
| 101 | 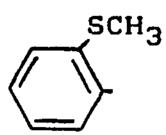 | 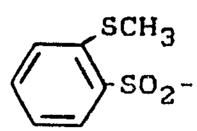 | $OCH_3$ | $OCH_3$ | $OCH_3$ | H | |

Tabelle 1-Fortsetzung

| Beispiel Nr. | $R^1$ | $R^2$ | $R^3$ | $R^4$ | $R^5$ | M | Schmelzpunkt(°C) |
|---|---|---|---|---|---|---|---|
| 102 | 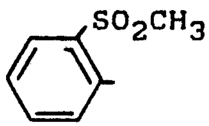 | 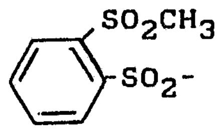 | $OCH_3$ | $OCH_3$ | $OCH_3$ | H | |
| 103 | 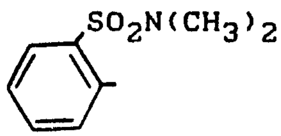 | 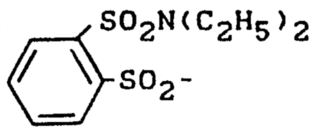 | $OCH_3$ | $OCH_3$ | $OCH_3$ | H | |
| 104 | 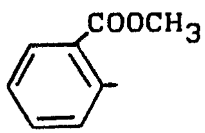 | 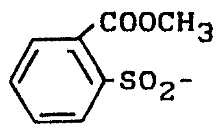 | $OCH_3$ | $CH_3$ | $CH_3$ | H | |
| 105 | 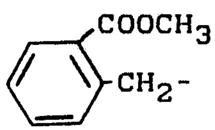 | 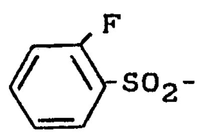 | $OCH_3$ | $OCH_3$ | $OCH_3$ | H | |
| 106 | 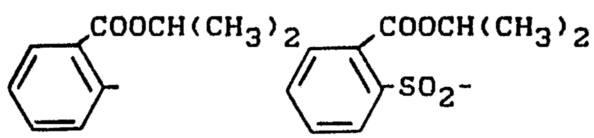 | | $OCH_3$ | $CH_3$ | $CH_3$ | H | |
| 107 | 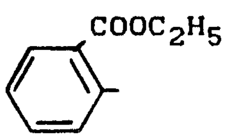 | 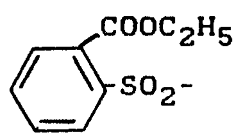 | $OCH_3$ | $CH_3$ | $CH_3$ | H | |

Tabelle 1-Fortsetzung

| Beispiel Nr. | $R^1$ | $R^2$ | $R^3$ | $R^4$ | $R^5$ | M | Schmelzpunkt (°C) |
|---|---|---|---|---|---|---|---|
| 108 | 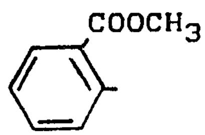 | 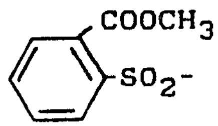 | 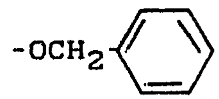 | $CH_3$ | $CH_3$ | H | |
| 109 | 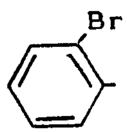 | 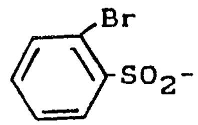 | $-OCH(CH_3)_2$ | $CH_3$ | $CH_3$ | H | |
| 110 | 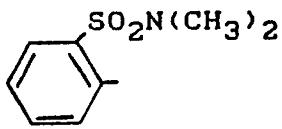 | 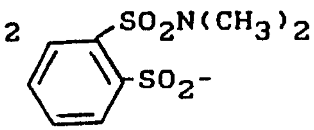 | $OCH_3$ | $CH_3$ | $CH_3$ | H | |
| 111 | 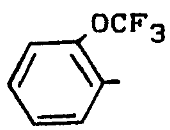 | 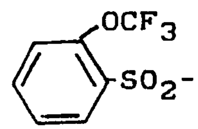 | $-OCH_2CH=CH_2$ | $CH_3$ | $CH_3$ | H | |
| 112 | 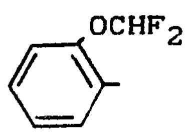 | 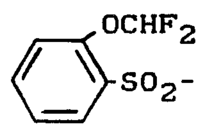 | $-OCH_2CH(CH_3)_2$ | $CH_3$ | $CH_3$ | H | |
| 113 | 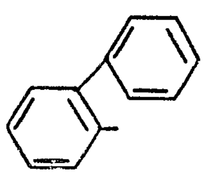 | 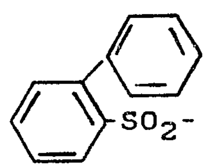 | $OCH_3$ | $OCH_3$ | $OCH_3$ | H | |

Tabelle 1-Fortsetzung

| Beispiel Nr. | $R^1$ | $R^2$ | $R^3$ | $R^4$ | $R^5$ | M | Schmelzpunkt (°C) |
|---|---|---|---|---|---|---|---|
| 114 | 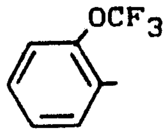 | $CH_3-SO_2-$ | $-OCH_2-$ 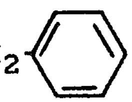 | $CH_3$ | $OCH_3$ | H | |
| 115 | 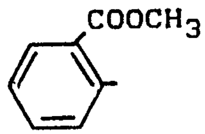 | $C_4H_9-SO_2-$ | $OCH_3$ | $OCH_3$ | $OCH_3$ | H | |
| 116 | 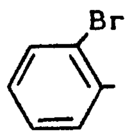 | $C_8F_{17}-SO_2-$ | $OCH_3$ | $CH_3$ | $CH_3$ | H | |
| 117 | 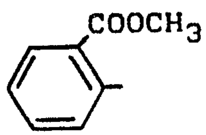 | H | $-OCH_2CH_2CH_2Cl$ | $CH_3$ | $OCH_3$ | H | |

(2a) 1:1-Addukt von Beispiel (2) mit p-Toluolsulfonsäure (Öl).

(44a) 1:1-Addukt von Beispiel (44) mit Schwefelsäure (amorph).

(45a) 1:1-Addukt von Beispiel (45) mit Schwefelsäure vom Schmelzpunkt 163°C.

(49a) 1:1-Addukt von Beispiel (49) mit Schwefelsäure (amorph).

## Herstellung von Ausgangsverbindungen der Formel (II)

### Beispiel (II-1)

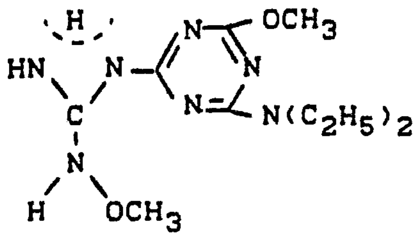

Eine Mischung aus 66,5 g (0,3 Mol) 2-Cyanamino-4-diethylamino-6-methoxy-s-triazin, 50 g (0,8 Mol) O-Methylhydroxylamin-Hydrochlorid und 300 ml Ethanol wird 15 Stunden unter Rückfluß zum Sieden erhitzt. Dann wird filtriert, das Filtrat eingeengt, der Rückstand in ca. 200 ml Wasser aufgenommen, mit Natronlauge alkalisch gestellt und das hierbei kristallin anfallende Produkt durch Absaugen isoliert.

Man erhält 35,0 g (43 % der Theorie) N'-(4-Diethylamino-6-methoxy-s-triazin-2-yl)-N"-methoxy-guanidin vom Schmelzpunkt 112°C.

Analog können die in der nachstehenden Tabelle 2 aufgeführten Verbindungen der Formel (II) hergestellt werden:

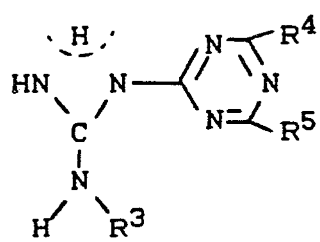

(II)

Tabelle 2-

| Bsp. Nr. | $R^3$ | $R^4$ | $R^5$ | Schmelzpunkt (°C) |
|---|---|---|---|---|
| II- 2 | $OCH_3$ | $SCH_3$ | $NHC_2H_5$ | 117 |
| II- 3 | $OCH_3$ | $SCH_3$ | $NHCH_3$ | |
| II- 4 | $OCH_3$ | Cl | $N(C_2H_5)_2$ | |
| II- 5 | $OC_2H_5$ | $OCH_3$ | $N(C_2H_5)_2$ | |
| II- 6 | $OC_3H_7$-n | $SCH_3$ | $NHC_2H_5$ | |
| II- 7 | $OCH(CH_3)_2$ | $OCH_3$ | $NHCH_3$ | |
| II- 8 | $OCH_2-CH=CH_2$ | $OCH_3$ | $N(CH_3)_2$ | |
| II- 9 | $OC_4H_9$-n | $OCH_3$ | $N(CH_3)_2$ | |
| II-10 | $OCH_2$ 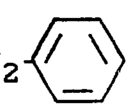 | $OCH_3$ | $N(CH_3)_2$ | |

Tabelle 2-Fortsetzung

| Bsp. Nr. | $R^3$ | $R^4$ | $R^5$ | Schmelzpunkt (°C) |
|---|---|---|---|---|
| II-11 | $OCH_2COOC_2H_5$ | $OCH_3$ | $N(CH_3)_2$ | |
| II-12 | $OCH_2$- 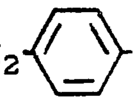 -$CH_3$ | $OCH_3$ | $N(CH_3)_2$ | |
| II-13 | $OCH_3$ | $CH_3$ | $OCH_3$ | 126 |
| II-14 | $OCH_2$- 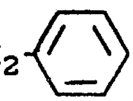 | $CH_3$ | $OCH_3$ | 112 |
| II-15 | $OC_8H_{17}$ | $CH_3$ | $OCH_3$ | 95 |
| II-16 | $OCH_2$- 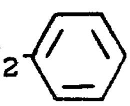 | $SCH_3$ | $NHC_2H_5$ | 122 |
| II-17 | $OCH_3$ | $CH_3$ | $OC_2H_5$ | |
| II-18 | $OCH_2$- 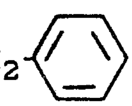 | $CH_3$ | $OC_2H_5$ | (amorph) |
| II-19 | $OCH_3$ | $CH_3$ | $CH_3$ | |

Tabelle 2-Fortsetzung

| Bsp. Nr. | $R^3$ | $R^4$ | $R^5$ | Schmelzpunkt (°C) |
| --- | --- | --- | --- | --- |
| II-20 | $OCH_2$- 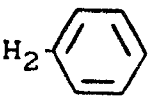 | $CH_3$ | $CH_3$ | |
| II-21 | $OCH_2-CH=CH_2$ | $CH_3$ | $CH_3$ | |
| II-22 | $OCH_3$ | $OCH_3$ | $OCH_3$ | |
| II-23 | $OCH_2$- 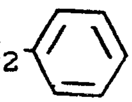 | $OCH_3$ | $OCH_3$ | |
| II-24 | $OCH_2-C(CH_3)_3$ | $CH_3$ | $CH_3$ | |
| II-25 | $OCH_2CH_2-OCH_3$ | $OCH_3$ | $OCH_3$ | |
| II-26 | $OCH_2CH_2-OC_2H_5$ | $CH_3$ | $OCH_3$ | |
| II-27 | $OCH_2CH_2-SCH_3$ | $CH_3$ | $OC_2H_5$ | |
| II-28 | $OCH_2CH_2-SC_2H_5$ | $CH_3$ | $CH_3$ | |

Tabelle 2-Fortsetzung

| Bsp. Nr. | $R^3$ | $R^4$ | $R^5$ | Schmelzpunkt (°C) |
| --- | --- | --- | --- | --- |
| II-29 | $OCH_2-CONH_2$ | $OCH_3$ | $OCH_3$ | |
| II-30 | $OCH_2-CH=CHCl$ | $CH_3$ | $OCH_3$ | |
| II-31 | OCH 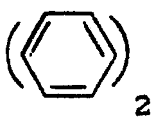 | $CH_3$ | $CH_3$ | |
| II-32 | OC 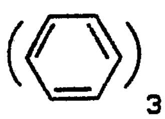 | $CH_3$ | $CH_3$ | |

Herstellung von Ausgangsverbindungen der Formel (III)

Beispiel (III-1)

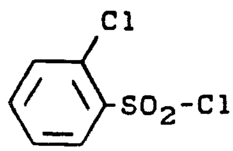

295 ml Phosphorylchlorid ("Phosphoroxychlorid") werden bei 20°C bis 30°C tropfenweise zu einer Mischung aus 172 g (0,8 Mol) 2-Chlor-benzolsulfonsäure-Natriumsalz, 300 ml Acetonitril und 300 ml Sulfolan gegeben. Das Reaktionsgemisch wird 4 Stunden bei 70°C gerührt, anschließend auf 5°C abgekühlt und mit Eiswasser verdünnt. Nach Extrahieren mit Petrolether, Waschen der Extraktionslösung mit Wasser, Trocknen, Filtrieren und Einengen wird das im Rückstand verbliebene Produkt durch Vakuumdestillation gereinigt.

Man erhält 117 g (70 % der Theorie) 2-Chlor-benzolsulfonsäurechlorid vom Siedepunkt 110°C/1,1 mbar.

Beispiel (III-2)

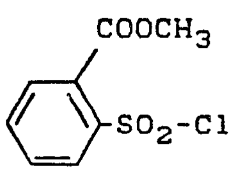

75,5 g (0,5 Mol) 2-Aminobenzoesäuremethylester werden in 176 ml konzentrierter Salzsäure und 100 ml Essigsäure gelöst. Hierzu wird bei 0°C eine Lösung von 34,4 g Natriumnitrit in 70 ml Wasser getropft. Nach 15 minütigem Nachrühren wird das Reaktionsgemisch langsam zu einer auf 0°C gekühlten gesättigten Lösung von Schwefeldioxid in 450 ml Essigsäure gegeben. Nach Entfernung des Kühlbades wird bis zum Ende der Gasentwicklung gerührt, wobei 10 g Kupfer(II)-chlorid portionsweise eingetragen werden. Nach Verdünnen mit Eiswasser, Extrahieren mit Methylenchlorid, Waschen der Extraktionslösung mit Wasser, Trocknen, Filtrieren und Einengen wird das im Rückstand verbliebene Produkt durch Vakuumdestillation gereinigt.

Man erhält 45 g (38 % der Theorie) 2-Methoxycarbonylbenzolsulfonsäurechlorid vom Siedepunkt 150°C/1,33 mbar.

Analog können die in der nachstehenden Tabelle 3 aufgeführten Verbindungen der Formel (III) hergestellt werden:

$$R^1-SO_2-Cl \qquad (III)$$

Tabelle 3

| Bsp.-Nr. | $R^1$ | Siedepunkt/Druck |
|---|---|---|
| III- 3 | 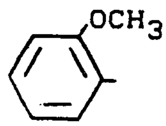 | (Öl, Zersetzung bei Destillation) |
| III- 4 | 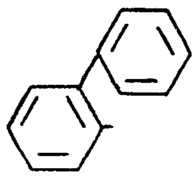 | [Schmelzpunkt: 100°C] |
| III- 5 | 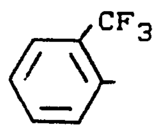 | (Öl) |
| III- 6 | 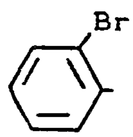 | 142°C/4 mbar |
| III- 7 | 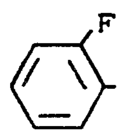 | 106°C/4 mbar |
| III- 8 | 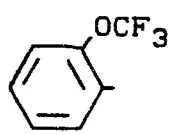 | [Schmelzpunkt: 32°C] |
| III- 9 | 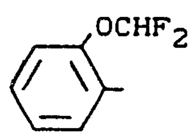 | (Öl, Zersetzung bei Destillation) |
| III-10 | 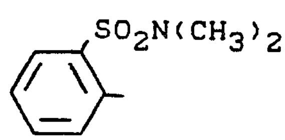 | [Schmelzpunkt: 103°C] |
| III-11 | 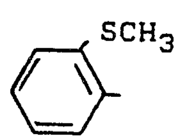 | (Öl, Zersetzung bei Destillation) |

Tabelle 3-Fortsetzung

| Bsp.-Nr. | $R^1$ | Siedepunkt/Druck |
|---|---|---|
| III-12 | 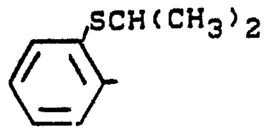 | 90°C/ 1,33 mbar |
| III-13 | 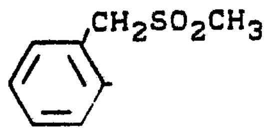 | [Schmelzpunkt:120°C] |
| III-14 | 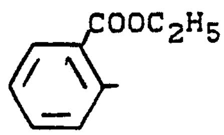 | 155°C/ 5,32 mbar |
| III-15 | 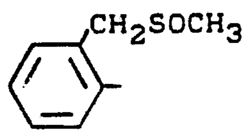 | |
| III-16 | 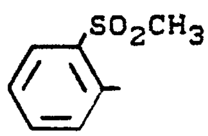 | [Schmelzpunkt:128°C] |
| III-17 | 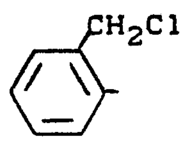 | |

Herstellung von Ausgangsstoffen der Formel (VI)

Beispiel (VI-1)

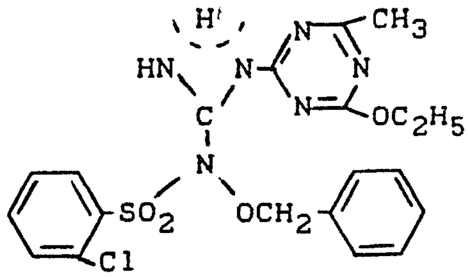

4,2 g (0,02 Mol) 2-Chlor-benzolsulfonsäurechlorid werden zu einer auf 0°C bis 5°C gekühlten Mischung aus 6,0 g (0,02 Mol) N'-(4-Ethoxy-6-methyl-s-triazin-2-yl)-N"-benzyloxy-guanidin und 30 ml Pyridin gegeben und das Gemisch wird 48 Stunden bei 20°C gerührt. Nach Einengen wird der Rückstand in Methylenchlorid aufgenommen, mit 2N-Salzsäure gewaschen, getrocknet, filtriert und sorgfältig eingeengt.

Man erhält 6,1 g (64 % der Theorie) N'-(4-Ethoxy-6-methyl-s-triazin-2-yl)-N"-benzyloxy-N"-(2-chlor-benzolsulfonyl)-guanidin als öligen Rückstand.

Analog können die in der nachstehenden Tabelle 4 aufgeführten Verbindungen der Formel (VI) hergestellt werden:

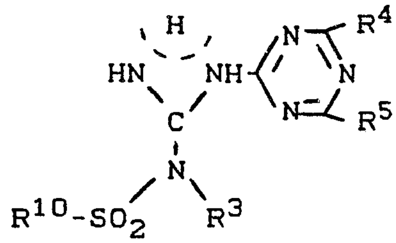

(VI)

Tabelle 4

| Beispiel Nr. | $R^{10}$ | $R^3$ | $R^4$ | $R^5$ | Schmelzpunkt (°C) |
|---|---|---|---|---|---|
| VI-2 | 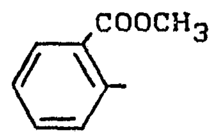 | $-OCH_2-$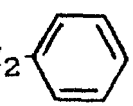 | $CH_3$ | $OCH_3$ | (amorph) |
| VI-3 | 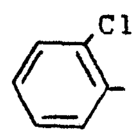 | $OCH_3$ | $CH_3$ | $OCH_3$ | |
| VI-4 | $CH_3-$ | $-OCH_2CH=CH_2$ | $OCH_3$ | $OCH_3$ | |
| VI-5 | $C_8F_{17}-$ | $-OCH_2CH(CH_3)_2$ | $CH_3$ | $CH_3$ | |
| VI-6 | $C_4H_9-$ | $OC_2H_5$ | $CH_3$ | $OC_2H_5$ | |
| VI-7 | $(CH_3)_2N-$ | $OCH_2CH_2CH_2CH_3$ | $CH_3$ | $OCH_3$ | |

Herstellung von Ausgangsstoffen der Formel (VII)

Beispiel (VII-1)

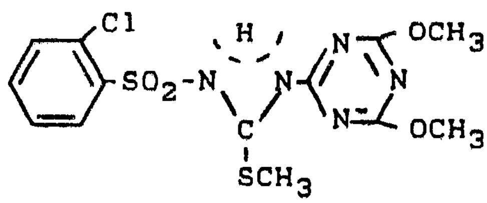

11g (0,4 Mol) Natriumhydrid (80%-ig) werden bei 20°C portionsweise zu einer Suspension von 31,2 g (0,2 Mol) 2-Amino-4,6-dimethoxy-s-triazin in 200 ml Tetrahydrofuran gegeben. Nach zwölfstündigem Rühren werden 60 g (0,2 Mol) N-(2-Chlor-benzolsulfonyl)-S',S''-dimethyl-imino-dithiokohlensäureester dazugegeben, wobei die Reakionstemperatur auf 60°C ansteigt. Das Reaktionsgemisch wird 5 Stunden bei 20°C gerührt, mit 800 ml Wasser verdünnt und filtriert. Nach Ansäuern mit konzentrierter Salzsäure kristallisiert das Produkt und wird durch Absaugen isoliert.

Man erhält 42 g (48 % der Theorie) N'-(4,6-Dimethoxy-s-triazin-2-yl)-N"-(2-chlor-benzolsulfonyl)-S-methyl-isothioharnstoff vom Schmelzpunkt 176°C.

Analog können die in der nachstehenden Tabelle 5 aufgeführten Verbindungen der Formel (VII) hergestellt werden:

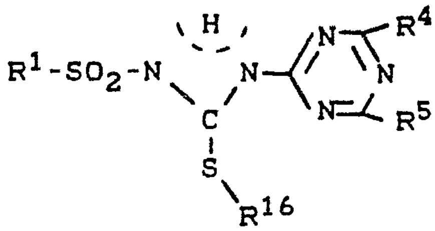

(VII)

Tabelle 5

| Bsp. Nr. | $R^1$ | $R^4$ | $R^5$ | $R^{16}$ | Schmelzpunkt (°C) |
|---|---|---|---|---|---|
| VII- 2 | 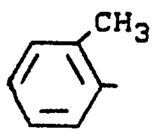 | $OCH_3$ | $OCH_3$ | $CH_3$ | 159 |
| VII- 3 | 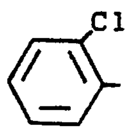 | $CH_3$ | $N(CH_3)_2$ | $CH_3$ | 175 |
| VII- 4 | 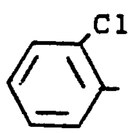 | $CH_3$ | $OCH_3$ | $CH_3$ | 148 |
| VII- 5 | 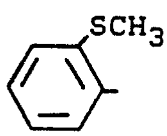 | $CH_3$ | $OCH_3$ | $CH_3$ | 167 |
| VII- 6 | 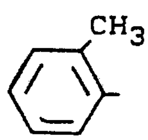 | 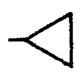 | 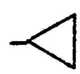 | $CH_3$ | 170 |
| VII- 7 | 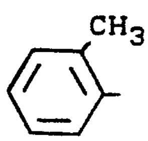 | $CH_3$ | $OC_2H_5$ | $CH_3$ | 104 |
| VII- 8 | 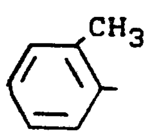 | $CH_3$ | $CH_3$ | $CH_3$ | 119 |

Tabelle 5-Fortsetzung

| Bsp. Nr. | $R^1$ | $R^4$ | $R^5$ | $R^{16}$ | Schmelzpunkt (°C) |
|---|---|---|---|---|---|
| VII- 9 | 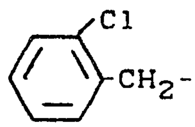 | $CH_3$ | $CH_3$ | $CH_3$ | 165 |
| VII-10 | 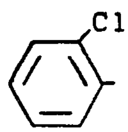 | $CH_3$ | $CH_3$ | $CH_3$ | 151 |
| VII-11 | 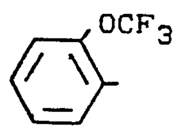 | $CH_3$ | $OCH_3$ | $CH_3$ | |
| VII-12 | 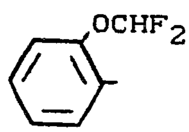 | $OCH_3$ | $OCH_3$ | $CH_3$ | |
| VII-13 | 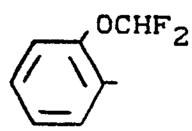 | $CH_3$ | $OCH_3$ | $CH_3$ | |
| VII-14 | 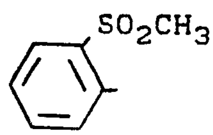 | $CH_3$ | $OCH_3$ | $CH_3$ | |
| VII-15 | 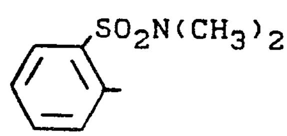 | $CH_3$ | $OCH_3$ | $CH_3$ | |
| VII-16 | 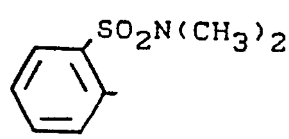 | $OCH_3$ | $OCH_3$ | $CH_3$ | |
| VII-17 | 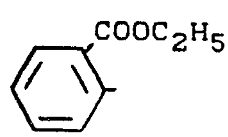 | $OCH_3$ | $OCH_3$ | $CH_3$ | |

| Bsp. Nr. | $R^1$ | $R^4$ | $R^5$ | $R^{16}$ | Schmelzpunkt (°C) |
|---|---|---|---|---|---|
| VII-18 | 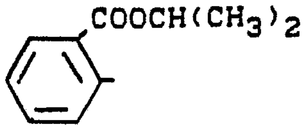 | $OCH_3$ | $CH_3$ | $CH_3$ | |
| VII-19 | 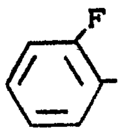 | $CH_3$ | $OCH_3$ | $CH_3$ | |
| VII-20 | 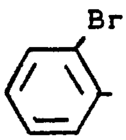 | $CH_3$ | $OCH_3$ | $CH_3$ | |
| VII-21 | 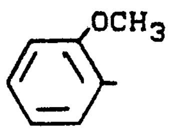 | $OCH_3$ | $OCH_3$ | $CH_3$ | |
| VII-22 | 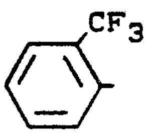 | $OCH_3$ | $CH_3$ | $CH_3$ | |
| VII-23 | 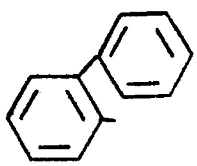 | $CH_3$ | $OCH_3$ | $CH_3$ | |
| VII-24 | 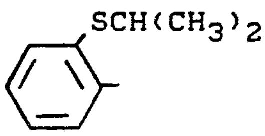 | $CH_3$ | $OCH_3$ | $CH_3$ | 136 |
| VII-25 | 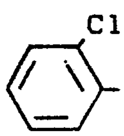 | $C_2H_5$ | $OCH_3$ | $CH_3$ | 132 |
| VII-26 | 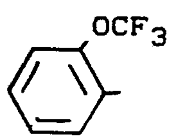 | $CH_3$ | $OC_2H_5$ | $CH_3$ | 103 |

Herstellung von Ausgangsstoffen der Formel (VIII)

Beispiel (VIII-1)

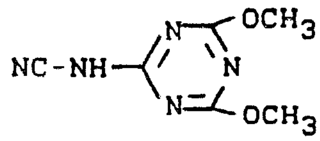

52,7 g (0,3 Mol) 2-Chlor-4,6-dimethoxy-s-triazin werden zu einer Lösung von 30 g (0,3 Mol) Cyanamid-Dinatriumsalz in 600 ml Aceton gegeben, und das Reaktionsgemisch wird 6 Stunden unter Rückfluß zum Sieden erhitzt. Nach Abdestillieren des Lösungsmittels wird der kristalline Rückstand in 250 ml Wasser gelöst und die Lösung mit konzentrierter Salzsäure angesäuert. Das kristallin angefallene Produkt wird durch Absaugen isoliert.

Man erhält 33 g (61 % der Theorie) 2-Cyanamino-4,6-dimethoxy-s-triazin mit einem Schmelzpunkt über 300°C.

Beispiel (VIII-2)

(a)

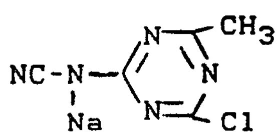

Zu einer Suspension von 8,2 g (0,05 Mol) 2,4-Dichlor-6-methyl-1,3,5-triazin in 100 ml Eiswasser tropft man bei einer Temperatur von 0°C bis 5°C 4,5 g (0,05 Mol) Cyanamid-Dinatriumsalz50 ml Wasser in der Weise, daß ein pH-Wert von 9,5 nicht überschritten wird. Anschließend wird noch eine Stunde

bei 20°C nachgerührt, mit 40 g Natriumchlorid versetzt und ca. 0,5 bis 1 Stunde bei 20°C nachgerührt.

Nach dem Absaugen und Trocknen erhält man 9,1 g (95 % der Theorie) des Natriumsalzes von 4-Chlor-2-cyanamino-6-methyl-1,3,5-triazin vom Schmelzpunkt 190°C (Zers.).

(b)

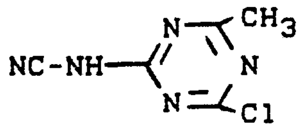

Zu einer Suspension von 2 g (0,01 Mol) des Natriumsalzes von 4-Chlor-2-cyanamino-6-methyl-1,3,5-triazin in 50 ml Wasser wird bei 0°C bis 10°C 1 ml konzentrierte Salzsäure gegeben. Der entstehende Niederschlag wird abgesaugt und getrocknet.

Man erhält so 1,3 g (77 % der Theorie) 4-Chlor-2-cyanamino-6-methyl-1,3,5-triazin vom Schmelzpunkt 105°C (Zers.).

(c)

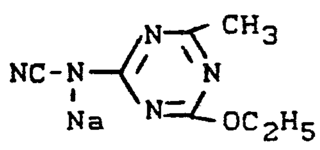

Zu einer Lösung von 29 g (0,15 Mol) des Natriumsalzes von 4-Chlor-2-cyanamino-6-methyl-1,3,5-triazin in 500 ml Ethanol tropft man eine Lösung von 3,7 g (0,16 Mol) Natrium in 100 ml Ethanol in der Weise, daß eine Reaktionstemperatur von 30°C nicht überschritten wird. Anschließend wird bei 20°C 3 Stunden nachgerührt, mit 400 ml Ethanol verdünnt und filtriert. Das Filtrat wird im Vakuum eingeengt, wobei eine Temperatur von 45°C nicht überschritten wird.

Man erhält so 29,9 g (99 % der Theorie) des Natriumsalzes von 2-Cyanamino-4-ethoxy-6-methyl-1,3,5-triazin vom Schmelzpunkt 200°C (Zers.).

(d)

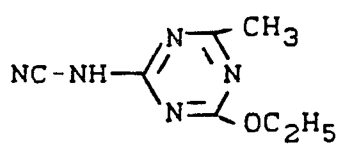

Zu einer Lösung von 30,1 g (0,15 Mol) des Natriumsalzes von 2-Cyanamino-4-ethoxy-6-methyl-1,3,5-triazin in 300 ml Wasser werden bei 0°C bis 10°C 50 ml 20%-ige Salzsäure gegeben und 15 Minuten bei 10°C nachgerührt. Der Niederschlag wird abgesaugt und getrocknet.

Man erhält 26,5 g (98% der Theorie) 2-Cyanamino-4-ethoxy-6-methyl-1,3,5-triazin vom Schmelzpunkt 195°C (Zers.).

(e)

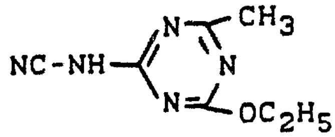

Zu einer Lösung von 2,5 g (0,11 Mol) Natrium in 100 ml Ethanol gibt man portionsweise 8,5 g (0,05 Mol) 4-Chlor-2-cyanamino-6-methyl-1,3,5-triazin in der Weise, daß eine Temperatur von 35°C nicht überschritten wird. Anschließend wird eine Stunde bei 20°C nachgerührt und im Vakuum in der Weise eingeengt, daß eine Badtemperatur von 50°C nicht überschritten wird. Der Rückstand wird in 50 ml Wasser gelöst und mit 6 ml konzentrierter Salzsäure angesäuert. Die entstandenen Kristalle werden abgesaugt und getrocknet.

Man erhält 8,1 g (91 % der Theorie) 2-Cyanamino-4-ethoxy-6-methyl-1,3,5-triazin vom Schmelzpunkt 195°C (Zers.).

Analog können die in der nachstehenden Tabelle 6 aufgeführten Verbindungen der Formel (VIII) hergestellt werden:

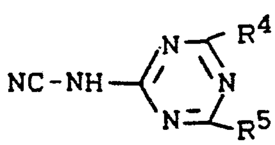

(VIII)

Tabelle 6

| Bsp. Nr. | $R^4$ | $R^5$ | Schmelzpunkt (°C) |
|---|---|---|---|
| VIII- 3 | $OCH_3$ | $N(C_2H_5)_2$ | 114 |
| VIII- 4 | $SCH_3$ | $NHC_2H_5$ | |
| VIII- 5 | $OCH_3$ | $NHCH_3$ | 210 |
| VIII- 6 | Cl | $N(C_2H_5)_2$ | 156 |
| VIII- 7 | $OCH_3$ | $CH_3$ | 184 |
| VIII- 8 | $OCH(CH_3)_2$ | $CH_3$ | |
| VIII- 9 | $SCH_3$ | $CH_3$ | |
| VIII-10 | $SC_2H_5$ | $CH_3$ | |
| VIII-11 | $SCH(CH_3)_2$ | $CH_3$ | |

Tabelle 6-Fortsetzung

| Bsp. Nr. | $R^4$ | $R^5$ | Schmelzpunkt (°C) |
|---|---|---|---|
| VIII-12 | $NHCH_3$ | $CH_3$ | |
| VIII-13 | $NHCH(CH_3)_2$ | $CH_3$ | |
| VIII-14 | $N(CH_3)_2$ | $CH_3$ | |
| VIII-15 | $CH_3$ | $CH_3$ | |

0173956

Herstellung von Ausgangsverbindungen der Formel (XV)

Beispiel (XV-1)

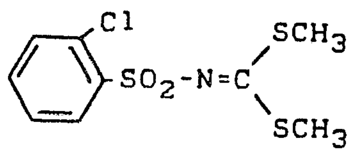

Zu einer Lösung von 20 g (0,1 Mol) 2-Chlor-benzolsulfonsäureamid in 80 ml Dimethylformamid werden bei 20°C gleichzeitig (aus verschiedenen Tropftrichtern) 8 g (0,2 Mol) Natriumhydroxid - gelöst in 15 ml Wasser - und 6 ml (0,11 Mol) Schwefelkohlenstoff tropfenweise gegeben. Nach einstündigem Rühren werden 13 ml (0,22 Mol) Methyliodid zugetropft, und das Reaktionsgemisch wird eine weitere Stunde bei 20°C gerührt. Durch Zugabe von 500 ml Wasser wird das Produkt ausgefällt und durch Absaugen isoliert.

Man erhält 22,1 g (75 % der Theorie) N-(2-Chlor-benzolsulfonyl)-S',S"-dimethyl-isothiocarbamidsäureester vom Schmelzpunkt 112°C.

Analog können die in der nachstehenden Tabelle 7 aufgeführten Verbindungen der Formel (XV) hergestellt werden:

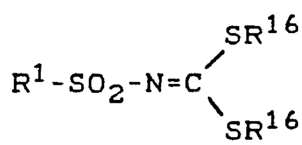 (XV)

Tabelle 7

| Bsp. Nr. | $R^1$ | $R^{16}$ | Schmelzpunkt (°C) |
|---|---|---|---|
| XV- 2 | 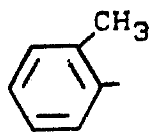 | $CH_3$ | 103 |
| XV- 3 | 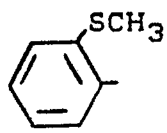 | $CH_3$ | 143 |
| XV- 4 | 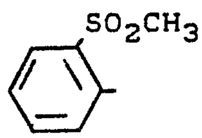 | $CH_3$ | |
| XV- 5 | 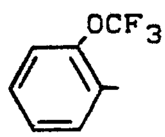 | $CH_3$ | 88 |
| XV- 6 | 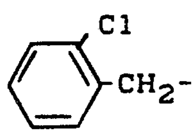 | $CH_3$ | 96 |
| XV- 7 | 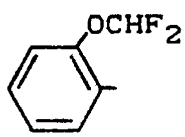 | $CH_3$ | 112 |
| XV- 8 | 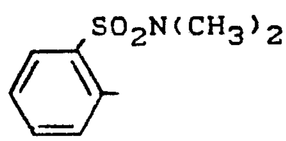 | $CH_3$ | |
| XV- 9 | 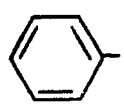 | $CH_3$ | 100 |
| XV-10 | 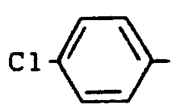 | $CH_3$ | 94 |

Tabelle 7-Fortsetzung

| Bsp. Nr. | $R^1$ | $R^{16}$ | Schmelz-punkt (°C) |
|---|---|---|---|
| XV-11 | 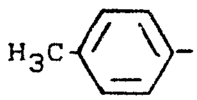 | $CH_3$ | 107 |
| XV-12 | 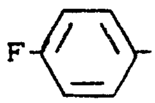 | $CH_3$ | 113 |
| XV-13 | 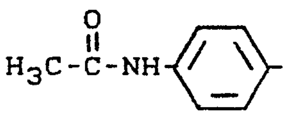 | $CH_3$ | 170 |
| XV-14 | 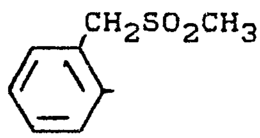 | $CH_3$ | |
| XV-15 | 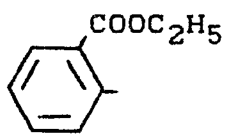 | $CH_3$ | |
| XV-16 | 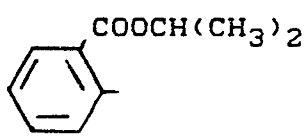 | $CH_3$ | |
| XV-17 | 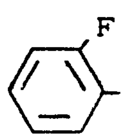 | $CH_3$ | |
| XV-18 | 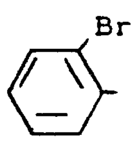 | $CH_3$ | |
| XV-19 | 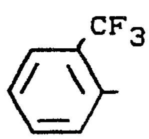 | $CH_3$ | |

Tabelle 7-Fortsetzung

| Bsp. Nr. | $R^1$ | $R^{16}$ | Schmelzpunkt (°C) |
|---|---|---|---|
| XV-20 | 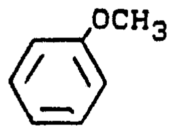 | $CH_3$ | |
| XV-21 | 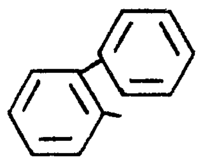 | $CH_3$ | |
| XV-22 | 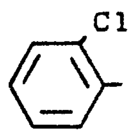 | $C_2H_5$ | 64-66 |
| XV-23 | 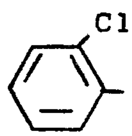 | $CH(CH_3)_2$ | 65-67 |
| XV-24 | 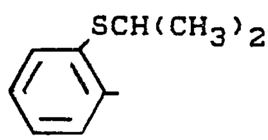 | $CH_3$ | 98 |
| XV-25 | 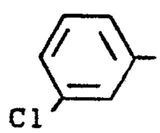 | $CH_3$ | 84 |

## Herstellung von Ausgangsstoffen der Formel (XVI)

### Beispiel (XVI-1)

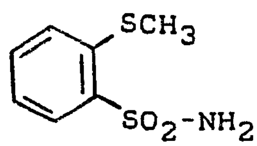

50 ml konzentriertes Ammoniak werden bei 20°C bis 30°C unter Rühren zu einer Mischung von 60 g (0,27 Mol) 2-Methylthio-benzolsulfonsäurechlorid und 60 ml Aceton getropft. Man rührt eine Stunde bei 20°C nach und gießt das Reaktionsgemisch dann in 600 ml 10 prozentige Natronlauge. Diese Lösung wird filtriert und dann mit konzentrierter Salzsäure angesäuert. Das hierbei kristallin angefallene Produkt wird durch Absaugen isoliert.

Man erhält 19 g (30 % der Theorie) 2-Methylthio-benzolsulfonsäureamid von Schmelzpunkt 149°C.

Nach dem im vorausgehenden Beispiel exemplarisch beschriebenen Verfahren können die in der nachstehenden Tabelle 8 aufgeführten Verbindungen der Formel (XVI) hergestellt werden:

$$R^1-SO_2-NH_2 \qquad (XVI)$$

Tabelle 8

| Bsp. Nr. | $R^1$ | Schmelz-punkt (°C) |
|---|---|---|
| XVI- 2 | 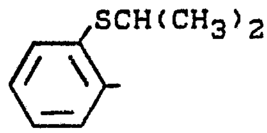 | 102 |
| XVI- 3 | 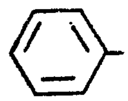 | 156 |
| XVI- 4 | 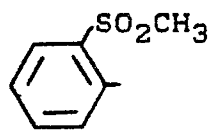 | 235 |
| XVI- 5 | 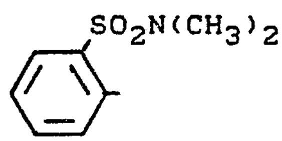 | 142 |
| XVI- 6 | 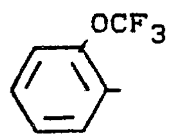 | 186-189 |
| XVI- 7 | 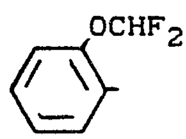 | 134 |
| XVI- 8 | 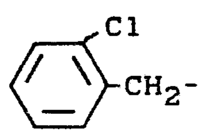 | |
| XVI- 9 | 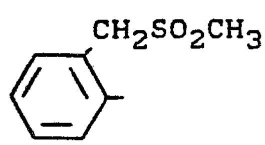 | 175 |
| XVI-10 | 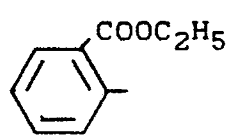 | 78 |

Tabelle 8-Fortsetzung

| Bsp. Nr. | $R^1$ | Schmelzpunkt (°C) |
|---|---|---|
| XVI-11 | 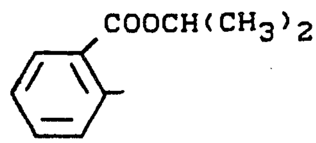 | |
| XVI-12 | 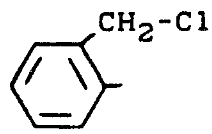 | |

## Beispiel A

Pre-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalem Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test zeigen z.B. die folgenden Verbindungen der Herstellungsbeispiele eine ausgezeichnete Wirksamkeit: (2) und (20).

Beispiel B

Post-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test zeigen z.B. die folgenden Verbindungen der Herstellungsbeispiele eine ausgezeichnete Wirksamkeit: (2), (20) und (32).

0173956

Patentansprüche

1). Sulfonylguanidinotriazin-Derivate der allgemeinen Formel (I)

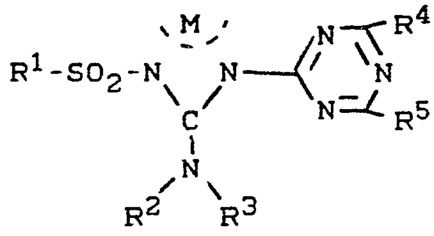

(I)

in welcher

M für Wasserstoff oder ein Metalläquivalent steht,

$R^1$ für einen gegebenenfalls substituierten Phenylrest

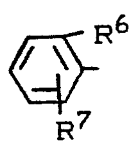

steht, worin

$R^6$ und $R^7$ gleich oder verschieden sind und für Wasserstoff, Halogen [wie insbesondere Fluor, Chlor, und/oder Brom], Cyano, Nitro, $C_1$-$C_4$-Alkyl, [welches gegebenenfalls durch Fluor, Chlor, Brom, Cyano, $C_1$-$C_4$-Alkoxy-carbonyl, $C_1$-$C_4$-Alkylamino-carbonyl, Di-($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_4$-Alkoxy [welches gegebenenfalls

durch Fluor, Chlor, Brom, Cyano, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_4$-Alkylthio, $C_1$-$C_4$-Alkylsulfinyl oder für $C_1$-$C_4$-Alkylsulfonyl [welche gegebenenfalls durch Fluor, Chlor, Brom, Cyano oder $C_1$-$C_4$-Alkoxy-carbonyl substituiert sind], für $C_1$-$C_4$-Alkylamino-sulfonyl, Di-($C_1$-$C_4$-alkyl)-amino-sulfonyl, $C_1$-$C_4$-Alkoxyamino-sulfonyl, Benzyloxy-aminosulfonyl, $C_1$-$C_4$-Alkoxy-$C_1$-$C_4$- alkyl-amino-sulfonyl, für Phenyl oder Phenoxy, für $C_1$-$C_4$-Alkoxysulfonyl, für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano oder $C_1$-$C_4$-Alkoxy-carbonyl substituiert ist], für $C_3$-$C_6$-Alkenyloxy [welches gegebenenfalls durch Fluor, Chlor, Brom, Cyano oder $C_1$-$C_4$-Alkoxy-carbonyl substituiert ist], für $C_3$-$C_6$-Alkenylthio [welches gegebenenfalls durch Fluor, Chlor, Brom, Cyano oder $C_1$-$C_4$-Alkoxy-carbonyl substituiert ist], für $C_1$-$C_6$-

Alkoxy-carbonyl [welches gegebenenfalls durch Fluor, Chlor, Brom, Cyano oder $C_1$-$C_4$-Alkoxy substituiert ist], für $C_3$-$C_6$-Cycloalkoxy-carbonyl, für $C_1$-$C_6$-Alkylthiocarbonyl [welches gegebenenfalls durch Fluor, Chlor, Brom, Cyano oder $C_1$-$C_4$-Alkoxy-carbonyl substituiert ist], für Aminocarbonyl, $C_1$-$C_4$-Alkylamino-carbonyl, Di-($C_1$-$C_4$-alkyl)-amino-carbonyl, $C_1$-$C_4$-Alkoxy-amino-carbonyl, $C_3$-$C_4$-Alkenyloxy-amino-carbonyl, Benzyloxyaminocarbonyl, $C_1$-$C_4$-Alkoxy-$C_1$-$C_4$-alkyl-amino-carbonyl, Dimethylhydrazinocarbonyl, Dimethylhydrazinosulfonyl, für $C_3$-$C_6$-Alkinyloxy oder $C_3$-$C_6$-Alkinylthio stehen,

in welcher weiter

$R^1$ für einen gegebenenfalls substituierten Benzylrest

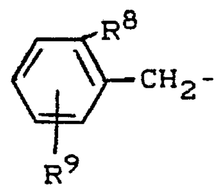

steht, worin

$R^8$ und $R^9$ gleich oder verschieden sind und für Wasserstoff, Halogen [wie insbesondere Fluor, Chlor oder Brom], Cyano, Nitro, $C_1$-$C_4$-Alkyl [welches gegebenenfalls durch Fluor, und/oder Chlor substituiert ist], $C_1$-$C_4$-Alkoxy [welches gegebenenfalls durch Fluor und/oder Chlor substituiert ist], $C_1$-$C_4$-Alkoxy-carbonyl, $C_1$-$C_4$-Alkylthio, $C_1$-$C_4$-Alkylsulfinyl, $C_1$-$C_4$-Alkylsulfonyl oder Di-($C_1$-$C_4$-alkyl)-aminosulfonyl stehen;

in welcher weiter

$R^2$ für Wasserstoff, $C_1$-$C_4$-Alkyl oder einen Sulfonylrest

$$R^{10}-SO_2-$$

steht, worin

$R^{10}$ für $C_1$-$C_{12}$-Alkyl [welches gegebenenfalls durch Fluor, Chlor, Brom, Cyano, Nitro, $C_1$-$C_4$-Alkoxy-carbonyl, $C_1$-$C_4$-Alkylamino-carbonyl, Di-($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_{12}$-Alkoxy [welches gegebenenfalls durch Fluor, Chlor, Brom, Cyano, Nitro, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_2$-$C_{12}$-Alkenyl [welches gegebenenfalls durch Fluor, Chlor, Brom, Cyano, Nitro, $C_1$-$C_4$-

Alkoxy, $C_1$-$C_4$-Alkylthio oder Phenyl substituiert ist], für $C_2$-$C_{12}$-Alkenyloxy [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_1$-$C_8$-Alkylamino, Di-($C_1$-$C_4$-alkyl)-amino, $C_3$-$C_6$-Cycloalkyl-amino oder Di-($C_3$-$C_6$-cycloalkyl)-amino [welche gegebenenfalls durch Fluor, Chlor, Brom, Cyano, Nitro, Phenyl, Phenoxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiert sind], für $C_2$-$C_8$-Alkenylamino [welches gegebenenfalls durch Fluor, Chlor, Brom, Cyano, Nitro oder Phenyl substituiert ist], für Phenylamino oder Benzylamino [welche gegebenenfalls durch Fluor, Chlor, Brom, $C_1$-$C_4$-Alkyl, Trifluormethyl, $C_1$-$C_4$-Alkoxy, $C_1$-$C_2$-Fluoralkoxy, $C_1$-$C_4$-Alkylthio, $C_1$-$C_4$-Alkylsulfinyl, $C_1$-$C_4$-Alkylsulfonyl, Cyano, Nitro und/oder $C_1$-$C_4$-Alkoxy-carbonyl substituiert sind], für Benzyl [welches gegebenenfalls durch Fluor, Chlor, Brom, Cyano, Nitro, $C_1$-$C_4$-Alkyl, Trifluormethyl, $C_1$-$C_4$-Alkoxy-carbonyl, $C_1$-$C_4$-Alkoxy oder Di-($C_1$-$C_4$-alkyl)-aminosulfonyl substituiert ist] oder für einen gegebenenfalls substituierten Phenylrest

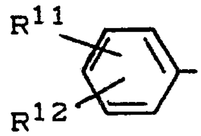

steht, worin

$R^{11}$ und $R^{12}$ gleich oder verschieden sind und die oben für $R^6$ und $R^7$ angegebenen Bedeutungen haben, jedoch nicht in jedem Einzelfall mit $R^6$ und $R^7$ identisch sind;

in welcher weiter

$R^3$ für den Rest $-O-R^{13}$ steht, worin

$R^{13}$ für $C_1$-$C_{12}$-Alkyl [welches gegebenenfalls durch Fluor, Chlor, Brom, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_3$-$C_6$-Cycloalkyl, $C_3$-$C_6$-Cycloalkyl-$C_1$-$C_2$-alkyl, $C_3$-$C_6$-Alkenyl [welches gegebenenfalls durch Fluor, Chlor oder Brom substituiert ist], $C_3$-$C_6$-Alkinyl, $C_1$-$C_4$-Alkoxy-carbonyl-$C_1$-$C_2$-alkyl, Aminocarbonyl-methyl, $C_1$-$C_4$-Alkylamino-carbonylmethyl, Di-($C_1$-$C_4$-alkyl)-amino-carbonylmethyl oder für Phenyl, Benzyl oder Phenylethyl [welche gegebenenfalls durch Fluor, Chlor, Nitro, Cyano, $C_1$-$C_4$-Alkyl, $C_1$-$C_4$-Alkoxy oder $C_1$-$C_4$-Alkoxycarbonyl substituiert sind] steht;

in welcher weiter

$R^3$ für den Rest

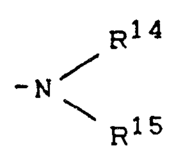

steht, worin

$R^{14}$ für Wasserstoff oder $C_1$-$C_4$-Alkyl steht und

$R^{15}$ für Wasserstoff, $C_1$-$C_4$-Alkyl [welches gegebenenfalls durch Fluor, Chlor, Brom, Cyano, $C_1$-$C_4$-Alkoxy oder $C_1$-$C_4$-Alkoxycarbonyl substituiert ist], für $C_3$-$C_6$ Cycloalkyl, für Phenyl, Benzyl oder Phenylethyl [welche gegebenenfalls durch Fluor, Chlor, Brom, Cyano, Nitro, $C_1$-$C_4$- Alkyl, $C_1$-$C_4$-Alkoxy oder $C_1$-$C_4$-Alkoxycarbonyl substituiert sind], für $C_1$-$C_4$-Alkyl-carbonyl, $C_1$-$C_4$-Alkoxy-carbonyl, $C_1$-$C_4$-Alkyl-sulfonyl oder Phenylsulfonyl [welches gegebenenfalls durch Fluor, Chlor, Brom, Cyano, Nitro oder Methyl substituiert ist] steht;

in welcher weiter - für den Fall, daß $R^2$ für einen Sulfonylrest $R^{10}$-$SO_2$- steht, worin $R^{10}$ die oben angegebene Bedeutung hat - $R^3$ auch für Wasserstoff steht;

in welcher weiter

$R^4$ für Fluor, Chlor, Brom, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für $C_1$-$C_4$-Alkylthio

[welches gegebenenfalls durch Fluor und/oder Chlor substituiert ist] oder für $C_1$-$C_4$-Alkyl- oder Di-($C_1$-$C_4$-alkyl)-amino [welche gegebenenfalls durch Fluor substituiert sind] steht und

$R^5$ für Fluor, Chlor, Brom, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für $C_1$-$C_4$-Alkylthio [welches gegebenenfalls durch Fluor und/oder Chlor substituiert ist], für $C_1$-$C_4$-Alkyl- oder Di-($C_1$-$C_4$-alkyl)-amino [welche gegebenenfalls durch Fluor substituiert sind] steht,

sowie 1:1-Addukte von Verbindungen der Formel (I) mit starken Säuren,

wobei folgende Verbindungen ausgenommen sind:

N'-(4-Methoxy-6-methyl-s-triazin-2-yl)-N"-dimethylamino-N'''-(2-difluormethoxy-benzolsulfonyl)-guanidin, N'-(4,6-Dimethoxy-s-triazin-2-yl)-N"-methoxy-N",N'''-bis-(2-trifluormethyl-benzolsulfonyl)-guanidin, N'-(4-Dimethylamino-6-methyl-s-triazin-2-yl)-N"-methoxy-N'''-(2-methyl-benzolsulfonyl)-guanidin und N'-(4-Dimethylamino-6-methyl-s-triazin-2-yl)-N"-dimethyl-amino-N'''-(2-methyl-benzolsulfonyl)-guanidin.

2). Verfahren zur Herstellung von Sulfonylguanidinotriazin-Derivaten der allgemeinen Formel (I) gemäß Anspruch 1, dadurch gekennzeichnet, daß man

(a) für den Fall, daß M für Wasserstoff steht, Guanidinotriazin-Derivate der Formel (II)

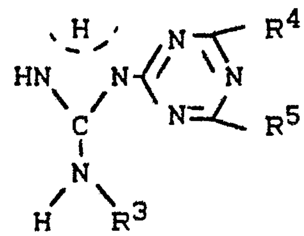

(II)

in welcher

$R^3$, $R^4$ und $R^5$ die oben angegebenen Bedeutungen haben,

mit Sulfonsäurechloriden der Formel (III)

$R^1$-$SO_2$-Cl (III)

in welcher

$R^1$ die oben angegebene Bedeutung hat,

in Gegenwart von Säureakzeptoren und gegebenenfalls falls in Gegenwart von Verdünnungsmitteln umsetzt;

oder daß man

(b) für den Fall, daß M für Wasserstoff steht und $R^2$ für Wasserstoff oder $C_1$-$C_4$-Alkyl steht, die nach dem oben unter (a) angegebenen Verfahren erhältlichen Sulfonylguanidinotriazin Derivate der Formel (ID)

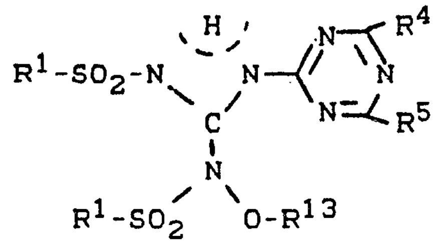

(ID)

in welcher

$R^1$, $R^4$, $R^5$ und $R^{13}$ die oben angegebenen Bedeutungen haben

mit Aminoverbindungen der Formel (IV)

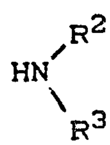

(IV)

in welcher

$R^2$ für Wasserstoff oder $C_1$-$C_4$-Alkyl steht und
$R^3$ die oben angegebene Bedeutung hat,

bzw. mit Hydrochloriden von Aminoverbindungen der Formel (IV), gegebenenfalls in Gegenwart von Säureakzeptoren und gegebenenfalls in Gegenwart von Verdünnungsmitteln umsetzt;

oder daß man

(c) für den Fall, daß M für Wasserstoff steht und $R^2$ für einen gegebenenfalls substituierten Sulfonylrest

$R^{10}-SO_2-$ steht, worin

$R^{10}$ die oben angegebene Bedeutung hat,

Sulfonylguanidinotriazin-Derivate der Formel (IE)

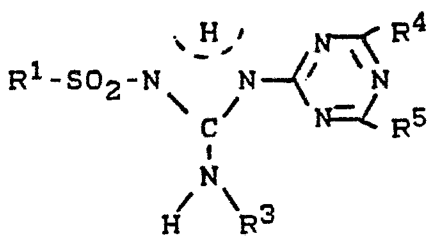

(IE)

in welcher

$R^1$, $R^3$, $R^4$ und $R^5$ die oben angegebenen Bedeutungen haben,

mit Sulfonsäurechloriden der Formel (V)

$$R^{10}-SO_2-Cl \quad (V)$$

in welcher

$R^{10}$ die oben angegebene Bedeutung hat,

in Gegenwart von Säureakzeptoren und gegebenenfalls in Gegenwart von Verdünnungsmitteln umsetzt;

oder daß man

(d) ebenfalls für den Fall, daß M für Wasserstoff steht und $R^2$ für einen gegebenenfalls substituierten Sulfonylrest

$R^{10}-SO_2-$     steht, worin

$R^{10}$ die oben angegebene Bedeutung hat,

Sulfonylguanidinotriazin-Derivate der Formel (VI)

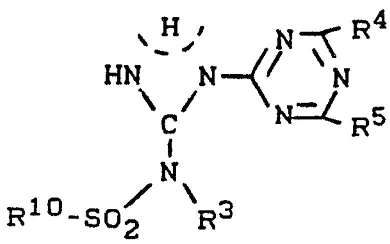

(VI)

in welcher

$R^3$, $R^4$, $R^5$ und $R^{10}$ die oben angegebenen Bedeutungen haben,

mit Sulfonsäurechloriden der Formel (III)

$$R^1-SO_2-Cl \qquad (III)$$

in welcher

$R^1$ die oben angegebene Bedeutung hat,

in Gegenwart von Säureakzeptoren und gegebenenfalls in Gegenwart von Verdünnungsmitteln umsetzt;

oder daß man

(e) für den Fall, daß M für Wasserstoff steht und $R^2$ für Wasserstoff oder $C_1$-$C_4$-Alkyl steht, Sulfonyl-isothioureidotriazin-Derivate der Formel (VII)

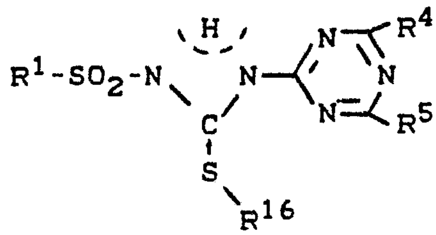

(VII)

in welcher

$R^1$, $R^4$ und $R^5$ die oben angegebenen Bedeutungen haben und

$R^{16}$ für $C_1$-$C_4$-Alkyl oder Benzyl steht,

mit Aminoverbindungen der Formel (IV)

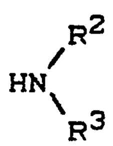

(IV)

in welcher

$R^2$ für Wasserstoff oder $C_1$-$C_4$-Alkyl steht und
$R^3$ die oben angegebene Bedeutung hat,

gegebenenfalls in Gegenwart von Verdünnungsmitteln umsetzt;

oder daß man

(f) für den Fall, daß M für ein Metalläquivalent steht, die nach den oben unter (a), (b), (c), (d) und (e) angegebenen Verfahren erhältlichen Verbindungen der Formel (I), in welcher M für Wasserstoff steht und $R^1$, $R^2$, $R^3$, $R^4$ und $R^5$ die oben angegebenen Bedeutungen haben,

mit Metallhydroxiden, -hydriden oder -alkanolaten oder mit metallorganischen Verbindungen, gegebenenfalls in Gegenwart von Verdünnungsmitteln umsetzt;

oder daß man

(g) für den Fall, daß 1:1-Addukte von Verbindungen der Formel (I) mit starken Säuren herzustellen sind, Verbindungen der Formel (I), in welcher M für Wasserstoff steht und $R^1$, $R^2$, $R^3$, $R^4$ und $R^5$ die oben angegebenen Bedeutungen haben,

mit starken Säuren, gegebenenfalls in Gegenwart von Verdünnungsmitteln umsetzt.

3). Herbizide Mittel, gekennzeichnet durch einen Gehalt an mindestens einem Sulfonylguanidinotriazin-Derivat der allgemeinen Formel (I) gemäß Anspruch 1.

4). Verwendung von Sulfonylguanidinotriazin-Derivaten der allgemeinen Formel (I) gemäß Anspruch 1 zur Bekämpfung von unerwünschtem Pflanzenwachstum.

5). Verfahren zur Herstellung von herbiziden Mitteln, dadurch gekennzeichnet, daß man Sulfonylguanidinotriazin-Derivate der allgemeinen Formel (I) gemäß Anspruch 1 mit Streckmitteln und/oder oberflächenaktiven Mitteln vermischt.

6). Sulfonylisothioureidotriazin-Derivate der allgemeinen Formel (VIIA)

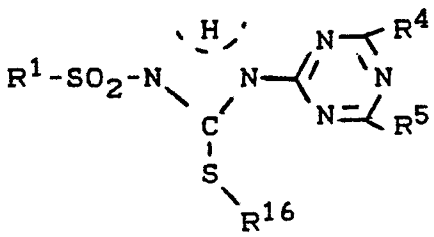

(VIIA)

in welcher

$R^1$ für einen substituierten Phenylrest

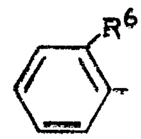

steht, worin

$R^6$ für $C_1$-$C_4$-Fluoralkoxy, $C_1$-$C_4$-Alkylthio, $C_1$-$C_4$-Alkylsulfinyl oder $C_1$-$C_4$-Alkylsulfonyl steht,

$R^4$ für Fluor, Chlor, Brom, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für $C_1$-$C_4$-Alkylthio [welches gegebenenfalls durch Fluor und/oder Chlor substituiert ist] oder für $C_1$-$C_4$-Alkyl- oder Di-($C_1$-$C_4$-alkyl)-amino [welche gegebenenfalls durch Fluor substituiert sind] steht,

$R^5$ für Fluor, Chlor, Brom, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für $C_1$-$C_4$-Alkylthio [welches gegebenenfalls durch Fluor und/oder Chlor substituiert ist], für $C_1$-$C_4$-Alkyl- oder Di-($C_1$-$C_4$-alkyl)-amino [welche gegebenenfalls durch Fluor substituiert sind] steht und

$R^{16}$ für $C_1$-$C_4$-Alkyl oder Benzyl steht.

7). Verfahren zur Herstellung von Sulfonylisothioureidotriazin-Derivaten der Formel (VIIA) gemäß Anspruch 6, dadurch gekennzeichnet, daß man N-Sulfonyl-imino-dithiokohlensäureester der Formel (XVA)

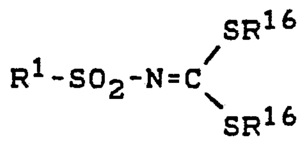 (XVA)

in welcher

$R^1$ und $R^{16}$ die unter Anspruch 6 angegebenen Bedeutungen haben,

mit Aminotriazinen der Formel (XI)

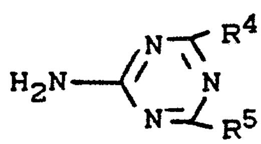 (XI)

in welcher

$R^4$ und $R^5$ die unter Anspruch 6 angegebenen Bedeutungen haben,

gegebenenfalls in Gegenwart von Basen und gegebenenfalls in Gegenwart von Verdünnungsmitteln umsetzt, nach Reaktionsende mit Wasser verdünnt und mit einer starken Säure ansäuert.

8). Sulfonylguanidinotriazin-Derivate der allgemeinen Formel (VI)

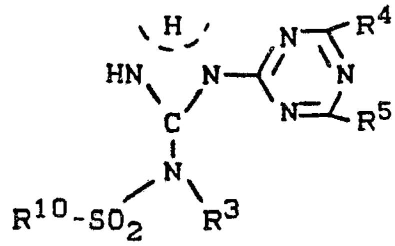 (VI)

in welcher

$R^3$ für den Rest $-O-R^{13}$ steht, worin

$R^{13}$ für $C_1$-$C_{12}$-Alkyl [welches gegebenenfalls durch Fluor, Chlor, Brom, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_3$-$C_6$-Cycloalkyl, $C_3$-$C_6$-Cycloalkyl-$C_1$-$C_2$-alkyl, $C_3$-$C_6$-Alkenyl [welches gegebenenfalls durch Fluor, Chlor oder Brom substituiert ist], $C_3$-$C_6$-Alkinyl, $C_1$-$C_4$-Alkoxy-carbonyl-$C_1$-$C_2$-alkyl, Aminocarbonyl-methyl, $C_1$-$C_4$-Alkylamino-carbonylmethyl, Di-($C_1$-$C_4$-alkyl)-amino-carbonylmethyl, oder für Phenyl, Benzyl oder Phenylethyl [welche gegebenenfalls durch Fluor, Chlor, Nitro, Cyano, $C_1$-$C_4$-Alkyl, $C_1$-$C_4$-Alkoxy oder $C_1$-$C_4$-Alkoxycarbonyl substituiert sind] steht;

in welcher weiter

$R^3$ für den Rest 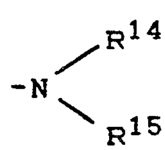 steht, worin

$R^{14}$ für Wasserstoff oder $C_1$-$C_4$-Alkyl steht und

$R^{15}$ für Wasserstoff, $C_1$-$C_4$-Alkyl [welches gegebenenfalls durch Fluor, Chlor, Brom, Cyano, $C_1$-$C_4$-Alkoxy oder $C_1$-$C_4$-Alkoxycarbonyl substituiert ist], für $C_3$-$C_6$-Cycloalkyl, für Phenyl, Benzyl oder Phenylethyl [welche gegebenenfalls durch Fluor, Chlor, Brom, Cyano, Nitro, $C_1$-$C_4$-Alkyl, $C_1$-$C_4$-Alkoxy oder $C_1$-$C_4$-Alkoxycarbonyl substituiert sind], für $C_1$-$C_4$-Alkyl-carbonyl, $C_1$-$C_4$-Alkoxy-carbonyl, $C_1$-$C_4$-Alkyl-sulfonyl oder Phenylsulfonyl [welches gegebenenfalls durch Fluor, Chlor, Brom, Cyano, Nitro oder Methyl substituiert ist] steht;

$R^4$ für Fluor, Chlor, Brom, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/oder Chlor substituiert ist], für $C_1$-$C_4$-Alkylthio [welches gegebenenfalls durch Fluor und/oder Chlor substituiert ist] oder für $C_1$-$C_4$-Alkyl- oder Di-($C_1$-$C_4$-alkyl)-amino [welche gegebenenfalls durch Fluor substituiert sind] steht,

$R^5$ für Fluor, Chlor, Brom, Cyclopropyl, $C_1$-$C_4$-Alkyl [welches gegebenenfalls durch Fluor und/oder Chlor substituiert ist], für $C_1$-$C_4$-Alkoxy [welches gegebenenfalls durch Fluor und/

oder Chlor substituiert ist], für $C_1$-$C_4$-Alkylthio [welches gegebenenfalls durch Fluor und/oder Chlor substituiert ist], für $C_1$-$C_4$-Alkyl- oder Di-($C_1$-$C_4$-alkyl)-amino [welche gegebenenfalls durch Fluor substituiert sind] steht

und in welcher ferner

$R^{10}$ für $C_1$-$C_{12}$-Alkyl [welches gegebenenfalls durch Fluor, Chlor, Brom, Cyano, Nitro, $C_1$-$C_4$-Alkoxy-carbonyl, $C_1$-$C_4$-Alkylamino-carbonyl, Di-($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_{12}$-Alkoxy [welches gegebenenfalls durch Fluor, Chlor, Brom, Cyano, Nitro, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_2$-$C_{12}$-Alkenyl [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_2$-$C_{12}$-Alkenyloxy [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_1$-$C_8$-Alkylamino, Di-($C_1$-$C_4$-alkyl)amino, $C_3$-$C_6$-Cycloalkyl-amino oder Di--($C_3$-$C_6$-cycloalkyl)-amino [welche gegebenenfalls durch Fluor, Chlor, Brom, Cyano,

Nitro, Phenyl, Phenoxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiert sind], für $C_2$-$C_8$-Alkenylamino [welches gegebenenfalls durch Fluor, Chlor, Brom, Cyano, Nitro oder Phenyl substituiert ist], für Phenylamino oder Benzylamino [welche gegebenenfalls durch Fluor, Chlor, Brom, $C_1$-$C_4$-Alkyl, Trifluormethyl, $C_1$-$C_4$-Alkoxy, $C_1$-$C_2$-Fluoralkoxy, $C_1$-$C_4$-Alkylthio, $C_1$-$C_4$-Alkylsulfinyl, $C_1$-$C_4$-Alkylsulfonyl, Cyano, Nitro und/oder $C_1$-$C_4$-Alkoxy-carbonyl substituiert sind], für Benzyl [welches gegebenenfalls durch Fluor, Chlor, Brom, Cyano, Nitro, $C_1$-$C_4$-Alkyl, Trifluormethyl, $C_1$-$C_4$-Alkoxy-carbonyl, $C_1$-$C_4$-Alkoxy oder Di-($C_1$-$C_4$-alkyl)-aminosulfonyl substituiert ist] oder für einen gegebenenfalls substituierten Phenylrest

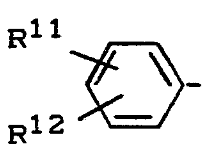

steht, worin

$R^{11}$ und $R^{12}$ gleich oder verschieden sind und für Wasserstoff, Fluor, Chlor, Brom, Cyano, Nitro, Methyl, Trifluormethyl, $C_1$-$C_2$-Alkoxy [welches gegebenenfalls durch Fluor,

Chlor, Cyano oder $C_1$-$C_3$-Alkoxy-carbonyl substituiert ist], $C_1$-$C_3$-Alkylthio, $C_1$-$C_3$-Alkyl-sulfinyl oder $C_1$-$C_3$-Alkylsulfonyl [welche gegebenenfalls durch Fluor und/oder Chlor substituiert sind], Dimethylaminosulfonyl, Diethylaminosulfonyl, N-Methoxy-N-methylaminosulfonyl, Phenyl oder $C_1$-$C_4$-Alkoxy-carbonyl [welches gegebenenfalls durch Fluor, Chlor, Cyano oder $C_1$-$C_2$-Alkoxy substituiert ist] stehen.

9). Verfahren zur Herstellung von Sulfonylguanidinotriazin-Derivaten der allgemeinen Formel (VI) gemäß Anspruch 8, dadurch gekennzeichnet, daß man Guanidinotriazin-Derivate der Formel (II) gemäß Anspruch 2 mit Sulfonsäurechloriden der Formel (V) gemäß Anspruch 2 in Gegenwart von Säureakzeptoren und gegebenenfalls in Gegenwart von Verdünnungsmitteln umsetzt.

Europäisches Patentamt

# EUROPÄISCHER RECHERCHENBERICHT

0173956
Nummer der Anmeldung

EP 85110821.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| P,A | EP - A1 - 0 121 082 (BAYER)<br>* Ansprüche 1-7 *<br>-- | 1-6,8,9 | C 07 D 251/46<br>C 07 D 251/16<br>C 07 D 251/18<br>A 01 N 47/44 |
| A | EP - A1 - 0 117 014 (DU PONT)<br>* Zusammenfassung *<br>-- | 1,3,4 | |
| A | DE - A1 - 3 243 533 (SANDOZ)<br>* Ansprüche 1,7 *<br>-- | 1,3 | |
| A | DD - A - 71 015 (KOCHMANN)<br>* Anspruch *<br>---- | 3 | |
| | | | RECHERCHIERTE SACHGEBIETE (Int. Cl 4) |
| | | | C 07 D 251/00 |

Der vorliegende Recherchenbericht wurde für alle Patentansprüche erstellt.

| Recherchenort | Abschlußdatum der Recherche | Prüfer |
|---|---|---|
| WIEN | 18-10-1985 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82